(19) Europäisches Patentamt
European Patent Office
Office européen des brevets

(11) **EP 3 622 068 B1**

(12) **EUROPEAN PATENT SPECIFICATION**

(45) Date of publication and mention
of the grant of the patent:
**28.02.2024   Bulletin 2024/09**

(51) International Patent Classification (IPC):
**C12N 15/09** (2006.01)   **C12N 15/10** (2006.01)
**C12Q 1/6827** (2018.01)

(21) Application number: **18797828.3**

(22) Date of filing: **10.05.2018**

(52) Cooperative Patent Classification (CPC):
(C-Sets available)
**C12Q 1/6827**   (Cont.)

(86) International application number:
**PCT/US2018/032157**

(87) International publication number:
**WO 2018/209145 (15.11.2018 Gazette 2018/46)**

(54) **METHODS FOR DETERMINATION OF MUTATIONS IN SINGLE REPLICATION EVENTS**

VERFAHREN ZUR BESTIMMUNG VON MUTATIONEN IN EINZELREPLIKATIONSEREIGNISSEN

PROCÉDÉS POUR LA DÉTERMINATION DE MUTATIONS DANS DES ÉVÉNEMENTS DE RÉPLICATION UNIQUES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **10.05.2017   US 201762504484 P**
**06.06.2017   US 201762516028 P**

(43) Date of publication of application:
**18.03.2020   Bulletin 2020/12**

(73) Proprietors:
• **The Broad Institute, Inc.**
**Cambridge, MA 02142 (US)**
• **Massachusetts Institute Of Technology**
**Cambridge, MA 02139 (US)**

(72) Inventors:
• **KIM, Soohong**
**Cambridge**
**MA 02142 (US)**
• **BLAINEY, Paul**
**Cambridge**
**MA 02142 (US)**
• **BRODY, Yehuda**
**Cambridge**
**MA 02142 (US)**
• **KIMMERLING, Robert**
**Cambridge**
**MA 02139 (US)**

(74) Representative: **FRKelly**
**Waterways House**
**Grand Canal Quay**
**Dublin D02 PD39 (IE)**

(56) References cited:
**WO-A1-2015/013191      US-A1- 2009 292 482**

• **SZIKRISZT B. ET AL.,: "A comprehensive survey of mutagenic impact of common cancer cytotoxics", GENOME BIOLOGY, vol. 17, 9 May 2016 (2016-05-09), pages 99-115, XP9525277,**
• **BEHJATI S. ET AL.,: "Genome sequencing of normal cells reveals developmental lineages and mutational processes", NATURE, vol. 513, 18 September 2014 (2014-09-18), pages 422-425, XP9525283,**
• **LODATO M.A. ET AL.,: "Somatic mutation in single human neurons tracks developmental and transcriptional history", SCIENCE, vol. 350, 2 October 2015 (2015-10-02), XP002801900,**

- **PAUL C. BLAINEY: "The future is now: single-cell genomics of bacteria and archaea", FEMS MICROBIOLOGY REVIEWS, vol. 37, no. 3, 11 February 2013 (2013-02-11), pages 407-427, XP55132194, ISSN: 0168-6445, DOI: 10.1111/1574-6976.12015**
- **BLAINEY: "The future is now: single- cell genomics of bacteria and archaea", FEMS Microbiology Reviews, vol. 37, no. 3, May 2013 (2013-05), pages 407-427, XP055132194,**

(52) Cooperative Patent Classification (CPC): (Cont.)

C-Sets
**C12Q 1/6827, C12Q 2535/122;
C12Q 1/6827, C12Q 2535/122, C12Q 2565/629**

**Description**

**TECHNICAL FIELD**

The subject matter disclosed herein is generally directed to methods, devices, and reagents for mutation detection in mammalian and bacterial cells during single replication events.

**BACKGROUND**

Somatic mutations are implicated in age-related diseases including cancer, neurodegeneration, and organ failure in humans (Fernández et al. 2016; Rossi et al. 2008; Campbell et al. 2015; Vijg et al. 2017; Lodato et al. 2017). Somatic mutations steadily accumulate in our cells, but most somatic mutations are harmless. Occasionally a mutation affects a gene or a regulatory element in a way that leads to a phenotypic consequence (Stratton et al. 2009 Nature 458, 719-724). The detailed analysis of somatic mutations provides insights into the causes of mutagenesis. Moreover, identification and quantitative analysis of human somatic mutation accumulation is extremely important for several fields such as improving healthcare through preventative medicine, detection of potential carcinogenic materials, estimating the baseline risk of cancer and other diseases of aging etc. Understanding the intrinsic and extrinsic factors that contribute to mutagenesis (including mutation-inducing cancer therapies) is currently a top priority in cancer prevention and treatment (Drost et al. 2017; Wu et al. 2016; Vijg et al. 2017). However, available methods for somatic mutation detection are far from satisfactory, limiting our progress in understanding how mutations accumulate in cells and impact health (Gawad et al. 2016; Baslan and Hicks 2017). The current knowledge about somatic mutations rate is incomplete and mostly measured indirectly. Despite the complexity of nucleoside chemistry, the wide range of DNA lesions characterized in humans, and the large variety of DNA replication and repair enzymes, only 30 distinct somatic mutation signatures have been defined from human tumor sequencing, and the etiology of more than half remains unknown(Petljak and Alexandrov 2016). Analysis of quantitative, high-resolution, and unbiased somatic mutation data has the best potential to link observed mutational spectra with molecular mechanisms. However, the distributed and asynchronous nature of mutations in somatic cells makes these mutations difficult to detect and accurately quantify. Specifically, published rates of somatic mutation vary widely from $10^{-11}$ to $10^{-7}$ single nucleotide variants (SNVs) per cell per cell division(Li et al. 2014; Lynch 2010; Ju et al. 2017; Holstege et al. 2014; Blokzijl et al. 2016; Milholland et al. 2017) due to differences across cell types and reliance on uncertain cell division rate estimates(Tomasetti et al. 2017). Without strong selection such as that occurring in tumor development(McGranahan and Swanton 2017), these somatic mutation rates result in variant allele frequencies lower than can be reliably detected in bulk samples using standard sequencing approaches, which produce consensus false positive SNV error rates of $10^{-6}$ to $10^{-5}$ per base.

Available approaches for somatic variant analysis have yielded valuable insights, but also have technical limitations that compromise quantitative mutation analysis. Accurate detection of rare alleles in bulk samples can be achieved by molecular consensus sequencing methods that use barcodes to track reads from individual input molecules, but this comes at the cost of added protocol complexity, a requirement for ultra-deep sequencing that makes genome-wide analysis challenging (Merkle et al. 2017; Martincorena et al. Science. 2015 Sep 25;349(6255):1483-9) and does not provide direct insight into intra-lineage structures or dynamics. For example, Martincorena et al. used computational approaches on big data sets to study mutations. To enrich samples for somatic variants, single cells can be isolated and cloned or processed for sequencing directly. These cells or clones are typically separated by a large and unknown number of cell division events, which contributes to uncertainty in mutation rate calculations and severely limits power to determine exactly when mutations arose within a lineage or correlations among the mutations (Szikriszt et al. 2016 Genome Biology 17:99; Blokzijl et al. 2016; Drost et al. 2017; Milholland et al. 2017). For example, Szikriszt et al. used random single cell expansion and analysis to detect mutations caused by cytotoxic agents, but the method does not allow lineage construction Direct single-cell methods do not require live cells or suffer from a selection bias against slower- or non-growing cells, but despite recent advances, remain compromised in the sensitivity and accuracy of variant detection compared with bulk approaches (Gawad et al. 2016; Chen et al. Science. 2017 Apr 14;356(6334):189-194; Lodato et al. 2015). For example, Chen et al. used sister cells for SNP correction in a single-cell whole genome amplification (WGA) method Mutation accumulation experiments that run for hundreds of generations or more to enrich lineages with large numbers of mutations are widely used for fast-growing bacterial cells (Tenaillon et al. 2016) but are impractical for mammalian cells. Other approaches for measuring mutation rates *in vitro* target specific genomic loci, which can lead to strongly biased estimates of genome-wide rates and genome-wide mutation spectra (Araten et al. 2005). Mutation analysis relying on powerful yeast genetics and tools not available in higher eukaryotes has been performed (Kennedy et al., PLoS Genet 2015 11(4): e1005151). (Genome Biology (2016) 17:99). Thus, there is a need for quantitative and accurate genome-wide somatic mutation analyses that are unbiased by positive or negative selection.

Bacteria have a relatively rapid mutation rate. Antimicrobial resistance (AMR) is the ability of a microbe to resist the effects of medication previously used to treat them. This broader term also covers antibiotic resistance, which applies to bacteria and antibiotics. Resistance arises through one of three ways: natural resistance in certain types of bacteria, genetic mutation, or by one species acquiring resistance from another. Resistance can appear spontaneously because of random mutations; or more commonly following gradual buildup over time, and because of misuse of antibiotics or antimicrobials. Resistant microbes are increasingly difficult to treat, requiring alternative medications or higher doses, both of which may be more expensive or more toxic. Microbes resistant to multiple antimicrobials are called multidrug resistant (MDR); or sometimes superbugs. Antimicrobial resistance is on the rise with millions of deaths every year. All classes of microbes develop resistance: fungi develop antifungal resistance, viruses develop antiviral resistance, protozoa develop antiprotozoal resistance, and bacteria develop antibiotic resistance. The increase in resistant strains include higher morbidity and mortality, longer patient hospitalization, and an increase in treatment costs. (B. Murray, New Engl. J. Med. 330: 1229-1230 (1994)). Thus, there is a need for novel methods to analyze mutations in bacteria with the accuracy and the time resolution to measure mutations with high accuracy and specificity.

WO2015013191 discloses a method for detecting and evaluating somatic mutations in patients tumour tissue during treatment by the use of archival tumor samples.

## SUMMARY

Somatic mutations steadily accumulate in our cells. Most somatic mutations are harmless, but some lead to a phenotypic consequence. It is an objective of the present invention to provide a somatic mutation analysis approach that has combined 1) the ability to identify groups of variants that arise during the replication of an individual cell, 2) high accuracy and sensitivity for genome-wide somatic SNVs, and 3) minimal positive and negative mutation selection biases. A further objective of the present invention to provide methods and tools to elucidate exactly how mutations appear in the genome. A further objective of the present invention is to provide methods and tools to determine how mutations are correlated in space and time. For example, a mutation may occur in one generation that effects the mutation rate in downstream generations. A further objective of the present invention is to provide a quantitative model for neutral mutations in order to describe the selective forces that shape genomes. A further objective of the present invention is to provide for determining the effect of environmental stimuli and drugs on somatic mutations. It is another objective of the present disclosure to provide for personalized medicine by determining the effect of treatments on somatic mutations in cells obtained from a subject in need thereof. It is another objective of the present invention to provide for lineage sequencing of bacteria to detect mutations.

The methods of lineage sequencing of the present invention work in a controlled genetic background and uses expectations about inheritance states, such that every lineage of the pedigree is processed, enabling reconstruction of the entire history of mutations in the clonal population of cells, suppression of sequencing error, and highlighting of rare variants. The dataset obtained is "single-cell resolved" in this sense, with single generation resolution of mutations occurring during the growth of the initial population, and the data is extraordinarily accurate, far superior to standard sequencing approaches due to error correction enabled by the unique structure of lineage sequencing data. The unique data structure also enables determination of correlations among mutation rates along the genomes (after a single-cell replication event) and through time that cannot be observed using conventional approaches at any sequencing effort level.

In one aspect, the present invention provides for a method of detecting somatic mutations across a single cell lineage comprising: isolating single cells from a clonal population, each single cell representing a lineage segment of the clonal population; sequencing genomic DNA representing each single cell; and determining true somatic mutations along the single cell lineage of the clonal population based, at least in part, on the sequencing of the genomic DNA representing each single cell, wherein true somatic mutations are determined by tracing the mutations across the cell lineage for each generation and wherein each mutation is observed in at least two cells in the lineage. As used herein, the terms "true variants" or "true somatic mutations" refer to mutations detected by sequencing that are not the result of sequencing errors. In certain embodiments, the method further comprises culturing a sub-clonal cell population from each single cell representing a lineage segment and sequencing genomic DNA from each sub-clonal cell population. In certain embodiments, the method further comprises whole genome amplification (WGA) and sequencing amplified DNA from each single cell. In certain embodiments, the determining step comprises generating a lineage structure of the clonal population that identifies and maps the origin of variants within the clonal population. In certain embodiments, the clonal population is derived from expanding a single parent cell, preferably a eukaryotic cell. In certain embodiments, the clonal population is expanded for at least 2 generations, more preferably, between 5 and 6 generations. In certain embodiments, the single parent cell may be expanded for 2 to 10 generations. In certain embodiments, the method comprises: expanding a single eukaryotic cell into 2 or more generations; isolating single cells from the expanded cells; culturing the single cells, whereby a colony for each cell is obtained; sequencing genomic DNA from each colony; and determining true somatic mutations along the single cell lineage.

The true somatic mutations are determined by tracing the mutations across the cell lineage for each generation. Each mutation are observed in at least two cells in the lineage. The method may further comprise detecting a mutation signature. The method may further comprise determining a mutation rate. The method may further comprise determining the set of mutations that occurred in individual cell cycles in the lineage. The method may further comprise clustering of mutations in the genome in an individual cell cycle. The method may further comprise determining relationships in the number, type, spectrum, clustering and/or overlap of mutations in mother and daughter cells and/or along a sub-lineage.

In certain embodiments, the clonal population is derived from expanding a single parent cell exposed to or under exposure to one or more perturbations. The cell(s) may be exposed to the one or more perturbations prior to expanding and/or during the step of expanding. The cells may be exposed to a perturbation before expanding or both before and during expanding. The perturbation may be an environmental condition, a drug, or an agent capable of modulating expression of a gene. The environmental condition may be physical (e.g., temperature, atmospheric pressure, pH, growth media conditions, sheer stress,), chemical (e.g., carcinogen), or biological (e.g., cytokine, pathogen). The perturbation may also be a genetic perturbation of a coding on non-coding genomic region. The agent capable of modulating perturbation of a gene may be a CRISPR system, RNAi, TALE, or zinc finger protein. The agent may be inducible, such that the timing of perturbation may be controlled.

The method according to any embodiment herein may be performed in an automated device. In certain embodiments, the step of isolating single cells from a clonal population is performed in an automated device. In certain embodiments, the step of expanding a single parent cell is performed in an automated device. Automated device as used herein includes semi-automated devices. The device may be operably linked to a computing system.

In certain embodiments, the single cell may be loaded into a microfluidic device configured for segregation of single cells across the cell lineage. In certain embodiments, the single cells may be isolated with an optical tweezer. In certain embodiments, the single cells may be segregated into separate wells. In certain embodiments, expanding a single parent cell is performed on a microscope slide. In certain embodiments, the step of isolating single cells from a clonal population is performed on the microscope slide. The slide may be configured for cutting with UV light. The slide may be a polyethylene-naphthalate (PEN) membrane slide. Isolating single cells from the expanded cells may comprise laser microdissection. The cells may be captured on a sticky cap tube. The cells may be captured with catapulting.

In certain embodiments, methods for isolating single cells may comprise a combination of the methods described above. In certain embodiments, the expanding and isolating may be visually recorded, whereby single cells across are lineage are tracked. In certain embodiments, the sequencing may be whole genome or whole exome sequencing.

In certain embodiments, the single parent cell is a eukaryotic or bacterial cell. In certain embodiments, the single cell is obtained from a subject in need thereof. The single cell may be a stem cell or induced pluripotent stem cell (iPS).

In certain embodiments, the method further comprises detecting phenotypes during the step of expanding a single parent cell. In certain embodiments, more than one parent cell is expanded and the method further comprises selecting specific cells and/or sub-lineages for genome sequencing based on the observed phenotypes.

In another aspect, the present invention provides for a method of detecting mutations in bacteria during single replication events comprising: expanding a single a non-eukaryotic cell into 2 or more generations; segregating single cells from the expanded cells; culturing the single cells, whereby a colony for each cell is obtained; DNA sequencing each colony; and determining true mutations along the single cell lineage, wherein true mutations are determined by tracing the mutations across the cell lineage for each generation and wherein each mutation is observed in at least one pair of daughter cells. The cell may be expanded into 2 to 10 generations. The single cell may be exposed to a perturbation during the step of expanding into 2 or more generations. The perturbation may be an environmental condition, a drug, or an agent capable of modulating expression of a gene. The environmental condition may be physical, chemical, or biological. The drug may comprise an antibiotic, whereby mutations in response to the antibiotic are detected in single replication events. The method may further comprise detecting a mutation signature.

The single bacterial cell may be obtained by diluting a sample of bacteria. The single bacterial cell may be obtained by sorting a sample of bacteria. The single bacterial cell may be obtained by separation with an optical tweezer and live single cell microscopy.

The single bacterial cell may be loaded into a microfluidic device configured for segregation and recovery of single cells across the cell lineage. The single bacterial cells across a lineage may be segregated on a chip and expanded. The single bacterial cells across a lineage may be segregated into separate wells and expanded.

The method may further comprise determining the growth rate of the isolated single bacterial cells across a lineage. The growth rate may be determined in the presence of an antibiotic. The antibiotic may be in a low dose; such that resistant cells may be detected or growth rates of resistant and sensitive cells compared.

The DNA sequencing may comprise loading the bacterial cells from a cell lineage into a microfluidic device capable of segregating each colony and generating a sequencing library for each colony.

The single bacterial cell may be obtained from a subject in need thereof (e.g., a subject with an infection). The single bacterial cell may be obtained from an environmental sample.

These and other aspects, objects, features, and advantages of the example embodiments will become apparent to those having ordinary skill in the art upon consideration of the following detailed description of illustrated example embodiments.

**BRIEF DESCRIPTION OF THE DRAWINGS**

**FIG. 1** - shows a lineage sequencing approach using amplification by sub-clonal culture (left). The differences in false-positive rate and false-negative rate is shown in relation to concordance (right).

**FIG. 2** - shows a schematic representation of an exemplary method for somatic mutation detection. A single primary cell is expanded a few generations. Single cells are isolated into separate wells. The cells are recovered and cultured prior to sequencing. Errors are corrected by requiring that mutations be observed in a pair of daughter cells (i.e., concordance).

**FIG. 3 -** shows a pipeline for data analysis of lineage sequencing to detect mutations.

**FIG. 4** - shows that the data analysis allows 91.4% coverage of the human genome and can be used to filter candidate mutations.

**FIG. 5** - shows implementation of lineage sequencing using a microfluidic device with HT115 cells. The HT115 cells labeled (light shading) were successfully sub-cloned.

**FIG. 6** - shows the accumulation SNP mutations along 5-6 generations from the HT115 cell line (mutations per generation: mean = 169, SD = 27).

**FIG. 7** - shows the mutation signature in the HT115 cell line.

**FIG. 8** - shows the accumulation SNP mutations along 5-6 generations from the RPE1 cell line (mutations per generation: mean = 43, SD = 23).

**FIG. 9** - shows a comparison of mutation signatures between HT115 and RPE1 cells.

**FIG. 10** - shows that mutations distribute randomly across major genomic features in HT115 and RPE1 cells.

**FIG. 11** - shows that lineage sequencing allows construction of trees without prior knowledge of lineage structure.

**FIG. 12** - shows that late replication timing is associated with higher mutation rate in HT115 cells using lineage sequencing.

**FIG. 13** - shows a microfluidic device for integrating whole genome sequencing (WGS) sample preparation.

**FIG. 14** - shows clinical isolate sequencing and WGS library preparation of 384 clinical isolates.

**FIG. 15** - shows analysis of bacterial isolates using the microfluidic device of figure 13.

**FIG. 16** - shows that low-input environmental samples may be analyzed using the microfluidic device of figure 13.

**FIG. 17** - shows lineage sequencing (or pedigree sequencing) of bacteria.

**FIG. 18** - describes a solution to the problem of measuring all modes of evolution.

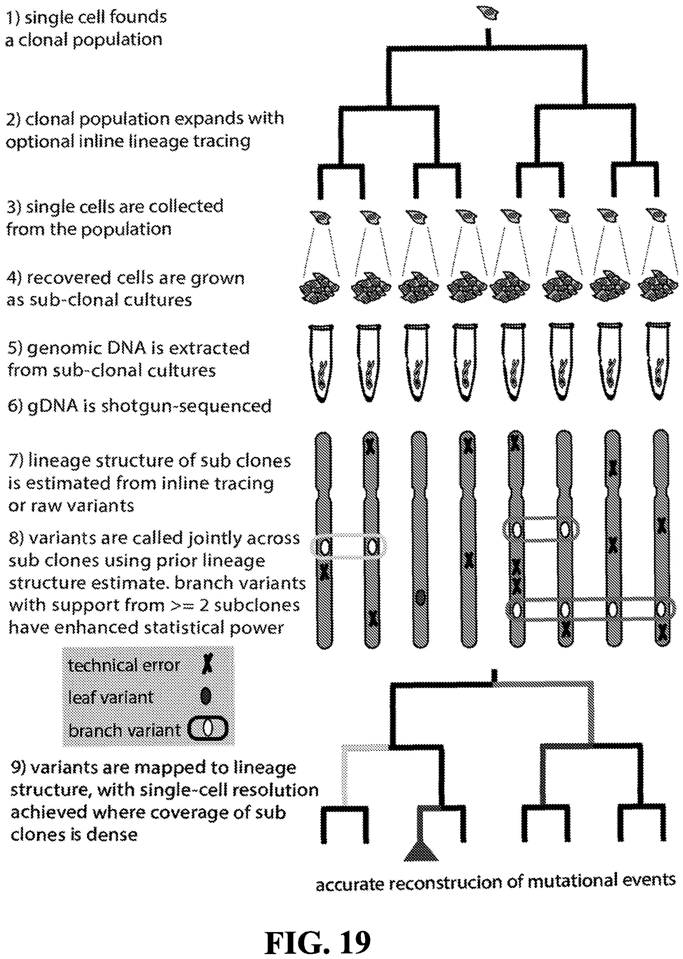

FIG. 19 - **Lineage sequencing concept and implementation.** Overview of lineage sequencing concept. Numbering indicates key conceptual steps and major steps in the implementation in this manuscript. Single cells are sampled from a clonal population and sequenced (steps 1-6; in this study sub-clonal culture is used to produce enough genomic DNA for PCR-free shotgun sequence library construction). Crucially, a prior estimate of the population lineage structure (step 7; either from single cell tracking by time-lapse imaging or from raw SNV calls) is used to identify novel somatic variants in a joint analysis of the sequence libraries (step 8). This use of the lineage information enables multiple sequence libraries to provide statistical support for somatic "branch variants", enhancing the sensitivity and specificity of somatic variant identification (for example, one schematically indicated "variant" is supported by four sequence datasets, and one "variant" is supported by two datasets). Where the coverage by sampled lineages (via sub clones in this study) is high, the mutations that appeared during the lineage experiment can be mapped with single cell resolution (step 9; e.g. segments in the dendrogram at bottom). Applicants term mutations occurring in the last round of cell division events "leaf variants", which by definition are supported by only a single sequence dataset. Leaf variants can also be analyzed but do not benefit from the enhanced statistical power that supports branch variants, and thus are cannot be reliably assigned to specific cellular events.

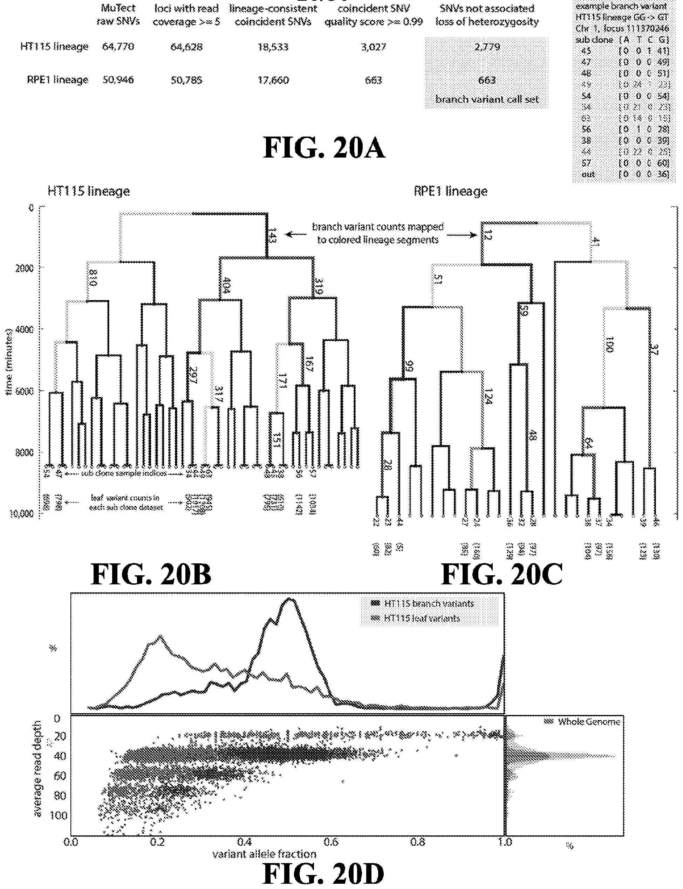

FIG. 20 - **HT115 and RPE1 lineage sequencing experiments by the "lineage -> called variants" approach. Figure 20a.** Scheme of the analysis pipeline for identifying branch variant SNVs by the "lineage -> called variants" approach. "Branch variants" are SNVs that occur at the same locus in two or more (but not all) sub clones and are consistent with prior lineage information. Variant counts at different stages of the informatics filtering steps used to identify high quality lineage structure concordant branch variants are shown for the HT115 and RPE1 lineage sequencing experiments. Detail on an example HT115 GG -> GT branch variant is shown. Allele counts from sequence reads at chromosome 1 diploid locus 111370246 are shown. Four sub clones sequence libraries (sub clone indices 49, 34, 63 and 44, marked in red color) show about half the reads indicating a variant T allele, where all the other sub clones support only the reference G alleles at this locus. This G -> T SNV is scored as one of 404 branch variants that appeared within the two cell cycles represented by the pink segment on the righthand side of the dendrogram representing the HT115 lineage experiment in part b. **Figure 20b and c.** Dendrograms representing the HT115 and RPE1 lineage experiments annotated with the index numbers of sequenced sub clones, the count of branch variants for resolved lineage segments (resolved to individual cell lifetimes in some cases), and the count of leaf variants for each sequenced sub clone (at bottom). "Leaf variants" are SNVs that are supported by only one sub clone and likely represent variants that occurred during or after the last generation of the lineage experiment. The x-axis of the dendrogram only shows linkage of the sub clones. The y-axis of the dendrogram represents the culture time course, with each cell division event observed by time lapse imaging marked by a branch point in the dendrogram. Single cells were recovered for sub culture from the HT115 lineage after 141 hours while cells were recovered from the RPE1 lineage after 168 hours. **Figure 20d.** HT115 branch variants are clonal. Histogram of allele fraction for detected variants. Comparison between branch variants (mutations occurring during lineage formation up to the last cell division) and leaf variants (mutations occurring within or subsequent to the last cell division event in the lineage). Branch variant SNVs show a bimodal allele fraction distribution peaked at 0.5 and 1.0 as expected for the measured ploidy (CNV analysis) at variant loci in this mostly diploid cell line. By contrast, sub-clonal mutations appear in the leaf variant group and show an allele fraction distribution peaked well below 0.5 as MuTect attempts to balance sensitivity for low allele fraction variants with false positive detections.

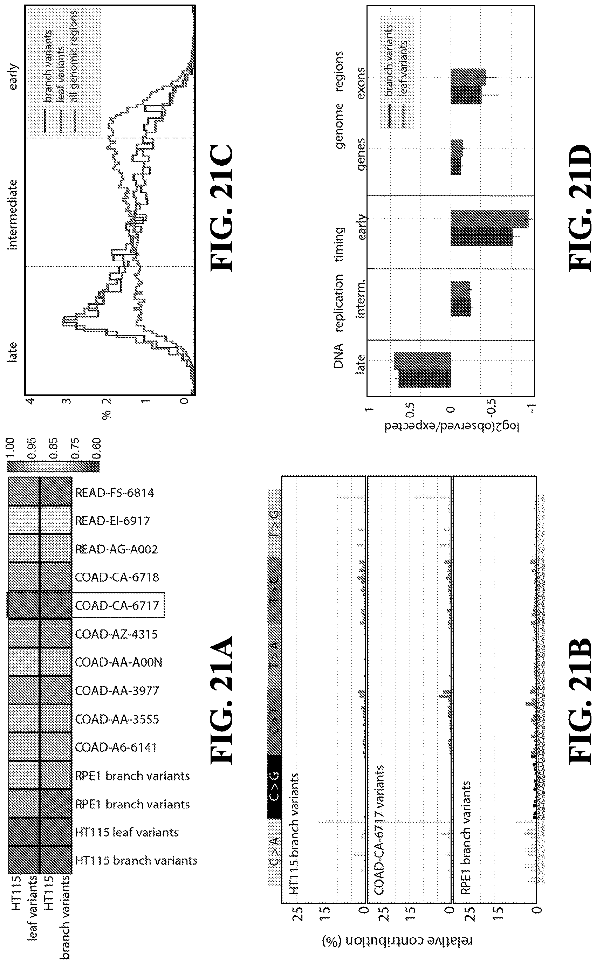

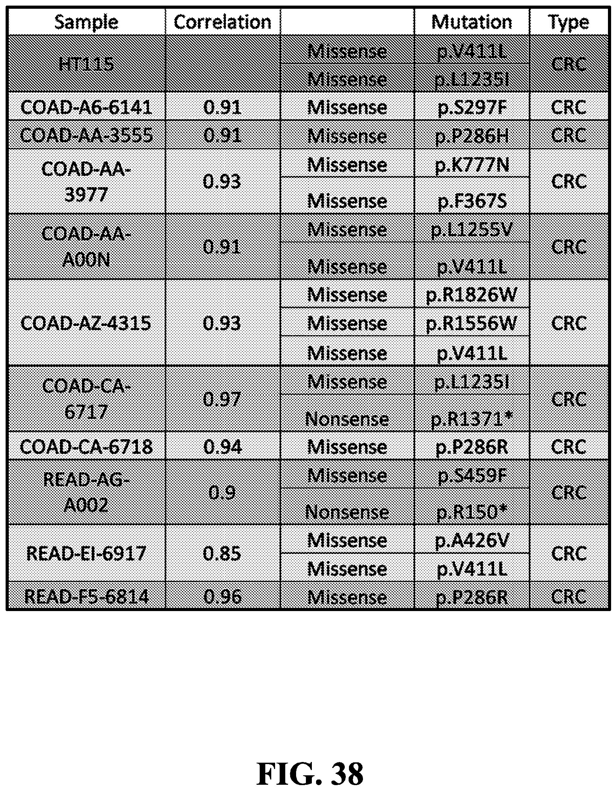

FIG. 21 - **Analysis of mutational patterns in human colon carcinoma epithelial cell line HT115 show POLE proofreading deficient behavior corresponding to previously published bulk POLE mutant colon tumor sample data. Figure 21a.** Heatmap showing the cosine similarity scores of comparisons of whole-genome HT115 variant SNV mutational spectra with whole-genome spectra from RPE1 samples and published data sets from POLE mutant tumor samples (Cancer Genome Atlas (TCGA), dbGAP: phs000178.v1.p1; sample annotations in Figure 38). The blue rectangle denotes the most similar tumor sample (COAD-CA-6717). **Figure 21b.** Comparison of detailed mutational spectra of all base substitutions observed in HT115 and RPE1 cell line branch variants and in the COAD-CA-6717 TCGA sample. HT115 and COAD-CA-6717 show highly similar spectra that differ from the RPE1 spectrum. **Figure 21c.** Distribution of DNA replication timing for all genomic positions and the somatic SNV branch variants and leaf variants from HT115 cell line. Both the branch and leaf variant sets show the expected enrichment in late-replicating regions and depletion in early-replicating regions versus the background distribution of replication timing at all genomic loci (red). **Figure 21d.** Quantification of the enrichment and depletion of SNVs in the indicated categories. SNVs are enriched in the late replicating regions while SNVs are depleted in RefSeq genic regions and further depleted in RefSeq exons (P<<0.01) in both the branch and leaf variant SNV sets.

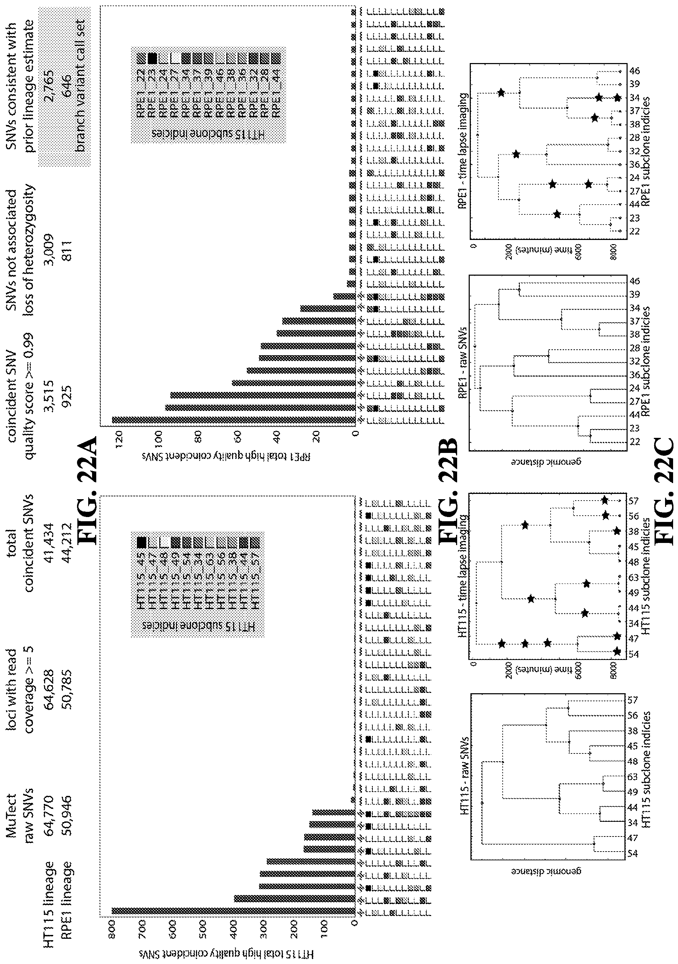

FIG. 22 - **Accuracy and sensitivity of lineage sequencing without microscopic tracking. Figure 22a.** Scheme of the analysis pipeline for identifying branch variants by the "Raw variants -> Lineage -> called variants" approach. Variant counts at different stages of the informatics filtering steps used to identify high quality lineage structure concordant branch variants are shown for the HT115 and RPE1 lineage sequencing experiments. The pipeline is similar to the Lineage -> variants pipeline (Figure 20a) except where lineage information is incorporated later, separately from SNV coincidence, and the source of the prior lineage estimate is analysis of raw SNVs (see parts b and c) rather than time lapse imaging. **Figure 22b.** Histogram of the number of high quality coincident SNVs for each set of sub clones in which such variants occurred for the HT115 (left) and the RPE1 (right) datasets. At bottom, each cluster is marked if consistent (+) or inconsistent (-) with the lineage structure indicated from the time lapse imaging. For each cell line, the group of sub clone sets with high frequencies of these SNVs are both internally self-consistent and consistent with the independent time lapse imaging data. **Figure 22c.** Comparison between dendrograms representing the HT115 and RPE1 lineages based on genomic distance among sub clone pairs and the time-lapse imaging data (for simplicity, only sub clones that were cultured and sequenced are represented). The round markers at dendrogram branch points and stars represent cell division events. The analyses based on genomic distance and time-lapse imaging indicate the same connectivity between sub clones, the information relevant to joint variant calling in lineage sequencing, but have different branch lengths, and lack full information on cell division events. The dendrograms based on time-lapse imaging show time-to-division in minutes, an example of a cellular phenotype that depends on direct observation of cells during the lineage experiment.

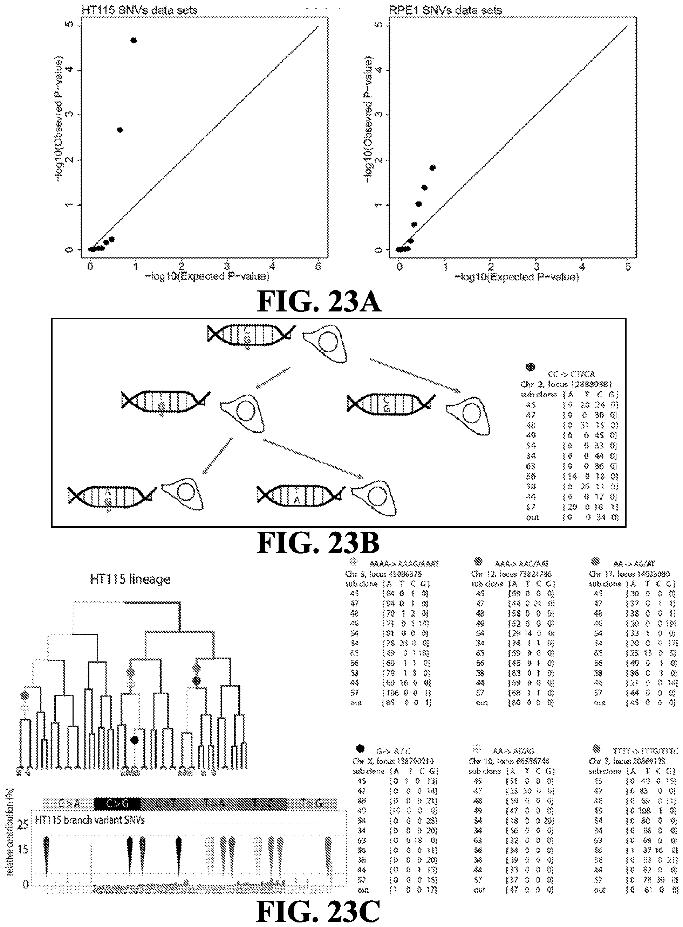

FIG. 23 - **Intra-lineage heterogeneous in mutation rate and multiple mutation events. Figure 23a.** Measured P-values from observed count are plotted vs calculated theoretical (Poisson) P-values for the branch variant set for each sub-lineage to form quantile-quantile plots (P-values for theoretical Poisson-distributed lineage-wise mutation count data versus observed data) for both HT115 (left) and RPE1 (right). The plotted points deviate strongly from the expected distribution (which would follow an x=y relationship) at both ends of the distribution, showing that the sub-lineages present in each dataset cannot be plausibly modeled by a Poisson distribution based on uniform mutation probability. **Figure 23b.** Scheme of persistent lesion mechanism for correlated mutation. DNA lesions (marked as **G\***) that are not repaired during S-phase compel the DNA polymerase to copy damaged DNA and replicate opposite lesions with a high probability of mutation. If the lesion has not been repaired before the next S-phase in the daughter cell carrying the lesion, an additional mutation at the identical genomic locus is likely to result. In case that the second mutation is different, this process can be readily detected. The example scheme represents the CC>CT and CC>CA mutations Applicants detected at the chromosome 2 locus 128889581 (marked with circle).

**Figure 23c.** Multiple mutation events found in the HT115 lineage. Read counts for each example are presented and marked with a symbol. The lineage segment where each example occurred is shown with shading. None of these events overlap the most probable mutation types found in the POLE signature.

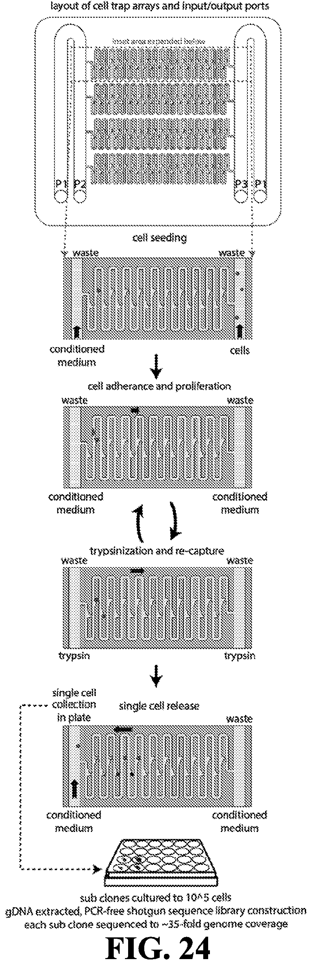

FIG. 24 - **Microfluidic system workflow for seeding, culturing, and collecting cells.** Diagram of the device microarchitecture including cell trap arrays and ports used to introduce buffers and cell culture medium. Flow across the cell trap arrays is used to manipulate cell position. The cell trap arrays are flanked on either side by bypassing fluidic channels which enable rapid fluidic exchange and media perfusion through the device while limiting the amount of shear forces experienced by captured cells. The inset diagrams show the series of operations used to seed a cell into a trap array which allow the cell to adhere to the device, proliferate, trypsinize, distribute, re-capture cells and release cells individually from a cell trap array for collection in standard laboratory plasticware for sub-clonal culture. Together, the device features and this protocol allow for long-term culture and manipulation of cells under time lapse imaging prior to isolation of single cells for downstream culture and analysis by lineage sequencing.

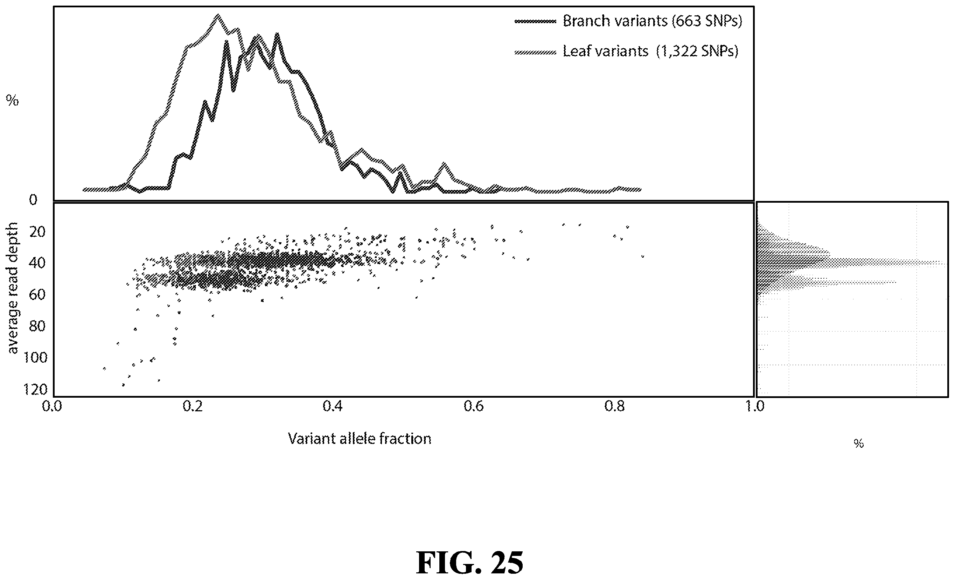

FIG. 25 - **RPE1 branch variants mutations are clonal. Upper panel:** Histogram of allele fraction distribution for RPE1 SNVs. Comparison between 'branch variants' and 'leaf variants'. Branch variants are clonal, with the branch variant allele fraction centered near a value of 0.33 as expected for the predominantly triploid RPE1 cells. Similarly, to the HT115 cells, RPE1 leaf variants show a shift to lower allele fraction values. **Lower panel:** scatter plot of variants; average read depth versus allele fraction; branch variants (blue), and leaf variants (green). On the right panel: normalized histogram of read coverage depth for RPE1 lineage; whole genome, branch variants, and leaf variants.

**FIG. 26 - Genomic DNA copy number measurements.** Copy Number measurements for the HT115 cell line shows that the genome is mostly diploid (upper plot), while the RPE1 cell line presents mostly triploid and tetraploid genome (bottom plot). The copy numbers values were calculated for every sample using the whole genome sequencing data (WGS), and the locus-wise copy number estimate used to inform SNV analysis.

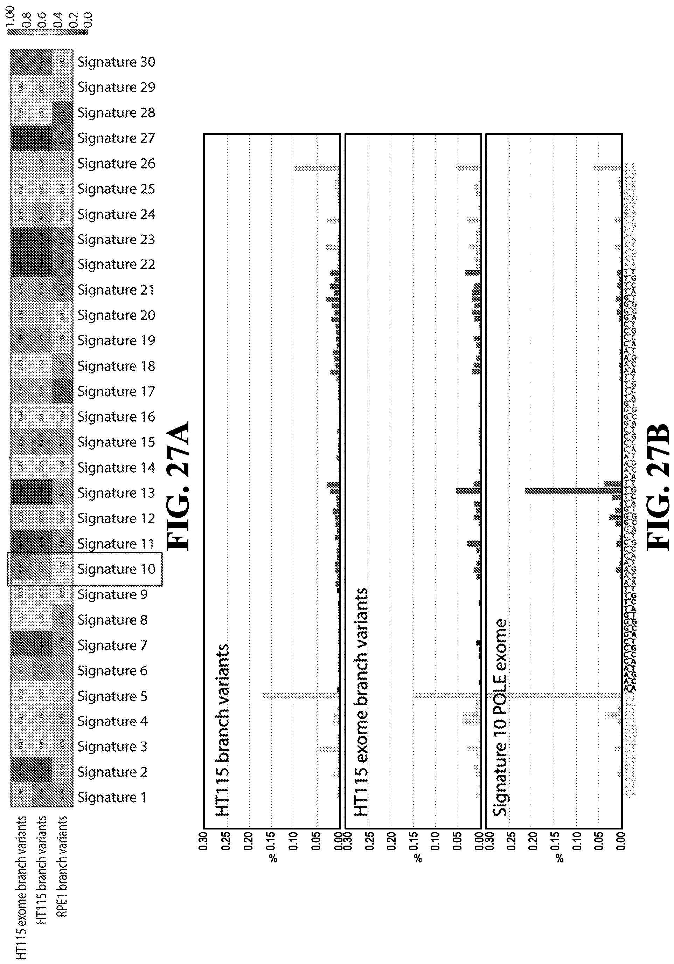

**FIG. 27 - POLE signature of mutational process similar to HT115 mutational spectra. Figure 27a.** Heatmap showing the cosine similarity scores between SNV mutational spectra of HT115 branch variants from whole-genome spectra, the derived whole exome spectra, and RPE1 mutational spectra with the COSMIC set of mutational signatures (derived from whole exome sequencing)(Forbes et al. 2015). The rectangle denotes the most similar signature for HT115, the POLE signature with cosine similarity = 0.79 (+/-0.02) to whole genome data and 0.85 (+/-0.04) to the whole exome SNVs. **Figure 27b.** Comparison of detailed mutational spectra of all base substitutions observed in HT115 branch variants from whole-genome spectra and the derived whole exome spectra and the POLE signature.

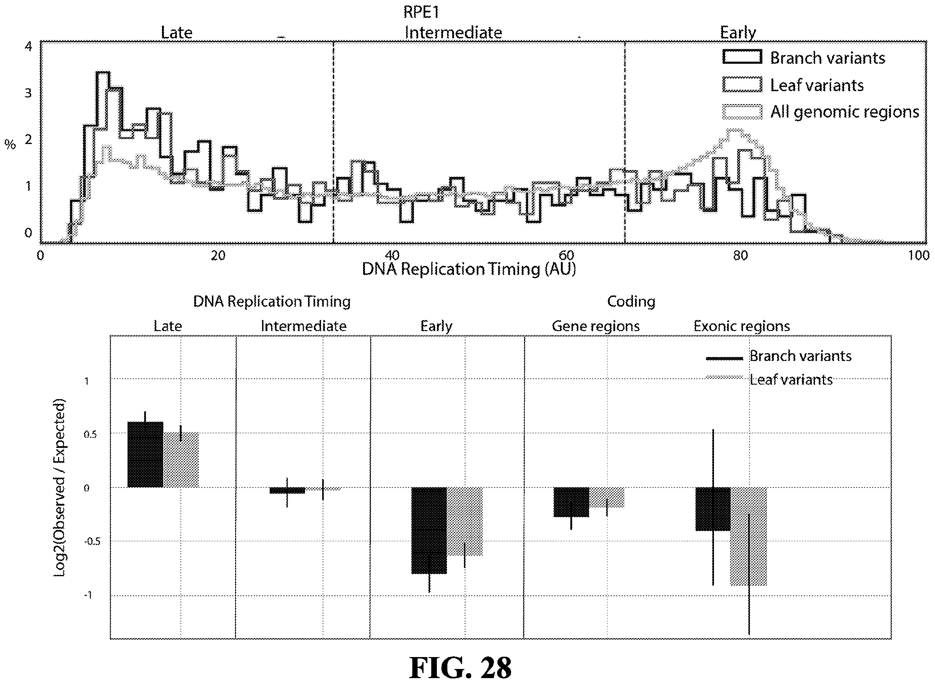

**FIG. 28 - RPE1 cells show unequal distribution of mutation rates across the** genome. Figure 28a. Distribution of the general DNA replication timings for RPE1 branch variants and leaf variants (blue and green respectively) for early- and late-replicating regions (p < 0.01 calculated by binomial test of observed vs expected ratio). Figure 28b. Enrichment and depletion of SNVs in the indicated genomic regions, assessed as the log2 ratio versus the genome-wide average. Mutations are enriched in the late replicating regions and depleted in the early replicating regions. Genomic regions (RefSeq gene regions - exons introns and UTRs) and also exonic regions (exons only) show depletion (p < 0.01) in both branch and leaf variants. Error bars were calculated by bootstrapping the data $10^4$ times and represent 95% confidence intervals.

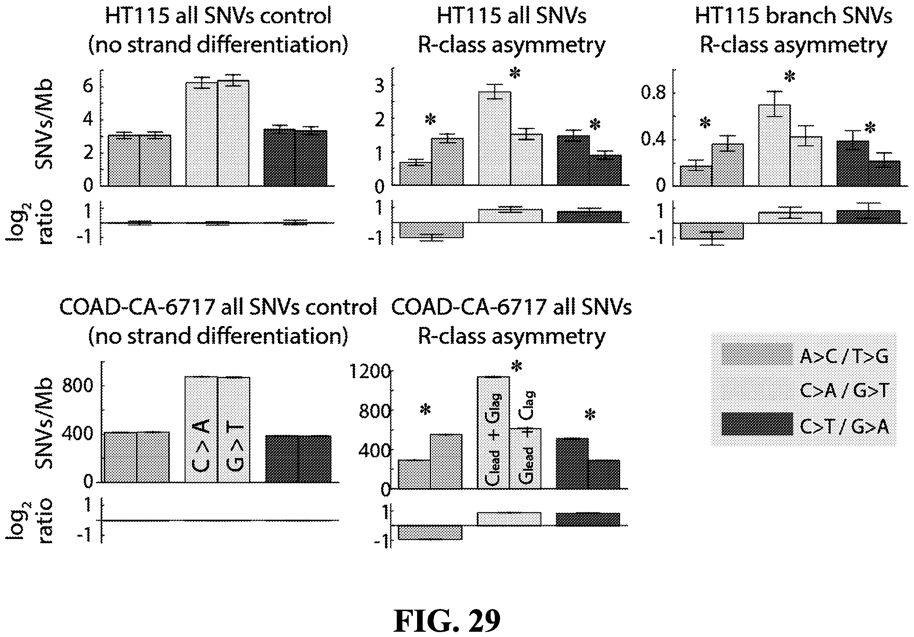

**FIG. 29 - POLE strand asymmetries are evident in the samples:** Applicants detected strand asymmetry associated with DNA replication ("R-class" asymmetry) in the HT115 andCOAD-CA-6717 TCGA variant sets. The left plots represent controls calculated by summing all mutations (with no differentiation of the replicated strand) and show a lack of asymmetry when the orientation of DNA replication is ignored (P<<0.01). R-class asymmetry consists of a skew based on the direction of DNA replication. For example, a skew toward C>A mutations in left-replicating regions where the strand synthesized by leading strand synthesis is the genomic reference. In right-replicating regions, a skew toward G>T mutations is seen where the genomic reference strand is synthesized by lagging strand synthesis. For each of three substitution groups (A>C/T>G, C>A/G>T, C>T/G>A) there are four options, for example 1) C>A in left-replicating regions 2) C>A in right-replicating regions 3) G>T in left-replicating regions and 4) G>T in right replicating regions. Left bars in each pair of bars in the top portion of plots (SNVs/Mb) show the sum of options (1) and (4) since they are both C>A with respect to the leading strand template. The right bars show the sum of (2) and (3) (since they are both G>T with respect to the leading strand template). The log2 ratios of these paired bars are shown in the bottom portion of each plot and represent the effect size of the enrichment or depletion for each substitution as assessed by the R-class asymmetry. Significant R-class asymmetry signal was observed in the total HT115 SNV set, the HT115 branch SNV set, and the COAD-CA-6717 TCGA sample SNV set.

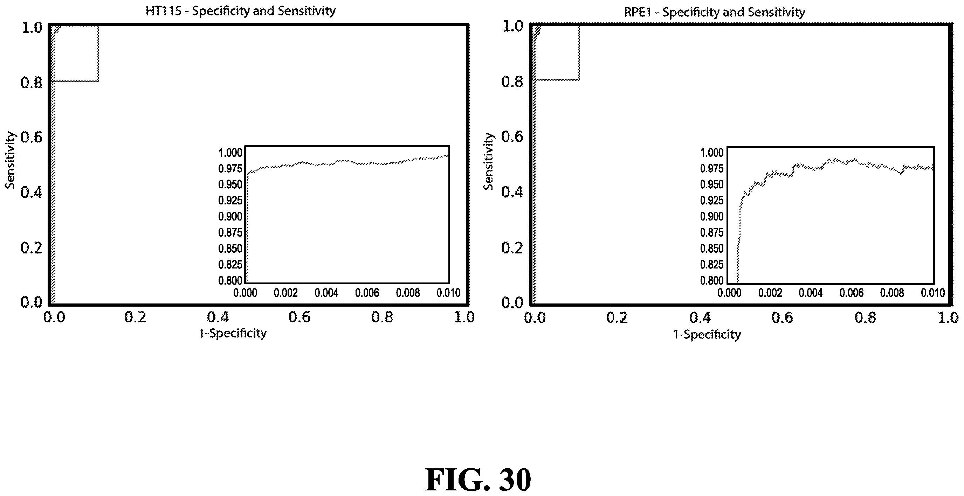

FIG. 30 - **Estimation for specificity and sensitivity of the branch SNV calls.** Applicants calculated the sensitivity and specificity for HT115 and RPE1 lineage sequencing. By using the 'variants -> lineage' approach together with the microscopic derived lineage structure Applicants were able to estimate the sensitivity and specificity of the lineage sequencing method. False positive calls were calculated by counting branch variant SNVs that appear as positive branch variants after scrambling the data, and false negative calls were estimated by counting branch variants with lower than threshold quality scores minus the false positive of these threshold values (methods).

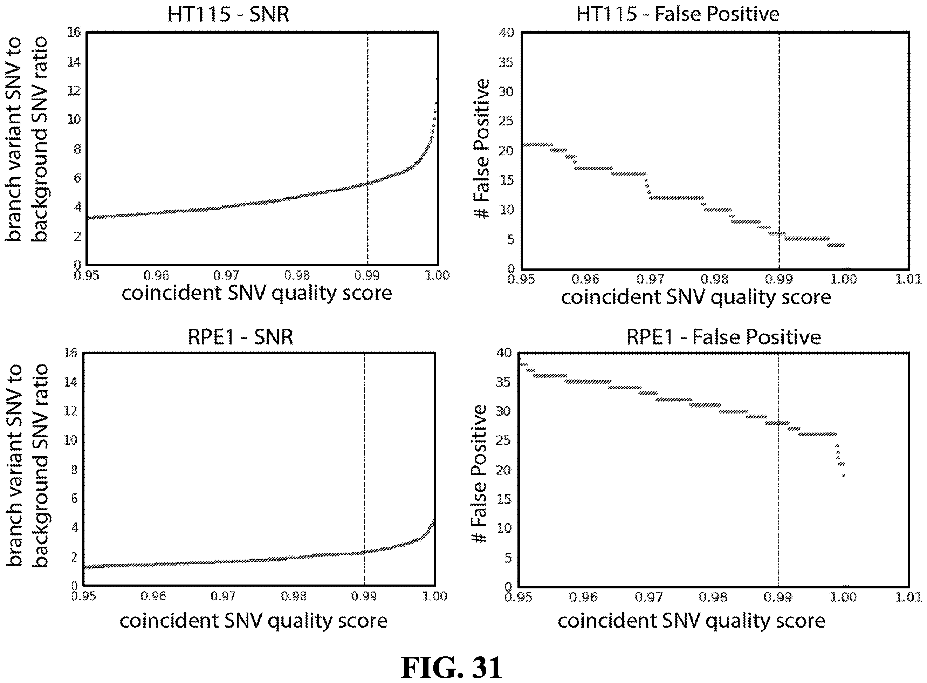

FIG. 31 - **Coincident SNV quality filtering for branch variant calling.** Branch variants are called from the set of SNVs that coincide at the identical genomic locus across multiple sub clones. Left plots: ratio of called branch variants consistent with consensus lineage structure to all other SNVs as the quality score for coincident SNVs was adjusted for the HT115 dataset (top) and the RPE1 dataset (bottom). A quality score threshold of 0.99 was applied to call branch variants in both datasets. Right plots: Estimating false positive branch variant counts for the HT115 and RPE1 datasets by counting the "branch variants" in the sub clone label-scrambled datasets as a function of the branch variant quality score threshold.

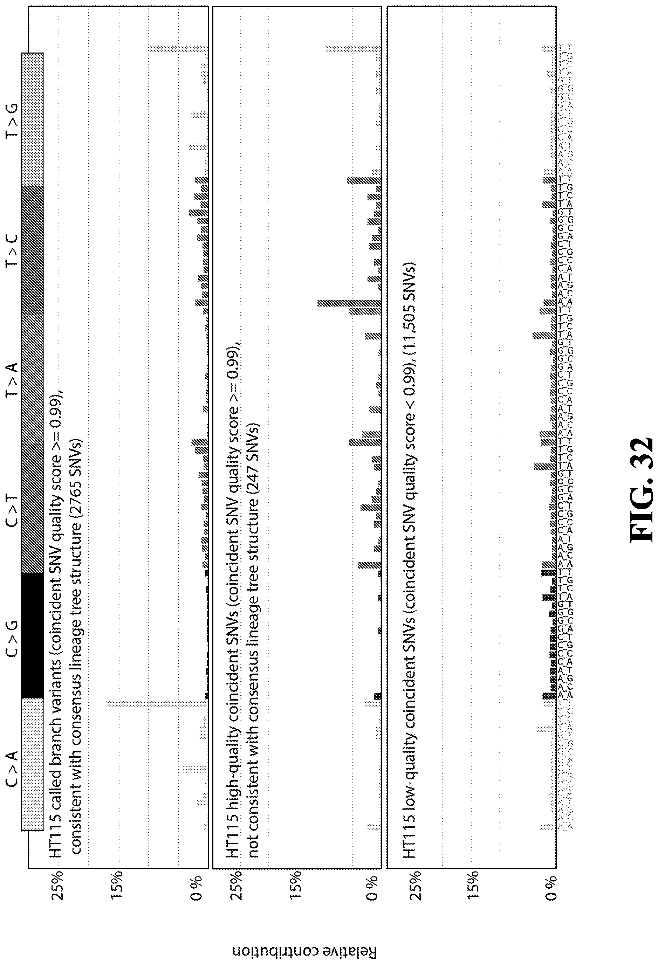

FIG. 32 - **Mutational spectra of filtered coincident SNVs differ from spectrum of called branch variants.** HT115 called branch variants (top plot, coincident SNV quality score >= 0.99 and consistent with the prior lineage estimate) show a mutational spectrum that matches reference POLE mutant whole-genome spectrum (not shown here). This called branch variant spectrum differs from the mutational spectrum of high quality coincident SNVs (middle plot, quality score < 0.99 not consistent with prior lineage estimate) and also the mutational spectrum of low-quality coincident variants (bottom plot, quality score < 0.99).

FIG. 33 - **HT115 lineages show similar branch variant mutational spectra.** Heatmap showing the cosine similarity score matrix across the mutational spectra of each sub-lineage in the HT115 experiment (which has high enough variant counts to support this analysis) and the complete branch and leaf variant call sets. The high similarity score matrix indicates the high specificity of these data and the consistency of the mutational spectrum across the HT115 lineage.

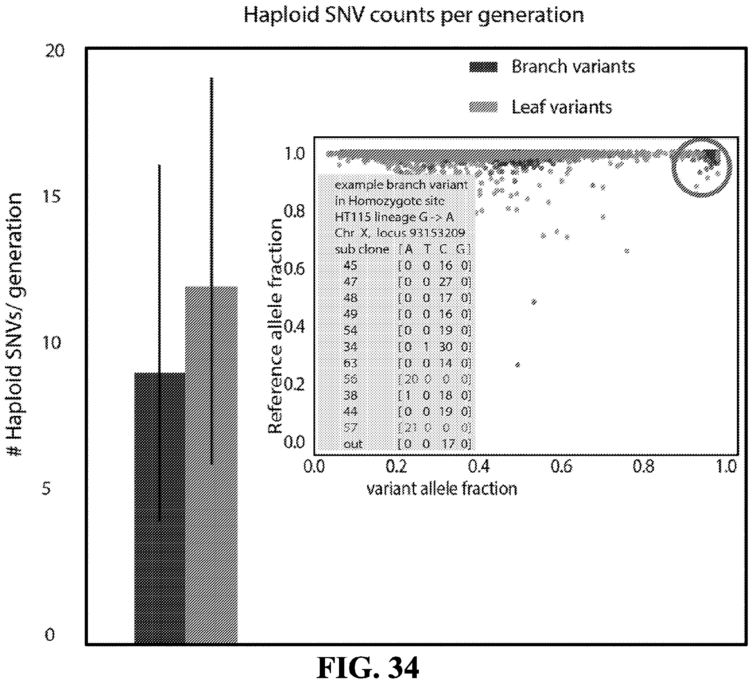

FIG. 34 - **Mutation rate at haploid loci can be measured using leaf variants.** Branch variants in the predominantly diploid HT115 cell line cluster at alternative allele fractions 0.5 and 1.0. Loci with both reference allele fraction and alternative allele fraction near 1.0 represent haploid sites. At these sites (circle), there is enhanced confidence that not only branch variants, but also that leaf variants are highly enriched for true variants. This is because the range of noise sources that produce errors in the leaf variant call set are unlikely to produce such a high alternative allele fraction. An example of one such specific leaf variant at one site on the haploid X chromosome with both reference allele fraction and alternative allele fraction near 1.0 is shown. This example shows a C to A mutation that appears only in sub clones 56 and 57. The accuracy of this leaf variant subset enables estimation of the mutation rate at haploid sites using leaf variants despite the fact leaf variants do not benefit from the suppression of false-positive variant calls in the branch variant call set resulting from the use of a prior lineage estimate. The number of branch and leaf variant SNVs is shown in the bar plot (error bars represent 95% confidence intervals calculated using the Poisson-distributed count approximation).

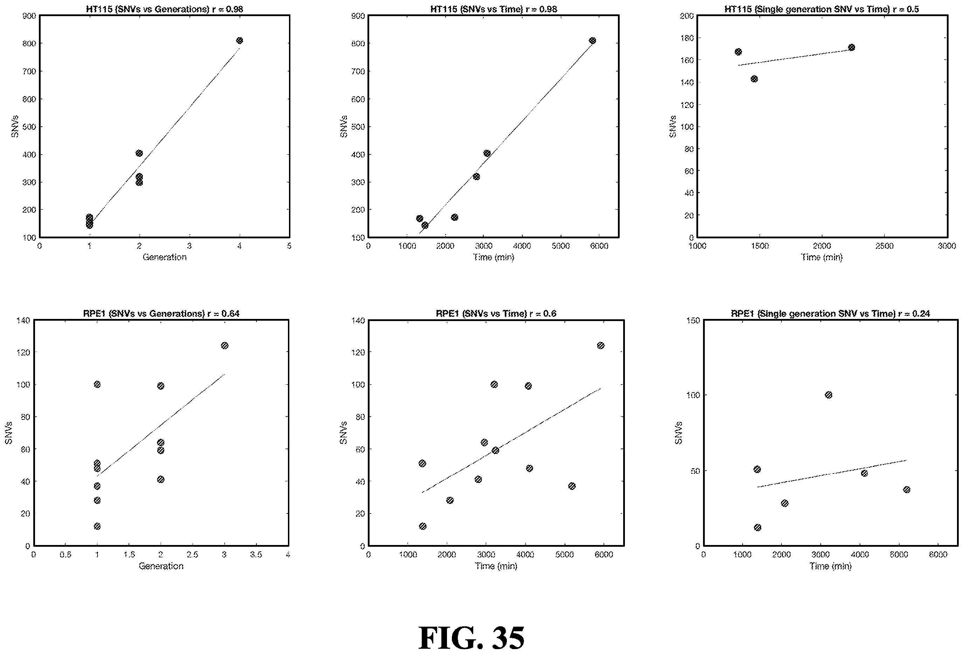

FIG. 35 - **Correlation of mutation count versus observed generation number and time to division.** Upper row presents results for HT115 cells; lower row presents results for RPE1 cells. Left, branch variants SNVs versus number of generations (or cell divisions); center, versus time for all non-overlapping lineage segments; right, versus time for single generation segments only. RPE1 (lower line) shows lower mutation counts and correlation coefficients for mutation counts versus time to division and generation number: $((\rho(mut,generations) = 0.636)$ and for times $((\rho(mut,times) = 0.602)$. HT115 cells have higher mutation counts and better-synchronized replication, resulting in higher correlation coefficients versus time to division and generation number: $(\rho(mut,generations) = 0.978)$, $(\rho(mut,times) = 0.984)$.

FIG. 36 - **multiple mutation events, RPE1 cells. Figure 36a.** The four multiple mutation events detected in RPE1 cells: the genomic location of each SNV and allele counts. The shaded dot on the upper left side corresponds to the shaded markers in parts b) and c). **Figure 36b.** The time occurrence for each SNV plotted on the lineage tree structure with resolved segments indicated. **Figure 36c.** Plot marking spectral location of RPE1 multiple mutation events in the context of the overall mutational spectrum for RPE1.

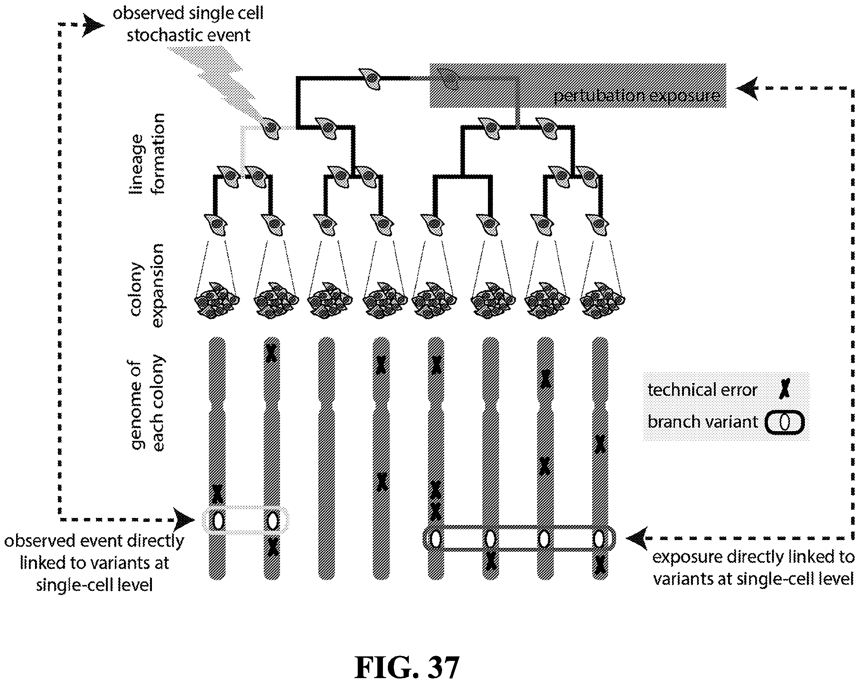

**Figure 37 - Scheme for directly associating single-cell events and exposures to single-cell sequence variation.** When the lineage is cultured under observation specific events observed to occur in single cells can be noted. Similarly, if particular cells in the lineage are exposed to perturbations such as chemical exposures, pathogens, radiation, or different culture conditions, the specific cells perturbed can be noted. Because lineage sequencing can assign novel somatic variants to lineage segments that correspond to individual cell lifetimes, Applicants expect it will be possible to directly associate single-cell phenotypes and exposures with single-cell resolved genomic variants using the lineage sequencing approach.

FIG. 38 - Examination of cosine similarity between HT115 branch variants mutational spectra with mutational spectra derived from tumor-normal whole genome sequencing of patients with previously identified POLE proofreading deficiency generated by the TCGA Research Network. Cosine similarity was measured with 95% intervals calculated by bootstrapping the HT115 spectra $10^4$ times. Showing for each sample the germline mutations in POLE gene. (CRC - colorectal cancer)

**FIG. 39 -** Steps 1 and 2 of an alternative lineage sequencing method (lineage sequencing 2.0). Upper panel shows the membrane slide. Lower panel depicts an image of a movie of dividing cells.

**FIG. 40** - Step 3 of an alternative lineage sequencing method (lineage sequencing 2.0) showing selection of lineages to extract from the slide.

**FIG. 41** - Step 4 of an alternative lineage sequencing method (lineage sequencing 2.0) showing a laser capture microscope set up to capture and isolate single cells.

**FIG. 42** - Step 4 of an alternative lineage sequencing method (lineage sequencing 2.0) showing a laser cutting the membrane slide and capture of a single cell with a sticky cap.

FIG. 43 - (left panel) shows an image of a movie depicting cells cut from a slide. (right panel) shows a slide where cells were cut out and captured.

**FIG. 44** - Step 4 of an alternative lineage sequencing method (lineage sequencing 2.0) showing catapulting a single cell to capture it.

**FIG. 45** - shows an image of a movie depicting cells captured from a slide using catapulting.

**FIG. 46** - Lineage sequence 2.0 proof of principle results.

**FIG. 47** - Training set for automatic lineage sequencing (upper panel) and image of a movie depicting cell division detected by the automated microscope (lower panel).

**FIG. 48** - Diagram depicting Lin-Diff-seq.

**FIG. 49** - Lineage generated using Lin-Diff-seq.

**FIG. 50** - Histogram of the number of high quality coincident SNVs for each set of sub clones in which such variants occurred for the HT115.

## DETAILED DESCRIPTION OF THE EXAMPLE EMBODIMENTS

### General Definitions

Unless defined otherwise, technical and scientific terms used herein have the same meaning as commonly understood by one of ordinary skill in the art to which this disclosure pertains. Definitions of common terms and techniques in molecular biology may be found in Molecular Cloning: A Laboratory Manual, 2nd edition (1989) (Sambrook, Fritsch, and Maniatis); Molecular Cloning: A Laboratory Manual, 4th edition (2012) (Green and Sambrook); Current Protocols in Molecular Biology (1987) (F.M. Ausubel et al. eds.); the series Methods in Enzymology (Academic Press, Inc.): PCR 2: A Practical Approach (1995) (M.J. MacPherson, B.D. Hames, and G.R. Taylor eds.): Antibodies, A Laboraotry Manual (1988) (Harlow and Lane, eds.): Antibodies A Laboraotry Manual, 2nd edition 2013 (E.A. Greenfield ed.); Animal Cell Culture (1987) (R.I. Freshney, ed.); Benjamin Lewin, Genes IX, published by Jones and Bartlet, 2008 (ISBN 0763752223); Kendrew et al. (eds.), The Encyclopedia of Molecular Biology, published by Blackwell Science Ltd., 1994 (ISBN 0632021829); Robert A. Meyers (ed.), Molecular Biology and Biotechnology: a Comprehensive Desk Reference, published by VCH Publishers, Inc., 1995 (ISBN 9780471185710); Singleton et al., Dictionary of Microbiology and Molecular Biology 2nd ed., J. Wiley & Sons (New York, N.Y. 1994), March, Advanced Organic Chemistry Reactions, Mechanisms and Structure 4th ed., John Wiley & Sons (New York, N.Y. 1992); and Marten H. Hofker and Jan van Deursen, Transgenic Mouse Methods and Protocols, 2nd edition (2011) .

As used herein, the singular forms "a", "an", and "the" include both singular and plural referents unless the context clearly dictates otherwise.

The term "optional" or "optionally" means that the subsequent described event, circumstance or substituent may or may not occur, and that the description includes instances where the event or circumstance occurs and instances where it does not.

The recitation of numerical ranges by endpoints includes all numbers and fractions subsumed within the respective ranges, as well as the recited endpoints.

The terms "about" or "approximately" as used herein when referring to a measurable value such as a parameter, an amount, a temporal duration, and the like, are meant to encompass variations of and from the specified value, such as variations of +/-10% or less, +/-5% or less, +/-1% or less, and +/-0.1% or less of and from the specified value, insofar such variations are appropriate to perform in the disclosed invention. It is to be understood that the value to which the modifier "about" or "approximately" refers is itself also specifically, and preferably, disclosed.

The terms "subject", "individual" or "patient" are used interchangeably throughout this specification, and typically and preferably denote humans, but may also encompass reference to non-human animals, preferably warm-blooded animals, even more preferably mammals, such as, e.g., non-human primates, rodents, canines, felines, equines, ovines, porcines, and the like. The term "non-human animals" includes all vertebrates, e.g., mammals, such as non-human primates, (particularly higher primates), sheep, dog, rodent (e.g. mouse or rat), guinea pig, goat, pig, cat, rabbits, cows, and non-mammals such as chickens, amphibians, reptiles etc. In one embodiment, the subject is a non-human mammal. In another embodiment, the subject is human. In another embodiment, the subject is an experimental animal or animal substitute as a disease model. The term does not denote a particular age or sex. Thus, adult and newborn subjects, as well as fetuses, whether male or female, are intended to be covered. Examples of subjects include humans, dogs, cats, cows, goats, and mice. The term subject is further intended to include transgenic species.

Reference throughout this specification to "one embodiment", "an embodiment," "an example embodiment," means that a particular feature, structure or characteristic described in connection with the embodiment is included in at least one embodiment of the present invention. Thus, appearances of the phrases "in one embodiment," "in an embodiment," or "an example embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention. For example, in the appended claims, any of the claimed embodiments can be used in any combination.

**Overview**

Mutation data reveal the dynamic equilibrium between DNA damage and repair processes in cells and are indispensable to the understanding of age-related diseases, tumorigenesis, tumor evolution, and the acquisition of drug resistance. However, available genome-wide methods have a limited ability to resolve rare somatic variants and the relationships between such variants. Embodiments disclosed herein provide methods for a DNA sequencing approach, *'lineage sequencing',* which carries out independent genome sequencing of lineages of close and known relationships in a clonal population and provides improved sensitivity and specificity of all genomic aberrations that occur in a given cell lineage, including CNVs, indels and SNP detection, in addition to unbiased precise/sensitive detection of de novo mutations genome-wide occurring during in vitro culture with time resolution as good as a single generation and dense lineage sampling. As used herein, the term "clonal" refers to cells derived from the same cell.

Lineage sequencing produces high-quality somatic mutation call sets with resolution as high as the single-cell level in subject lineages. Lineage sequencing entails sampling single cells from a population and sequencing sub-clonal sample sets derived from these cells. The method leverages knowledge of relationships among the cells to jointly call variants across the full sample set. This approach integrates data from multiple sequence libraries representing different sub-lineages (e.g., from sub-clonal sample sets) to support each variant and enables the precise assignment of mutations to lineage segments with resolution as high as a single cell cycle. In certain embodiments, cells can be cultured under continuous observation to link observed single-cell phenotypes with single-cell mutation data. In certain embodiments, the somatic mutation call sets produced by lineage sequencing are consistent with previous analyses in aggregate, while the high sensitivity, specificity, and resolution of the data provide a unique opportunity for quantitative analysis of variation in mutation rate, spectrum, and correlations among variants. In certain embodiments, lineage sequencing allows testing basic questions about mutagenesis, such as the uniformity of mutation rates within a lineage.

The methods of lineage sequencing of the present invention may apply the analysis to single cells in a small population of cells grown in vitro under controlled conditions. In certain embodiments, cells are grown under perturbation conditions. The lineage sequencing approach provides high signal to noise ratio for rare mutations, genome-wide scope, and single-cell single generation resolution. This method enables for the detection of the genetic alterations that are fixed into the genome of single cells within limited time periods in a controlled environment. One important application for this method is enabling direct and precise measurements for quantification of mutagenicity for different environmental sources. The invention is useful as a tool to accurately quantify different substances for their genetic effect on human cells and thusly quantify their mutagenicity. The currently used measurements lack single cell resolution, single generation resolution, and are biased against neutral/deleterious mutations. Minimally biased measurements with maximum resolution are crucial to reveal new information about the biochemical mechanisms of mutation and DNA repair. In addition, having the ability to accurately quantify the mutagenic effect of a drug meets an unmet clinical need to determine the mutagenic effects of therapies prior to their widespread deployment, and can improve treatments to extend life expectancies for cancer patients.

**Isolation of Single Cells from a Clonal Population**

In certain example embodiments, single cells arising from different lineage segments across a clonal population are isolated. As used herein a "clonal population" refers to a population of cells arising from a single parent cell. Thus, the single cells arising from different lineage segments represent daughter cells replicated from the original parent cell and subsequent daughter cells arising from replication of further generations of daughter cells. In certain example embodiments, the single cell from a given lineage segment may have undergone 1, 2, 3, 4 or 5 cell divisions prior to isolation. The terms "lineage segment" and "generation" may be used interchangeably herein. In certain example embodiments, the clonal population may be expanded for at least 2, at least 3, at least 4, at least 5, at least 6, at least 7, at least 8, at least 9, or at least 10 generations. Thus, a single cell is isolated from each generation or lineage segment (e.g., daughter cells from the last generation). The single cell arising from each generational lineage to be assayed may be isolated using any suitable technique for isolating single cells.

In certain embodiments, live cell imaging is used to track single cells. In certain embodiments, single cells across a lineage are visualized and/or recorded by a live cell imaging system. Live cell imaging is the study of living cells using time-lapse microscopy (see, e.g., Khodjakov, A. and Rieder, C. L., Imaging the division process in living tissue culture cells. Methods 38: 2-16 (2006); and Goldman, R. D. and Spector, D. L. (eds.), Live Cell Imaging: A Laboratory Manual. Cold Spring Harbor Press, Cold Spring Harbor, New York, 631 pages (2005)). Live cell imaging may be used in the present invention in combination with microfluidic systems, optical tweezers and laser capture microscopy. For example, the separation of cells to different quadrants using optical tweezers may be recorded. In another example, live cell imaging may also be used with a microfluidic device of the present invention to track cells. In another example, live cell imaging may be used to generate movies or recordings of dividing cells (e.g., on a slide). In certain embodiments, the recordings are used to capture cells of a lineage using laser capture microscopy.

In certain embodiments, depreciation is minimized (i.e., accurately identify, track and measure thousands of single cells via high-throughput microscopy). In certain embodiments, cells are labeled by the heterogeneous random uptake of fluorescent nanoparticles of different emission colors to generate a large number of unique digital codes (see, e.g., Rees et al., Nanoparticle vesicle encoding for imaging and tracking cell populations, Nat Methods. 2014 Nov;11(11):1177-81. doi: 10.1038/nmeth.3105. Epub 2014 Sep 14). Commercial highly fluorescent nanocrystals (e.g., Qtracker 525) exploits stochastic uptake dynamics of the nanoparticles. The uptake of nanoparticles and their subsequent encapsulation within vesicles inside the cell is a random process so the number of nanoparticle-labeled vesicles (NLVs) varies from cell to cell.

In certain example embodiments, the single cells may be isolated using a microfluidic device. Microfluidic devices may provide advantages in sorting and tracking of single cells, in automation, in reagent cost, and in control of experimental conditions.

Microfluidic devices disclosed herein may be silicone-based chips and may be fabricated using a variety of techniques, including, but not limited to, hot embossing, molding of elastomers, injection molding, LIGA, soft lithography, silicon fabrication and related thin film processing techniques. Suitable materials for fabricating the microfluidic devices include, but are not limited to, cyclic olefin copolymer (COC), polycarbonate, poly(dimethylsiloxane) (PDMS), and poly(methylacrylate) (PMMA). In one embodiment, soft lithography in PDMS may be used to prepare the microfluidic devices. For example, a mold may be made using photolithography which defines the location of the one or more flow channels and the array of microwells. The substrate material is poured into a mold and allowed to set to create a stamp. The stamp is then sealed to a solid support such as, but not limited to, glass.

Due to the hydrophobic nature of some polymers, such as PDMS, which absorbs some proteins and may inhibit certain biological processes, a passivating agent may be necessary (Schoffner et al. Nucleic Acids Research, 1996, 24:375-379). Suitable passivating agents are known in the art and include, but are not limited to, silanes, parylene, n-Dodecyl-b-D-matoside (DDM), pluronic, Tween-20, other similar surfactants, polyethylene glycol (PEG), albumin, collagen, and other similar proteins and peptides.

The microfluidic devices may further comprise inlet and outlet ports, or openings, which in turn may be connected to valves, tubes, channels, chambers, and syringes and/or pumps for the introduction and extraction of fluids into and from the microfluidic device. The microfluidic devices may be connected to fluid flow actuators that allow directional movement of fluids within the microfluidic device. Example actuators include, but are not limited to, *e.g.,* syringe pumps, mechanically actuated recirculating pumps, electroosmotic pumps, bulbs, bellows, diaphragms, or bubbles intended to force movement of fluids.

In example embodiments, the microfluidic device may be a hydrodynamic trap array device (see, e.g., Kimmerling et al., A microfluidic platform enabling single-cell RNA-seq of multigenerational lineages. Nat Commun. 2016 Jan 6;7:10220. doi: 10.1038/ncomms10220). In other example embodiments, the microfluidic device may be a system such as, but not limited to the Fluidigm system (see, e.g., www.fluidigm.com/products/c1-system). In other example embodiments, a microfluidic device may be used for generating a sequencing library integrating lysis, fragmentation, adapter tagging, purification, and size selection of 96 samples in parallel (see, e.g., International Publication Numbers WO 2015/050998 A2 and WO 2016/138290).

In one example embodiment, the single cells are isolated by loading the parent cell on a hydrodynamic trap device. In general, the device comprises an array of hydrodynamic traps that capture and culture single cells for multiple generations on a chip. The device can have multiple lanes for culturing more than one cell. In exemplary embodiments, a single cell is loaded into a lane of a trap array. The single cell is trapped in a first trap. Due to a pressure differential across the trap lanes, cells travel to the first unoccupied trap in the array. When the cell divides, a daughter cell is carried downstream and captured in a subsequent unoccupied trap. In certain embodiments, time lapse imaging of this process allows determination of single-cell proliferation kinetics. In certain embodiments, time lapse imaging of this process allows identification of lineal relationships between cells. In certain embodiments, each lane of traps can capture more than 4, more than 8, more than 16, more than 32, more than 64 or more than 128 cells, thus allowing lineage tracking for more than 2, 3, 4, 5, 6 or 7 generations. In certain embodiments, retrieval of cells is performed by reversing the pressure differential across trap lanes to collect each single cell as they exit the device from the loading channel.

In certain embodiments, single cells across a lineage may be isolated using an optical tweezer. The terms "optical tweezing," "optical trapping," "laser tweezing," "laser trapping" and "single-beam gradient force trap" are used interchangeably herein and refer to the use of a highly focused laser beam to provide an attractive or repulsive force, depending on the refractive index mismatch to physically hold and move microscopic dielectric objects similar to tweezers. Optical tweezing relies on a difference in refractive index between the cell and the surrounding solution and are applicable for use in combination with microfluidic systems (see, e.g., Landry et al., Optofluidic cell selection from complex microbial communities for single-genome analysis. Methods Enzymol. 2013;531:61-90). As such, cells (or other structures with a boundary defined by a refractive index gradient), but not the solution itself, can be trapped using this method. High-performance microscope objectives can focus light very tightly to create an optical trap that has a capture radius comparable to the wavelength of light used to create the trap.

These properties allow an optical trap to be used with surgical precision to move one cell when many others are nearby, while minimizing the possibility of carrying forward an extraneous cell or foreign DNA. Contamination by environmental DNA or double sorting is a significant practical concern in single-cell genomics, particularly in *de novo* applications, where reference sequence to identify contaminating reads is not available. By contrast, other popular methods such as fluorescence-activated flow cytometry and micromanipulation work by subdividing a liquid sample, and require a high dilution of cells in clean buffer to reduce the chance of contamination.

While idealized representations often depict spherical objects being trapped, in practice, cells of essentially any morphology can be moved using an optical trap.

In certain other example embodiments, single cells may be isolated using laser capture microscopy. Laser capture microdissection (LCM) is a cell separation technique that combines microscopy with laser beam technology and allows targeting of specific cells or tissue regions that need to be separated from others. (see, e.g., Vandewoestyne et al., Laser capture microdissection: should an ultraviolet or infrared laser be used? Anal Biochem. 2013 Aug 15;439(2):88-98. doi: 10.1016/j.ab.2013.04.023). Exemplary systems may be the MMI CellCut laser microdissection (LMD) system (Molecular Machines & Industries GmbH, Eching, Germany; www.molecular-machines.com/home)(see also, Espina, et al. Laser-capture microdissection technology. Expert. Rev. Mol. Diagn. 7, 647-657). In certain embodiments, cells of interest are grown on a slide and live cell imaging is used to record the dividing cells. In certain embodiments, one microscope is used for live cell imaging allowing for the locations of cells to be tracked and recorded. In certain embodiments, a second microscope is used for laser dissection of single cells identified by live cell imaging (e.g. cells tracked and recorded). In certain embodiments, the spatial coordinates of the cells on the slide are preserved when transferring from one microscope to another. In one embodiment, the slides are marked to maintain spatial orientation of the slides and the tracked cells. In certain embodiments, a laser is used to cut out cells from a coverslip (e.g., a coverslip that can be cut by a laser such as UV) and the cell captured using a surface configured for capturing cells (e.g., a sticky cap of an Eppendorf tube). Capturing may also be performed using catapulting (see, e.g., Horneffer et al., Principles of laser-induced separation and transport of living cells J Biomed Opt. 2007 Sep-Oct;12(5):054016; and Vogel et al., Principles of laser microdissection and catapulting of histologic specimens and live cells, Methods Cell Biol. 2007;82:153-205). Live cells captured using the live cell imaging and microdissection can be used to perform lineage sequencing as described herein.

In certain embodiments, the lineage tracing (e.g., live imaging, selecting cells of a lineage, and capturing single cells) is automated. In certain embodiments, a computer is a trained to select cells that are part of a lineage with training sets and live cell imaging. The computer can select the dividing cells and capture them for downstream processing. In certain embodiments, the method uses a network and training strategy that relies on the strong use of data augmentation to use the available annotated samples more efficiently (see, e.g., Ronneberger O., Fischer P., Brox T. (2015) U-Net: Convolutional Networks for Biomedical Image Segmentation. In: Navab N., Hornegger J., Wells W., Frangi A. (eds) Medical Image Computing and Computer-Assisted Intervention - MICCAI 2015. Lecture Notes in Computer Science, vol 9351. Springer, Cham).

In certain embodiments, an optical tweezer may be used in combination with laser capture microscopy to facilitate capture of single cells by moving single cells to a location for capture.

## Culturing Sub-Clonal Cell Populations

In certain embodiments, each isolated single cell from each generational lineage are culture to generate a sub-clonal cell population for each generational lineage. Each sub-clonal cell population may be cultured according to standard cell culture techniques. Imaging may be used to confirm seeding of a single cell in a single culture vessel or well. Sub-colonies may be grown to a density of between $10^3$ to $10^9$ cells and depending on the subsequent sequencing technique to be employed.

General techniques useful in the practice of this invention in cell culture and media uses are known in the art (e.g., Large Scale Mammalian Cell Culture (Hu et al. 1997. Curr Opin Biotechnol 8: 148); Serum-free Media (K. Kitano.

1991. Biotechnology 17: 73); or Large Scale Mammalian Cell Culture (Curr Opin Biotechnol 2: 375, 1991). The terms "culturing" or "cell culture" are common in the art and broadly refer to maintenance of cells and potentially expansion (proliferation, propagation) of cells *in vitro.* Typically, animal cells, such as mammalian cells, such as human cells, are cultured by exposing them to (i.e., contacting them with) a suitable cell culture medium in a vessel or container adequate for the purpose (e.g., a 96-, 24-, or 6-well plate, a T-25, T-75, T-150 or T-225 flask, or a cell factory), at art-known conditions conducive to *in vitro* cell culture, such as temperature of 37°C, 5% v/v $CO_2$ and > 95% humidity.

Methods related to stem cells and differentiating stem cells are known in the art (see, e.g., "Teratocarcinomas and embryonic stem cells: A practical approach" (E. J. Robertson, ed., IRL Press Ltd. 1987); "Guide to Techniques in Mouse Development" (P. M. Wasserman et al. eds., Academic Press 1993); "Embryonic Stem Cells: Methods and Protocols" (Kursad Turksen, ed., Humana Press, Totowa N.J., 2001 ); "Embryonic Stem Cell Differentiation in Vitro" (M. V. Wiles, Meth. Enzymol. 225: 900, 1993); "Properties and uses of Embryonic Stem Cells: Prospects for Application to Human Biology and Gene Therapy" (P. D. Rathjen et al., al.,1993). Differentiation of stem cells is reviewed, e.g., in Robertson. 1997. Meth Cell Biol 75: 173; Roach and McNeish. 2002. Methods Mol Biol 185: 1 -16; and Pedersen. 1998. Reprod Fertil Dev 10: 31). For further elaboration of general techniques useful in the practice of this invention, the practitioner can refer to standard textbooks and reviews in cell biology, tissue culture, and embryology (see, e.g., Culture of Human Stem Cells (R. Ian Freshney, Glyn N. Stacey, Jonathan M. Auerbach - 2007); Protocols for Neural Cell Culture (Laurie C. Doering - 2009); Neural Stem Cell Assays (Navjot Kaur, Mohan C. Vemuri - 2015); Working with Stem Cells (Henning Ulrich, Priscilla Davidson Negraes - 2016); and Biomaterials as Stem Cell Niche (Krishnendu Roy - 2010)). For further methods of cell culture solutions and systems, see International Patent publication WO2014159356A1.

The term "medium" as used herein broadly encompasses any cell culture medium conducive to maintenance of cells, preferably conducive to proliferation of cells. Typically, the medium will be a liquid culture medium, which facilitates easy manipulation (e.g., decantation, pipetting, centrifugation, filtration, and such) thereof.

Typically, the medium will comprise a basal medium formulation as known in the art. Many basal media formulations (available, e.g., from the American Type Culture Collection, ATCC; or from Invitrogen, Carlsbad, California) can be used, including but not limited to Eagle's Minimum Essential Medium (MEM), Dulbecco's Modified Eagle's Medium (DMEM), alpha modified Minimum Essential Medium (alpha-MEM), Basal Medium Essential (BME), Iscove's Modified Dulbecco's Medium (IMDM), BGJb medium, F-12 Nutrient Mixture (Ham), Liebovitz L-15, DMEMIF-12, Essential Modified Eagle's Medium (EMEM), RPMI-1640, Medium 199, Waymouth's MB 752/1 or Williams Medium E, and modifications and/or combinations thereof. Compositions of basal media are generally known in the art and it is within the skill of one in the art to modify or modulate concentrations of media and/or media supplements as necessary for the cells cultured.

Such basal media formulations contain ingredients necessary for mammalian cell development, which are known *per se.* By means of illustration and not limitation, these ingredients may include inorganic salts (in particular salts containing Na, K, Mg, Ca, Cl, P and possibly Cu, Fe, Se and Zn), physiological buffers (e.g., HEPES, bicarbonate), nucleotides, nucleosides and/or nucleic acid bases, ribose, deoxyribose, amino acids, vitamins, antioxidants (e.g., glutathione) and sources of carbon (e.g., glucose, sodium pyruvate, sodium acetate), etc.

For use in culture, basal media can be supplied with one or more further components. For example, additional supplements can be used to supply the cells with the necessary trace elements and substances for optimal growth and expansion. Furthermore, antioxidant supplements may be added, e.g., β-mercaptoethanol. While many basal media already contain amino acids, some amino acids may be supplemented later, e.g., L-glutamine, which is known to be less stable when in solution. A medium may be further supplied with antibiotic and/or antimycotic compounds, such as, typically, mixtures of penicillin and streptomycin, and/or other compounds, exemplified but not limited to, amphotericin, ampicillin, gentamicin, bleomycin, hygromycin, kanamycin, mitomycin, mycophenolic acid, nalidixic acid, neomycin, nystatin, paromomycin, polymyxin, puromycin, rifampicin, spectinomycin, tetracycline, tylosin, and zeocin.

Lipids and lipid carriers can also be used to supplement cell culture media. Such lipids and carriers can include, but are not limited to cyclodextrin, cholesterol, linoleic acid conjugated to albumin, linoleic acid and oleic acid conjugated to albumin, unconjugated linoleic acid, linoleic-oleic-arachidonic acid conjugated to albumin, oleic acid unconjugated and conjugated to albumin, among others. Albumin can similarly be used in fatty-acid free formulations.

Also contemplated is supplementation of cell culture media with mammalian plasma or sera. Plasma or sera often contain cellular factors and components that facilitate cell viability and expansion. Optionally, plasma or serum may be heat inactivated. Heat inactivation is used in the art mainly to remove the complement. Heat inactivation typically involves incubating the plasma or serum at 56°C for 30 to 60min, e.g., 30min, with steady mixing, after which the plasma or serum is allowed to gradually cool to ambient temperature. A skilled person will be aware of any common modifications and requirements of the above procedure. Optionally, plasma or serum may be sterilised prior to storage or use. Usual means of sterilisation may involve, e.g., filtration through one or more filters with pore size smaller than $1\mu m$, preferably smaller than $0.5\mu m$, e.g., smaller than $0.45\mu m$, $0.40\mu m$, $0.35\mu m$, $0.30\mu m$ or $0.25\mu m$, more preferably $0.2\mu m$ or smaller, e.g., $0.15\mu m$ or smaller, $0.10\mu m$ or smaller. Suitable sera or plasmas for use in media as taught herein may include human serum or plasma, or serum or plasma from non-human animals, preferably non-human mammals, such as, e.g., non-human primates (e.g., lemurs, monkeys, apes), foetal or adult bovine, horse, porcine, lamb, goat, dog, rabbit, mouse or rat serum or plasma, etc., or any combination of such. In certain preferred embodiments, a medium as taught herein may comprise bovine serum or plasma, preferably foetal bovine (calf) serum or plasma, more preferably foetal bovine (calf) serum (FCS or FBS). When culturing human cells, media may preferably comprise human serum or plasma, such as autologous or allogeneic human serum or plasma, preferably human serum, such as autologous or allogeneic human serum, more preferably autologous human serum or plasma, even more preferably autologous human serum.

In certain preferred embodiments, serum or plasma can be substituted in media by serum replacements, such as to provide for serum-free media (i.e., chemically defined media). The provision of serum-free media may be advantageous particularly with view to administration of the media or fraction(s) thereof to subjects, especially to human subjects (e.g., improved biosafety). By the term "serum replacement" it is broadly meant any a composition that may be used to replace the functions (e.g., cell maintenance and growth supportive function) of animal serum in a cell culture medium. A conventional serum replacement may typically comprise vitamins, albumin, lipids, amino acids, transferrin, antioxidants, insulin and trace elements. Many commercialized serum replacement additives, such as KnockOut Serum Replacement (KOSR), N2, B27, Insulin-Transferrin-Selenium Supplement (ITS), and G5 are well known and are readily available to those skilled in the art.

Plasma or serum or serum replacement may be comprised in media as taught herein at a proportion (volume of plasma or serum or serum replacement /volume of medium) between about 0.5% v/v and about 40.0% v/v, preferably between about 5.0% v/v and about 20.0% v/v, e.g., between about 5.0% v/v and about 15.0 % v/v, more preferably between about 8.0% v/v and about 12.0% v/v, e.g., about 10.0% v/v.

**Sequencing**

After the target cell density is achieved, genomic DNA may be extracted from each sub-clonal population using standard genomic DNA extraction technique. Sequencing libraries may then be prepared using standard techniques known in the art. An example sequencing library construction protocol is further described in the working examples below. In preferred embodiments, the invention does not require an amplification step.

In preferred embodiments, the present disclosure uses next generation sequencing in order to detect mutations genome wide. Exemplary next generation sequencing technologies include, for example, Illumina sequencing, Ion Torrent sequencing, 454 sequencing, SOLiD sequencing, and nanopore sequencing amongst others. Methods for constructing sequencing libraries are known in the art (see, e.g., Head et al., Library construction for next-generation sequencing: Overviews and challenges. Biotechniques. 2014; 56(2): 61-77).

In an embodiment, sequencing is performed on colonies of expanded single cells. Not being bound by a theory, artifacts caused by amplification will not be caused in the expanded cells and the expanded cells provide genetic material that can be later used to validate the results. Whole-genome amplification (WGA) methods are limited by low accuracy of copy-number variation (CNV) detection and low amplification fidelity (Chen et al. 2017). Methods of expanding colonies in tissue culture are well known in the art. In certain example embodiments, sequencing is done using short-read shotgun sequencing. An exemplary short-read shotgun sequencing technique is described in further detail in the working examples below.

In certain embodiments, when colony expansion is difficult, single cells are sequenced after whole genome amplification of single cells (e.g., multiple displacement amplification (MDA) whole-genome amplification (WGA) chemistry). Since the development of MDA, many other WGA methods have been developed and successfully applied to single cells (Blainey 2013, Cai & Walsh et al, 2012). MDA works by the extension of 6-mer 3'-protected random primers on the DNA template (Dean, et al., 2001, Genome Res 11: 1095-1099). In MDA, a polymerase with strong strand displacement activity such as phi29 DNA polymerase or Bst DNA polymerase creates and displaces overlapping synthesis products from the template as single-stranded DNA under isothermal conditions (Dean, et al., 2001, Genome Res 11: 1095-1099; Zhang, et al., 2001, Mol Diagn 6: 141-150; Aviel-Ronen, et al., 2006, BMC Genomics 7). The displaced single-stranded DNA is a substrate for further priming and synthesis (Dean, et al., 2001, Genome Res 11: 1095-1099; Zhang, et al., 2001, Mol Diagn 6: 141-150). Phi29 DNA polymerase is typically specified for MDA due to its high accuracy owing to 3' - 5' exonuclease-mediated proofreading and exceptionally strong processivity in strand displacement synthesis, which can exceed 10,000 nt (Mellado, et al., 1980, Virology 104: 84-96; Blanco & Salas, 1984, Proceedings of the National Academy of Sciences 81: 5325; Blanco, et al., 1989, Journal of Biological Chemistry 264: 8935-8940; Morin, et al., 2012, Proceedings of the National Academy of Sciences 109: 8115-8120). This property of the polymerase evens out amplification on shorter genomic distances to produce high molecular weight products with more uniform amplification across the template than purely PCR-based methods, which typically produce products shorter than 1000 nt and exhibit greater amplification bias (Dean, et al., 2002, Proceedings of the National Academy of Sciences 99: 5261).

The terms "depth" or "coverage" as used herein refers to the number of times a nucleotide is read during the sequencing process. Depth can be calculated from the length of the original genome (G), the number of reads($N$), and the average read length($L$) as $N \times L/G$. For example, a hypothetical genome with 2,000 base pairs reconstructed from 8 reads with an average length of 500 nucleotides will have 2x redundancy. This parameter also enables one to estimate other quantities, such as the percentage of the genome covered by reads (sometimes also called coverage). A high coverage in shotgun sequencing is desired because it can overcome errors in base calling and assembly. The subject of DNA sequencing theory addresses the relationships of such quantities. Even though the sequencing accuracy for each individual nucleotide is very high, the very large number of nucleotides in the genome means that if an individual genome is only sequenced once, there will be a significant number of sequencing errors. Furthermore rare single-nucleotide polymorphisms (SNPs) are common. Hence to distinguish between sequencing errors and true SNPs, it is necessary to increase the sequencing accuracy even further by sequencing individual genomes a large number of times.

The term "deep sequencing" as used herein indicates that the total number of reads is many times larger than the length of the sequence under study. The term "deep" as used herein refers to a wide range of depths greater than $1 \times$ up to $100 \times$.

The terms "low-pass sequencing" or "shallow sequencing" as used herein refers to a wide range of depths greater than or equal to $0.1 \times$ up to $1 \times$.

The term "ultra-deep" as used herein refers to higher coverage (>100-fold), which allows for detection of sequence variants in mixed populations.

**Single Nucleotide Variant Analysis**

Sequencing data may then be used to identify somatic mutations (single nucleotide variants) in the clonal population. In certain example embodiments, this may comprise identifying of single nucleotide variants that occurred at the same locus in two or more, but less than all, sub-clones. Such groups of matching single nucleotide variants that coincide at the same genomic locus in multiple sub-clones may putatively represent de novo somatic mutations that occurred during expansion of the clonal population. These single nucleotide variants may be referred to as "branch variants." By contrast, single nucleotide variants shared by all sub clones were most likely present in the founding parent cell. Single nucleotide variants appearing in only one sub-clone likely represent variants that either appeared in the last round of cell division, appeared early in sub-clonal culture (or later if strongly selected), or represent technical errors in sequencing or variant calling. In certain example embodiments, determining true somatic mutations along a single cell lineage may comprise defining set of branch variants comprising variants present in at least two sub-clones. Raw single nucleotide variants may be identified using methods such as MuTect (Cibulskis et al. 2013) or other comparable methods.

Using the branch variant set, mutational events and the flow of mutations through the lineage segments may be quantitatively reconstructed. Coincident single nucleotide variants, that is identical single nucleotide variants appearing at matching genomic loci in two or more but not all sub clones, are identified. In certain example embodiments, coincident single nucleotide events are selected where the quality of coincident single nucleotide variant classification is assessed by calculating the probability for each sample to belong to its consensus-assigned group ("reference" or "alternative") considering the base content in the read alignment at the locus in question for each sample. For example, the probability may be calculated using a binomial distribution test for each of the samples. Assuming each sample can belong to an assigned reference or variant group (H0) or to another group (H1). The calculated probability can provide a heuristic score to rank the quality of coincident single nucleotide variants groups. For example:

H0: P= P1, the probability weight that the sample belongs to group 1, the assigned reference or variant group.

H1 :P=P2, the probability weight that the sample belongs to group 2, a different group than assigned.

$$\mathrm{P1} = P(alt_{allele} = x \in group1) = \binom{\#alt}{total\_nucleotides} P1^{\#alt}(1 - P1)^{\#!alt}$$

$$\mathrm{P2} = P(alt_{allele} = x \in group2) = \binom{\#alt}{total\_nucleotides} P2^{\#alt}(1 - P2)^{\#!alt}$$

$$\mathrm{P(H0 \mid alternative\ allele = x)} = \frac{P1}{P1+P2}$$

For each classified single nucleotide variant, the probability for every sample may be calculated and the minimal probability chosen to represent the quality of the coincident classification option to the single nucleotide variant. Single nucleotide variants with a quality of this coincident classification less than 0.99 may be depleted.

In certain example embodiments, a lineage structure of the clonal population may be determined that identifies and maps the single nucleotide variants within the clonal population. In certain example embodiments, the lineage structure may be determined by analysis of time lapse imaging of cells in the microfluidic trap array device. Single nucleotide variants may be grouped as coincident single nucleotide variants and branch variants called by evaluating the coincident single nucleotide variant quality score for the highest scoring group of sub clones over all the groups of sub clones consistent with the lineage structure determined by time lapse imaging. The coincident single nucleotide variant quality score may be determined as follows:

$$\text{Coincident SNV Quality Score} = (\text{MAX P}_{\text{clusters}}(\text{Min P}_{\text{sample}}(\frac{P1}{P1+P2})))$$

In another example embodiment, the sequencing data may be used to estimate the most likely lineage structure in each lineage experiment. For example, coincident single nucleotide variants may be identified by analysis of the of raw SNV calls from the sub clones generated by MuTect (see methods section on Lineage sequencing analysis pipeline). Sub clones sharing the same SNVs may be grouped without restrictions by hierarchical clustering and for each coincident SNV, the quality score may be calculated as:

$$\text{Coincident SNV Quality Score} = (\text{Min P}_{\text{samples}}(\frac{P1}{P1+P2})).$$

The frequency of coincident SNVs for each group of sub clones may then be tabulated and ranked to generate plots such as those show in FIG. 22B. The groups of sub clones sharing a large number of high-scoring coincident SNVs may nest in such a way as to indicate a single lineage structure for each sample analysis. This lineage structure may then be subsequently used for calling branch variants. The prior estimated lineage structure may also be used to filter the high-quality coincident SNV list for coincident SNVs that were consistent with the lineage structure and produce the final branch variant call set.

By grouping the samples in each branch variant SNV and comparing to the base call at the same locus in a sister lineage, the reference allele and alternative allele may be determined, and a variant allele fraction calculated without depending on an external reference allele. However, for the first cell division, no such sister lineage exists within the dataset, and an external reference may be used. Suitable example methods for selecting a reference set are further discussed in the working examples below.

**Determining Mutation Spectra/Signatures**

In certain example embodiments, the method may further comprise determining a mutation spectrum or signature. Mutational spectra may be defined using standard approaches and reported in the standard format(Alexandrov et al. 2013b). Each SNV may be classified into one of six subtypes; C:G>A:T, C:G>G:C, C:G>T:A, T:A>A:T, T:A>C:G, and T:A>G:C. and further refined by including the sequence context of each mutated base (one base 3' and one base 5'). For example, a C:G>T:A mutation can be characterized as TpCpG>TpTpG (mutated base underlined and presented as the pyrimidine partner of the mutated base pair) generating 96 possible mutation types (6 types of substitution * 4 types of 5' base * 4 types of 3' base). The mutational spectra may be compared with patterns extracted from clinical and cell line (ATCC) sample. The similarity between two mutational patterns A and B, defined as a non-negative vector with 96 mutation types, was computed by cosine similarity:

$$sim(A, B) = \frac{\sum_{i=1}^{n} A_i B_i}{\sqrt{\sum_{i=1}^{n} A_i^2} \sqrt{\sum_{i=1}^{n} B_i^2}}$$

The cosine similarity value may be used to determine if mutational spectra are more similar to one another. 95% confidence intervals may be calculated by bootstrapping the SNV list $10^4$ times.

Accordingly, the methods disclosed herein may also be used to determine characteristics pathways or phenotypes of a particular cell lineages. For example, the methods disclosed herein may be used on a single cells derived from a patient to further diagnose a particular disease sub-type or phenotype, which in turn may be used to guide appropriate therapeutic regiments. In certain embodiments, patient cells are analyzed for genotoxicity to a particular drug or therapeutic regimen.

Cells

In certain embodiments, the cell is derived from cells taken from a subject, such as a cell line. A wide variety of cell lines for tissue culture models are known in the art. Examples of cell lines include, but are not limited to, HT115, RPE1, C8161, CCRF-CEM, MOLT, mIMCD-3, NHDF, HeLa-S3, Huh1, Huh4, Huh7, HUVEC, HASMC, HEKn, HEKa, MiaPaCell, Panc1, PC-3, TF1, CTLL-2, C1R, Rat6, CV1, RPTE, A10, T24, J82, A375, ARH-77, Calu1, SW480, SW620, SKOV3, SK-UT, CaCo2, P388D1, SEM-K2, WEHI-231, HB56, TIB55, Jurkat, J45.01, LRMB, Bcl-1, BC-3, IC21, DLD2, Raw264.7, NRK, NRK-52E, MRC5, MEF, Hep G2, HeLa B, HeLa T4, COS, COS-1, COS-6, COS-M6A, BS-C-1 monkey kidney epithelial, BALB/ 3T3 mouse embryo fibroblast, 3T3 Swiss, 3T3-L1, 132-d5 human fetal fibroblasts; 10.1 mouse fibroblasts, 293-T, 3T3, 721, 9L, A2780, A2780ADR, A2780cis, A172, A20, A253, A431, A-549, ALC, B16, B35, BCP-1 cells, BEAS-2B, bEnd.3, BHK-21, BR 293, BxPC3, C3H-10T1/2, C6/36, Cal-27, CHO, CHO-7, CHO-IR, CHO-K1, CHO-K2, CHO-T, CHO Dhfr -/-, COR-L23, COR-L23/CPR, COR-L23/5010, COR-L23/R23, COS-7, COV-434, CML T1, CMT, CT26, D17, DH82, DU145, DuCaP, EL4, EM2, EM3, EMT6/AR1, EMT6/AR10.0, FM3, H1299, H69, HB54, HB55, HCA2, HEK-293, HeLa, Hepa1c7, HL-60, HMEC, HT-29, Jurkat, JY cells, K562 cells, Ku812, KCL22, KG1, KYO1, LNCap, Ma-Mel 1-48, MC-38, MCF-7, MCF-10A, MDA-MB-231, MDA-MB-468, MDA-MB-435, MDCK II, MDCK II, MOR/0.2R, MONO-MAC 6, MTD-1A, MyEnd, NCI-H69/CPR, NCI-H69/LX10, NCI-H69/LX20, NCI-H69/LX4, NIH-3T3, NALM-1, NW-145, OPCN / OPCT cell lines, Peer, PNT-1A / PNT 2, RenCa, RIN-5F, RMA/RMAS, Saos-2 cells, Sf-9, SkBr3, T2, T-47D, T84, THP1 cell line, U373, U87, U937, VCaP, Vero cells, WM39, WT-49, X63, YAC-1, YAR, and transgenic varieties thereof. Cell lines are available from a variety of sources known to those with skill in the art (see, e.g., the American Type Culture Collection (ATCC) (Manassus, Va.)).

In certain embodiments, the cell is a microbe. In certain example embodiments, the microbe is a bacterium. Examples of bacteria that can be detected in accordance with the disclosed methods include without limitation any one or more of (or any combination of) *Acinetobacter baumanii, Actinobacillus sp., Actinomycetes, Actinomyces sp.* (such as *Actinomyces israelii* and *Actinomyces naeslundii), Aeromonas sp.* (such as *Aeromonas hydrophila, Aeromonas veronii biovar sobria (Aeromonas sobria),* and *Aeromonas caviae), Anaplasma phagocytophilum, Anaplasma marginal,e Alcaligenes xylosoxidans, Acinetobacter baumanii, Actinobacillus actinomycetemcomitans, Bacillus sp.* (such as *Bacillus anthracis, Bacillus cereus, Bacillus subtilis, Bacillus thuringiensis,* and *Bacillus stearothermophilus), Bacteroides sp.* (such as *Bacteroides fragilis), Bartonella sp.* (such as *Bartonella bacilliformis* and *Bartonella henselae, Bifidobacterium sp., Bordetella sp.* ( such as *Bordetella pertussis, Bordetella parapertussis,* and *Bordetella bronchiseptica), Borrelia sp.* (such as *Borrelia recurrentis,* and *Borrelia burgdorferi), Brucella sp.* (such as *Brucella abortus, Brucella canis, Brucella melintensis* and *Brucella suis), Burkholderia sp.* (such as *Burkholderia pseudomallei* and *Burkholderia cepacia), Campylobacter sp.* (such as *Campylobacter jejuni, Campylobacter coli, Campylobacter lari* and *Campylobacter fetus), Capnocytophaga sp., Cardiobacterium hominis, Chlamydia trachomatis, Chlamydophila pneumoniae, Chlamydophila psittaci, Citrobacter sp. Coxiella burnetii, Corynebacterium sp.* (such as, *Corynebacterium diphtheriae, Corynebacterium jeikeum* and *Corynebacterium), Clostridium sp.* (such as *Clostridium perfringens, Clostridium difficile, Clostridium botulinum* and *Clostridium tetani), Eikenella corrodens, Enterobacter sp.* (such as *Enterobacter aerogenes, Enterobacter agglomerans, Enterobacter cloacae* and *Escherichia coli, including opportunistic Escherichia coli,* such as *enterotoxigenic E. coli, enteroinvasive E. coli, enteropathogenic E. coli, enterohemorrhagic E. coli, enteroaggregative E. coli* and *uropathogenic E. coli) Enterococcus sp.* (such as *Enterococcus faecalis* and *Enterococcus faecium) Ehrlichia sp.* (such as *Ehrlichia chafeensia* and *Ehrlichia canis), Erysipelothrix rhusiopathiae, Eubacterium sp., Francisella tularensis, Fusobacterium nucleatum, Gardnerella vaginalis, Gemella morbillorum, Haemophilus sp.* (such as *Haemophilus influenzae, Haemophilus ducreyi, Haemophilus aegyptius, Haemophilus parainfluenzae, Haemophilus haemolyticus* and *Haemophilus parahaemolyticus, Helicobacter sp.* (such as *Helicobacter pylori, Helicobacter cinaedi* and *Helicobacter fennelliae), Kingella kingii, Klebsiella sp.* ( such as *Klebsiella pneumoniae, Klebsiella granulomatis* and *Klebsiella oxytoca), Lactobacillus sp., Listeria monocytogenes, Leptospira interrogans, Legionella pneumophila, Leptospira interrogans, Peptostreptococcus sp., Mannheimia hemolytica, Moraxella catarrhalis, Morganella sp., Mobiluncus sp., Micrococcus sp., Mycobacterium sp.* (such as *Mycobacterium leprae, Mycobacterium tuberculosis, Mycobacterium paratuberculosis, Mycobacterium intracellulare, Mycobacterium avium, Mycobacterium bovis,* and *Mycobacterium marinum), Mycoplasm sp.* (such as *Mycoplasma pneumoniae, Mycoplasma hominis,* and *Mycoplasma genitalium), Nocardia sp.* (such as *Nocardia asteroides, Nocardia cyriacigeorgica* and *Nocardia brasiliensis), Neisseria sp.* (such as *Neisseria gonorrhoeae* and *Neisseria meningitidis), Pasteurella multocida, Plesiomonas shigelloides. Prevotella sp., Porphyromonas sp., Prevotella melaninogenica, Proteus sp.* (such as *Proteus vulgaris* and *Proteus mirabilis), Providencia sp.* (such as *Providencia alcalifaciens, Providencia rettgeri* and *Providencia stuartii), Pseudomonas aeruginosa, Propionibacterium acnes, Rhodococcus equi, Rickettsia sp.* (such as *Rickettsia rickettsii, Rickettsia akari* and *Rickettsia prowazekii, Orientia tsutsugamushi (formerly: Rickettsia tsutsugamushi)* and *Rickettsia typhi), Rhodococcus sp., Serratia marcescens, Stenotrophomonas maltophilia, Salmonella sp.* (such as *Salmonella enterica, Salmonella typhi, Salmonella paratyphi, Salmonella enteritidis, Salmonella cholerasuis* and *Salmonella typhimurium), Serratia sp.* (such as *Serratia marcesans* and *Serratia liquifaciens), Shigella sp.* (such as *Shigella dysenteriae, Shigella flexneri, Shigella boydii* and *Shigella sonnei), Staphylococcus sp.* (such as *Staphylococcus aureus, Staphylococcus epidermidis, Staphylococcus hemolyticus, Staphylococcus saprophyticus), Streptococcus sp.* (such as *Streptococcus pneumoniae* (for example *chloramphenicol-resistant serotype 4 Streptococcus pneumoniae, spectinomycin-resistant serotype 6B Streptococcus pneumoniae, streptomycin-resistant serotype 9V Streptococcus pneumoniae, erythromycin-resistant serotype 14 Streptococcus pneumoniae, optochin-resistant serotype 14 Streptococcus pneumoniae, rifampicin-resistant serotype 18C Streptococcus pneumoniae, tetracycline-resistant serotype 19F Streptococcus pneumoniae, penicillin-resistant serotype 19F Streptococcus pneumoniae,* and *trimethoprim-resistant serotype 23F Streptococcus pneumoniae, chloramphenicol-resistant serotype 4 Streptococcus pneumoniae, spectinomycin-resistant serotype 6B Streptococcus pneumoniae, streptomycin-resistant*

*serotype 9V Streptococcus pneumoniae, optochin-resistant serotype 14 Streptococcus pneumoniae, rifampicin-resistant serotype 18C Streptococcus pneumoniae, penicillin-resistant serotype 19F Streptococcus pneumoniae, or trimethoprim-resistant serotype 23F Streptococcus pneumoniae), Streptococcus agalactiae, Streptococcus mutans, Streptococcus pyogenes, Group A streptococci, Streptococcus pyogenes, Group B streptococci, Streptococcus agalactiae, Group C streptococci, Streptococcus anginosus, Streptococcus equismilis, Group D streptococci, Streptococcus bovis, Group F streptococci, and Streptococcus anginosus Group G streptococci), Spirillum minus, Streptobacillus moniliformi, Treponema sp. (such as Treponema carateum, Treponema petenue, Treponema pallidum and Treponema endemicum, Tropheryma whippelii, Ureaplasma urealyticum, Veillonella sp., Vibrio sp. (such as Vibrio cholerae, Vibrio parahemolyticus, Vibrio vulnificus, Vibrio parahaemolyticus, Vibrio vulnificus, Vibrio alginolyticus, Vibrio mimicus, Vibrio hollisae, Vibrio fluvialis, Vibrio metchnikovii, Vibrio damsela and Vibrio furnisii), Yersinia sp. ( such as Yersinia enterocolitica, Yersinia pestis, and Yersinia pseudotuberculosis) and Xanthomonas maltophilia among others.*

In certain example embodiments, the microbe is a fungus. Examples of fungi that can be detected in accordance with the disclosed methods include without limitation any one or more of (or any combination of), Aspergillus, Blastomyces, Candidiasis, Coccidiodomycosis, Cryptococcus neoformans, Cryptococcus gatti, Histoplasma, Mucroymcosis, Pneumocystis, Sporothrix, fungal eye infections ringworm, Exserohilum, and Cladosporium.

In certain example embodiments, the fungus is a yeast. Examples of yeast that can be detected in accordance with disclosed methods include without limitation one or more of (or any combination of), *Aspergillus* species, a *Geotrichum* species, a *Saccharomyces* species, a *Hansenula* species, a *Candida* species, a *Kluyveromyces* species, a *Debaryomyces* species, a *Pichia* species, or combination thereof. In certain example embodiments, the fungi is a mold. Example molds include, but are not limited to, a *Penicillium* species, a *Cladosporium* species, a *Byssochlamys* species, or a combination thereof.

In certain example embodiments, the microbe is a protozoa. Examples of protozoa that can be detected in accordance with the disclosed methods and devices include without limitation any one or more of (or any combination of), Euglenozoa, Heterolobosea, Diplomonadida, Amoebozoa, Blastocystic, and Apicomplexa. Example Euglenoza include, but are not limited to, *Trypanosoma cruzi* (Chagas disease), *T. brucei gambiense, T. brucei rhodesiense, Leishmania braziliensis, Z. infantum, Z. mexicana, Z. major, Z. tropica,* and *L. donovani*. Example Heterolobosea include, but are not limited to, *Naegleria fowleri*. Example Diplomonadid include, but are not limited to, *Giardia intestinalis (G. lamblia, G. duodenalis)*. Example Amoebozoa include, but are not limited to, *Acanthamoeba castellanii, Balamuthia madrillaris, Entamoeba histolytica*. Example Blastocystis include, but are not limited to, *Blastocystic hominis*. Example Apicomplexa include, but are not limited to, *Babesia microti, Cryptosporidium parvum, Cyclospora cayetanensis, Plasmodium falciparum, P. vivax, P. ovale, P. malariae,* and *Toxoplasma gondii*

## Personalized Medicine

In certain embodiments, cells are obtained from a subject in need thereof (e.g., subject suffering from cancer, or infection). Single cells may be treated with potential drug candidates (therapeutic agent) for the subject. Mutation rates may be determined for the different drugs and drugs may be selected for treatment that cause the least amount of mutations for cells of the subject. In certain embodiments, the cells assayed are stem cells or induced pluripotent stem cells (iPS). Methods of obtaining cells from a subject are well known in the art. In certain embodiments, the therapeutically effective amount is decreased if the drug causes a significant amount of mutations. In certain embodiments, the therapeutically effective amount is increased if the drug causes a significant amount of mutations.

The terms "therapeutic agent", "therapeutic capable agent" or "treatment agent" are used interchangeably and refer to a molecule or compound that confers some beneficial effect upon administration to a subject. The beneficial effect includes enablement of diagnostic determinations; amelioration of a disease, symptom, disorder, or pathological condition; reducing or preventing the onset of a disease, symptom, disorder or condition; and generally counteracting a disease, symptom, disorder or pathological condition.

As used herein, "treatment" or "treating," or "palliating" or "ameliorating" are used interchangeably. These terms refer to an approach for obtaining beneficial or desired results including but not limited to a therapeutic benefit and/or a prophylactic benefit. By therapeutic benefit is meant any therapeutically relevant improvement in or effect on one or more diseases, conditions, or symptoms under treatment. For prophylactic benefit, the compositions may be administered to a subject at risk of developing a particular disease, condition, or symptom, or to a subject reporting one or more of the physiological symptoms of a disease, even though the disease, condition, or symptom may not have yet been manifested.

The term "effective amount" or "therapeutically effective amount" refers to the amount of an agent that is sufficient to effect beneficial or desired results. The therapeutically effective amount may vary depending upon one or more of: the subject and disease condition being treated, the weight and age of the subject, the severity of the disease condition, the manner of administration and the like, which can readily be determined by one of ordinary skill in the art. The term also applies to a dose that will provide an image for detection by any one of the imaging methods described herein. The specific dose may vary depending on one or more of: the particular agent chosen, the dosing regimen to be followed, whether it is administered in combination with other compounds, timing of administration, the tissue to be imaged, and the physical delivery system in which it is carried.

In certain embodiments, the subject in need thereof has cancer. Examples of cancer relevant for the present disclosure include but are not limited to carcinoma, lymphoma, blastoma, sarcoma, and leukemia or lymphoid malignancies. More particular examples of such cancers include without limitation: squamous cell cancer (e.g., epithelial squamous cell cancer), lung cancer including small-cell lung cancer, non-small cell lung cancer, adenocarcinoma of the lung, squamous carcinoma of the lung and large cell carcinoma of the lung, cancer of the peritoneum, hepatocellular cancer, gastric or stomach cancer including gastrointestinal cancer, pancreatic cancer, glioma, glioblastoma, cervical cancer, ovarian cancer, liver cancer, bladder cancer, hepatoma, breast cancer, colon cancer, rectal cancer, colorectal cancer, endometrial cancer or uterine carcinoma, salivary gland carcinoma, kidney or renal cancer, prostate cancer, vulval cancer, thyroid cancer, hepatic carcinoma, anal carcinoma, penile carcinoma, as well as CNS cancer, melanoma, head and neck cancer, bone cancer, bone marrow cancer, duodenum cancer, oesophageal cancer, thyroid cancer, or hematological cancer.

Other non-limiting examples of cancers or malignancies include, but are not limited to: Acute Childhood Lymphoblastic Leukemia, Acute Lymphoblastic Leukemia, Acute Lymphocytic Leukemia, Acute Myeloid Leukemia, Adrenocortical Carcinoma, Adult (Primary) Hepatocellular Cancer, Adult (Primary) Liver Cancer, Adult Acute Lymphocytic Leukemia, Adult Acute Myeloid Leukemia, Adult Hodgkin's Disease, Adult Hodgkin's Lymphoma, Adult Lymphocytic Leukemia, Adult Non-Hodgkin's Lymphoma, Adult Primary Liver Cancer, Adult Soft Tissue Sarcoma, AIDS-Related Lymphoma, AIDS-Related Malignancies, Anal Cancer, Astrocytoma, Bile Duct Cancer, Bladder Cancer, Bone Cancer, Brain Stem Glioma, Brain Tumours, Breast Cancer, Cancer of the Renal Pelvis and Urethra, Central Nervous System (Primary) Lymphoma, Central Nervous System Lymphoma, Cerebellar Astrocytoma, Cerebral Astrocytoma, Cervical Cancer, Childhood (Primary) Hepatocellular Cancer, Childhood (Primary) Liver Cancer, Childhood Acute Lymphoblastic Leukemia, Childhood Acute Myeloid Leukemia, Childhood Brain Stem Glioma, Glioblastoma, Childhood Cerebellar Astrocytoma, Childhood Cerebral Astrocytoma, Childhood Extracranial Germ Cell Tumours, Childhood Hodgkin's Disease, Childhood Hodgkin's Lymphoma, Childhood Hypothalamic and Visual Pathway Glioma, Childhood Lymphoblastic Leukemia, Childhood Medulloblastoma, Childhood Non-Hodgkin's Lymphoma, Childhood Pineal and Supratentorial Primitive Neuroectodermal Tumours, Childhood Primary Liver Cancer, Childhood Rhabdomyosarcoma, Childhood Soft Tissue Sarcoma, Childhood Visual Pathway and Hypothalamic Glioma, Chronic Lymphocytic Leukemia, Chronic Myelogenous Leukemia, Colon Cancer, Cutaneous T-Cell Lymphoma, Endocrine Pancreas Islet Cell Carcinoma, Endometrial Cancer, Ependymoma, Epithelial Cancer, Esophageal Cancer, Ewing's Sarcoma and Related Tumours, Exocrine Pancreatic Cancer, Extracranial Germ Cell Tumour, Extragonadal Germ Cell Tumour, Extrahepatic Bile Duct Cancer, Eye Cancer, Female Breast Cancer, Gaucher's Disease, Gallbladder Cancer, Gastric Cancer, Gastrointestinal Carcinoid Tumour, Gastrointestinal Tumours, Germ Cell Tumours, Gestational Trophoblastic Tumour, Hairy Cell Leukemia, Head and Neck Cancer, Hepatocellular Cancer, Hodgkin's Disease, Hodgkin's Lymphoma, Hypergammaglobulinemia, Hypopharyngeal Cancer, Intestinal Cancers, Intraocular Melanoma, Islet Cell Carcinoma, Islet Cell Pancreatic Cancer, Kaposi's Sarcoma, Kidney Cancer, Laryngeal Cancer, Lip and Oral Cavity Cancer, Liver Cancer, Lung Cancer, Lymphoproliferative Disorders, Macroglobulinemia, Male Breast Cancer, Malignant Mesothelioma, Malignant Thymoma, Medulloblastoma, Melanoma, Mesothelioma, Metastatic Occult Primary Squamous Neck Cancer, Metastatic Primary Squamous Neck Cancer, Metastatic Squamous Neck Cancer, Multiple Myeloma, Multiple Myeloma/Plasma Cell Neoplasm, Myelodysplastic Syndrome, Myelogenous Leukemia, Myeloid Leukemia, Myeloproliferative Disorders, Nasal Cavity and Paranasal Sinus Cancer, Nasopharyngeal Cancer, Neuroblastoma, Non-Hodgkin's Lymphoma During Pregnancy, Nonmelanoma Skin Cancer, Non-Small Cell Lung Cancer, Occult Primary Metastatic Squamous Neck Cancer, Oropharyngeal Cancer, Osteo-/Malignant Fibrous Sarcoma, Osteosarcoma/Malignant Fibrous Histiocytoma, Osteosarcoma/Malignant Fibrous Histiocytoma of Bone, Ovarian Epithelial Cancer, Ovarian Germ Cell Tumour, Ovarian Low Malignant Potential Tumour, Pancreatic Cancer, Paraproteinemias, Purpura, Parathyroid Cancer, Penile Cancer, Pheochromocytoma, Pituitary Tumour, Plasma Cell Neoplasm/Multiple Myeloma, Primary Central Nervous System Lymphoma, Primary Liver Cancer, Prostate Cancer, Rectal Cancer, Renal Cell Cancer, Renal Pelvis and Urethra Cancer, Retinoblastoma, Rhabdomyosarcoma, Salivary Gland Cancer, Sarcoidosis Sarcomas, Sezary Syndrome, Skin Cancer, Small Cell Lung Cancer, Small Intestine Cancer, Soft Tissue Sarcoma, Squamous Neck Cancer, Stomach Cancer, Supratentorial Primitive Neuroectodermal and Pineal Tumours, T-Cell Lymphoma, Testicular Cancer, Thymoma, Thyroid Cancer, Transitional Cell Cancer of the Renal Pelvis and Urethra, Transitional Renal Pelvis and Urethra Cancer, Trophoblastic Tumours, Urethra and Renal Pelvis Cell Cancer, Urethral Cancer, Uterine Cancer, Uterine Sarcoma, Vaginal Cancer, Visual Pathway and Hypothalamic Glioma, Vulvar Cancer, Waldenstrom's Macroglobulinemia, or Wilms' Tumour.

**Perturbations**

The present disclosure is applicable to measuring the mutation rate and detecting mutation signatures in response to an external stimuli (i.e., perturbation). Perturbations may include exposure to different physical parameters, exposure to different types, combinations and/ or concentrations of chemical, and biological agents, and analysis of different genetic perturbations, including engineered genetic perturbations. The perturbation may be a drug. In preferred embodiments, the drugs are cancer drugs or antimicrobial drugs (e.g., antibiotics).

The therapeutic agent is for example, a chemotherapeutic or biotherapeutic agent, radiation, or immunotherapy. Any suitable therapeutic treatment for a particular cancer may be administered. Examples of chemotherapeutic and biotherapeutic agents include, but are not limited to an angiogenesis inhibitor, such as angiostatin KI-3, DL-a-Difluoromethyl-omithine, endostatin, fumagillin, genistein, minocycline, staurosporine, and thalidomide; a DNA intercalator/cross-linker, such as Bleomycin, Carboplatin, Carmustine, Chlorambucil, Cyclophosphamide, cis-Diammineplatinum(II) dichloride (Cisplatin), Melphalan, Mitoxantrone, and Oxaliplatin; a DNA synthesis inhibitor, such as ($\pm$)-Amethopterin (Methotrexate), 3-Amino-1,2,4-benzotriazine 1,4-di oxide, Aminopterin, Cytosine $\beta$-D-arabinofuranoside, 5-Fluoro-5'-deoxyuridine, 5-Fluorouracil, Ganciclovir, Hydroxyurea, and Mitomycin C; a DNA-RNA transcription regulator, such as Actinomycin D, Daunorubicin, Doxorubicin, Homoharringtonine, and Idarubicin; an enzyme inhibitor, such as S(+)-Camptothecin, Curcumin, (-)-Deguelin, 5,6-Dichlorobenzimidazole I-$\beta$-D-ribofuranoside, Etoposide, Formestane, Fostriecin, Hispidin, 2-Imino-l-imidazoli-dineacetic acid (Cyclocreatine), Mevinolin, Trichostatin A, Tyrphostin AG 34, and Tyrphostin AG 879; a gene regulator, such as 5-Aza-2'-deoxycytidine, 5-Azacytidine, Cholecalciferol (Vitamin D3), 4-Hydroxytamoxifen, Melatonin, Mifepristone, Raloxifene, all trans-Retinal (Vitamin A aldehyde), Retinoic acid, all trans (Vitamin A acid), 9-cis-Retinoic Acid, 13-cis-Retinoic acid, Retinol (Vitamin A), Tamoxifen, and Troglitazone; a microtubule inhibitor, such as Colchicine, docetaxel, Dolastatin 15, Nocodazole, Paclitaxel, Podophyllotoxin, Rhizoxin, Vinblastine, Vincristine, Vindesine, and Vinorelbine (Navelbine); and an unclassified antitumor agent, such as 17-(Allylamino)-17-demethoxygeldanamycin, 4- Amino- 1,8-naphthalimide, Apigenin, Brefeldin A, Cimetidine, Dichloromethylene-diphosphonic acid, Leuprolide (Leuprorelin), Luteinizing Hormone-Releasing Hormone, Pifithrin-a, Rapamycin, Sex hormone-binding globulin, Thapsigargin, Vismodegib (Erivedge™), and Urinary trypsin inhibitor fragment (Bikunin). The antitumor agent may be a monoclonal antibody or antibody drug conjugate, such as rituximab (Rituxan®), alemtuzumab (Campath®), Ipilimumab (Yervoy®), Bevacizumab (Avastin®), Cetuximab (Erbitux®), panitumumab (Vectibix®), and trastuzumab (Herceptin®), Tositumomab and 1311-tositumomab (Bexxar®), ibritumomab tiuxetan (Zevalin®), brentuximab vedotin (Adcetris®), siltuximab (Sylvant™), pembrolizumab (Keytruda®), ofatumumab (Arzerra®), obinutuzumab (Gazyva™), 90Y-ibritumomab tiuxetan, 1311-tositumomab, pertuzumab (Perjeta™), ado-trastuzumab emtansine (Kadcyla™), Denosumab (Xgeva®), and Ramucirumab (Cyramza™). The antitumor agent may be a small molecule kinase inhibitor, such as Vemurafenib (Zelboraf®), imatinib mesylate (Gleevec®), erlotinib (Tarceva®), gefitinib (Iressa®), , lapatinib (Tykerb®), regorafenib (Stivarga®), sunitinib (Sutent®), sorafenib (Nexavar®), pazopanib (Votrient®), axitinib (Inlyta®), dasatinib (Sprycel®), nilotinib (Tasigna®), bosutinib (Bosulif®), ibrutinib (Imbruvica™), idelalisib (Zydelig®), crizotinib (Xalkori®), afatinib dimaleate (Gilotrif®), ceritinib (LDK378/Zykadia), trametinib(Mekinist®), dabrafenib (Tafinlar®), Cabozantinib (Cometriq™), vandetanib (Caprelsa®), The antitumor agent may be a proteosome inhibitor, such as bortezomib (Velcade®) and carfilzomib (Kyprolis®). Optionally, the antitumor agent is a neoantigen. Neoantigens are tumor-associated peptides that serve as active pharmaceutical ingredients of vaccine compositions that stimulate antitumor responses and are described in US patent number 9,115,402. The antitumor agent may be a cytokine such as interferons (INFs), interleukins (ILs), or hematopoietic growth factors. The antitumor agent may be INF-a, IL-2, Aldesleukin IL-2, Erythropoietin, Granulocyte-macrophage colony-stimulating factor (GM-CSF) or granulocyte colony-stimulating factor. The antitumor agent may be a targeted therapy such as toremifene (Fareston®), fulvestrant (Faslodex®), anastrozole (Arimidex®), exemestane (Aromasin®), letrozole (Femara®), ziv-aflibercept (Zaltrap®), Alitretinoin (Panretin®), temsirolimus (Torisel®), Tretinoin (Vesanoid®), denileukin diftitox (Ontak®), vorinostat (Zolinza®), romidepsin (Istodax®), bexarotene (Targretin®), pralatrexate (Folotyn®), lenaliomide (Revlimid®), belinostat (Beleodaq™), lenaliomide (Revlimid®), pomalidomide (Pomalyst®), Cabazitaxel (Jevtana®), enzalutamide (Xtandi®), abiraterone acetate (Zytiga®), radium 223 chloride (Xofigo®), or everolimus (Afinitor®). The antitumor agent may be a checkpoint inhibitor such as an inhibitor of the programmed death-1 (PD-1) pathway, for example an anti-PDI antibody (Nivolumab). The inhibitor may be an anti-cytotoxic T-lymphocyte-associated antigen (CTLA-4) antibody. The inhibitor may target another member of the CD28 CTLA4 Ig superfamily such as BTLA, LAG3, ICOS, PDL1 or KIR. A checkpoint inhibitor may target a member of the TNFR superfamily such as CD40, OX40, CD 137, GITR, CD27 or TIM-3. Additionally, the antitumor agent may be an epigenetic targeted drug such as HDAC inhibitors, kinase inhibitors, DNA methyltransferase inhibitors, histone demethylase inhibitors, or histone methylation inhibitors. The epigenetic drugs may be Azacitidine (Vidaza), Decitabine (Dacogen), Vorinostat (Zolinza), Romidepsin (Istodax), or Ruxolitinib (Jakafi).

In certain embodiments, the perturbation is an agent capable of modulating gene expression. In certain embodiments, the perturbation comprises a CRISPR system, RNAi, TALE, or zinc finger protein. Not being bound by a theory, targeting one or more genomic loci (e.g., genes) may have an effect on mutation rate. Cells may be obtained that already express a CRISPR system and guide RNA for a specific target. The CRISPR system may be inducible, such that the CRISPR system may be induced and lineage followed.

The present disclosure may be further illustrated based on aspects of CRISPR-Cas development and use as set forth in the following articles and particularly as relates to delivery of a CRISPR protein complex and uses of an RNA guided endonuclease in cells and organisms:

> Multiplex genome engineering using CRISPR-Cas systems. Cong, L., Ran, F.A., Cox, D., Lin, S., Barretto, R., Habib, N., Hsu, P.D., Wu, X., Jiang, W., Marraffini, L.A., & Zhang, F. Science Feb 15;339(6121):819-23 (2013);

> RNA-guided editing of bacterial genomes using CRISPR-Cas systems. Jiang W., Bikard D., Cox D., Zhang F, Marraffini LA. Nat Biotechnol Mar;31(3):233-9 (2013);

> One-Step Generation of Mice Carrying Mutations in Multiple Genes by CRISPR-Cas-Mediated Genome Engineering. Wang H., Yang H., Shivalila CS., Dawlaty MM., Cheng AW., Zhang F., JaemschR. Cell May 9;153(4):910-8 (2013);

> Optical control of mammalian endogenous transcription and epigenetic states. Konermann S, Brigham MD, Trevino AE, Hsu PD, Heidenreich M, Cong L, Platt RJ, Scott DA, Church GM, Zhang F. Nature. Aug 22;500(7463):472-6. doi: 10.1038/Nature12466. Epub 2013 Aug 23 (2013);

> Double Nicking by RNA-Guided CRISPR Cas9 for Enhanced Genome Editing Specificity. Ran, FA., Hsu, PD., Lin, CY., Gootenberg, JS., Konermann, S., Trevino, AE., Scott, DA., Inoue, A., Matoba, S., Zhang, Y., & Zhang, F. Cell Aug 28. pii: S0092-8674(13)01015-5 (2013-A);

> DNA targeting specificity of RNA-guided Cas9 nucleases. Hsu, P., Scott, D., Weinstein, J., Ran, FA., Konermann, S., Agarwala, V., Li, Y., Fine, E., Wu, X., Shalem, O., Cradick, TJ., Marraffini, LA., Bao, G., & Zhang, F. Nat Biotechnol doi:10.1038/nbt.2647 (2013);

> Genome engineering using the CRISPR-Cas9 system. Ran, FA., Hsu, PD., Wright, J., Agarwala, V., Scott, DA., Zhang, F. Nature Protocols Nov;8(11):2281-308 (2013-B);

> Genome-Scale CRISPR-Cas9 Knockout Screening in Human Cells. Shalem, O., Sanjana, NE., Hartenian, E., Shi, X., Scott, DA., Mikkelson, T., Heckl, D., Ebert, BL., Root, DE., Doench, JG., Zhang, F. Science Dec 12. (2013);

> Crystal structure of cas9 in complex with guide RNA and target DNA. Nishimasu, H., Ran, FA., Hsu, PD., Konermann, S., Shehata, SI., Dohmae, N., Ishitani, R., Zhang, F., Nureki, O. Cell Feb 27, 156(5):935-49 (2014);

> Genome-wide binding of the CRISPR endonuclease Cas9 in mammalian cells. Wu X., Scott DA., Kriz AJ., Chiu AC., Hsu PD., Dadon DB., Cheng AW., Trevino AE., Konermann S., Chen S., Jaenisch R., Zhang F., Sharp PA. Nat Biotechnol. Apr 20. doi: 10.1038/nbt.2889 (2014);

> CRISPR-Cas9 Knockin Mice for Genome Editing and Cancer Modeling. Platt RJ, Chen S, Zhou Y, Yim MJ, Swiech L, Kempton HR, Dahlman JE, Parnas O, Eisenhaure TM, Jovanovic M, Graham DB, Jhunjhunwala S, Heidenreich M, Xavier RJ, Langer R, Anderson DG, Hacohen N, Regev A, Feng G, Sharp PA, Zhang F. Cell 159(2): 440-455 DOI: 10.1016/j.cell.2014.09.014(2014);

> Development and Applications of CRISPR-Cas9 for Genome Engineering, Hsu PD, Lander ES, Zhang F., Cell. Jun 5;157(6):1262-78 (2014).

> Genetic screens in human cells using the CRISPR-Cas9 system, Wang T, Wei JJ, Sabatini DM, Lander ES., Science. January 3; 343(6166): 80-84. doi: 10.1126/science. 1246981 (2014);

> Rational design of highly active sgRNAs for CRISPR-Cas9-mediated gene inactivation, Doench JG, Hartenian E, Graham DB, Tothova Z, Hegde M, Smith I, Sullender M, Ebert BL, Xavier RJ, Root DE., (published online 3 September 2014) Nat Biotechnol. Dec;32(12):1262-7 (2014);

> In vivo interrogation of gene function in the mammalian brain using CRISPR-Cas9, Swiech L, Heidenreich M, Banerjee A, Habib N, Li Y, Trombetta J, Sur M, Zhang F., (published online 19 October 2014) Nat Biotechnol. Jan;33(1): 102-6 (2015);

> Genome-scale transcriptional activation by an engineered CRISPR-Cas9 complex, Konermann S, Brigham MD, Trevino AE, Joung J, Abudayyeh OO, Barcena C, Hsu PD, Habib N, Gootenberg JS, Nishimasu H, Nureki O, Zhang F., Nature. Jan 29;517(7536):583-8 (2015).

> A split-Cas9 architecture for inducible genome editing and transcription modulation, Zetsche B, Volz SE, Zhang F., (published online 02 February 2015) Nat Biotechnol. Feb;33(2):139-42 (2015);

> Genome-wide CRISPR Screen in a Mouse Model of Tumor Growth and Metastasis, Chen S, Sanjana NE, Zheng K, Shalem O, Lee K, Shi X, Scott DA, Song J, Pan JQ, Weissleder R, Lee H, Zhang F, Sharp PA. Cell 160, 1246-1260, March 12, 2015 (multiplex screen in mouse), and

> In vivo genome editing using Staphylococcus aureus Cas9, Ran FA, Cong L, Yan WX, Scott DA, Gootenberg JS, Kriz AJ, Zetsche B, Shalem O, Wu X, Makarova KS, Koonin EV, Sharp PA, Zhang F., (published online 01 April 2015), Nature. Apr 9;520(7546): 186-91 (2015).

> Shalem et al., "High-throughput functional genomics using CRISPR-Cas9," Nature Reviews Genetics 16, 299-311 (May 2015).

> Xu et al., "Sequence determinants of improved CRISPR sgRNA design," Genome Research 25, 1147-1157 (August 2015).

> Parnas et al., "A Genome-wide CRISPR Screen in Primary Immune Cells to Dissect Regulatory Networks," Cell 162, 675-686 (July 30, 2015).

> Ramanan et al., CRISPR-Cas9 cleavage of viral DNA efficiently suppresses hepatitis B virus," Scientific Reports 5:10833. doi: 10.1038/srep10833 (June 2, 2015)

> Nishimasu et al., Crystal Structure of Staphylococcus aureus Cas9," Cell 162, 1113-1126 (Aug. 27, 2015)

> BCL11A enhancer dissection by Cas9-mediated in situ saturating mutagenesis, Canver et al., Nature 527(7577):192-7 (Nov. 12, 2015) doi: 10.1038/nature15521. Epub 2015 Sep 16.

> Cpf1 Is a Single RNA-Guided Endonuclease of a Class 2 CRISPR-Cas System, Zetsche et al., Cell 163, 759-71 (Sep 25, 2015).

> Discovery and Functional Characterization of Diverse Class 2 CRISPR-Cas Systems, Shmakov et al., Molecular Cell, 60(3), 385-397 doi: 10.1016/j.molcel.2015.10.008 Epub October 22, 2015.

> Rationally engineered Cas9 nucleases with improved specificity, Slaymaker et al., Science 2016 Jan 1 351(6268): 84-88 doi: 10.1126/science.aad5227. Epub 2015 Dec 1.

> Gao et al, "Engineered Cpf1 Enzymes with Altered PAM Specificities," bioRxiv 091611; doi: http://dx.doi.org/10.1101/091611 (Dec. 4, 2016).

> Cox et al., "RNA editing with CRISPR-Cas13," Science. 2017 Nov 24;358(6366):1019-1027. doi: 10.1126/science.aaq0180. Epub 2017 Oct 25.

each of which is discussed briefly below:

> Cong *et al.* engineered type II CRISPR-Cas systems for use in eukaryotic cells based on both *Streptococcus thermophilus* Ca9 and also *Streptococcus pyogenes* Cas9 and demonstrated that Cas9 nucleases can be directed by short RNAs to induce precise cleavage of DNA in human and mouse cells. Their study further showed that Cas9 as converted into a nicking enzyme can be used to facilitate homology-directed repair in eukaryotic cells with minimal mutagenic activity. Additionally, their study demonstrated that multiple guide sequences can be encoded into a single CRISPR array to enable simultaneous editing of several at endogenous genomic loci sites within the mammalian genome, demonstrating easy programmability and wide applicability of the RNA-guided nuclease technology. This ability to use RNA to program sequence specific DNA cleavage in cells defined a new class of genome engineering tools. These studies further showed that other CRISPR loci are likely to be transplantable into mammalian cells and can also mediate mammalian genome cleavage. Importantly, it can be envisaged that several aspects of the CRISPR-Cas system can be further improved to increase its efficiency and versatility.

> Jiang *et al.* used the clustered, regularly interspaced, short palindromic repeats (CRISPR)-associated Cas9 endonuclease complexed with dual-RNAs to introduce precise mutations in the genomes of *Streptococcus pneumoniae* and *Escherichia coli.* The approach relied on dual-RNA:Cas9-directed cleavage at the targeted genomic site to kill unmutated cells and circumvents the need for selectable markers or counter-selection systems. The study reported reprogramming dual-RNA:Cas9 specificity by changing the sequence of short CRISPR RNA (crRNA) to make single- and multinucleotide changes carried on editing templates. The study showed that simultaneous use of two crRNAs enabled multiplex mutagenesis. Furthermore, when the approach was used in combination with recombineering, in *S. pneumoniae,* nearly 100% of cells that were recovered using the described approach contained the desired mutation, and in *E. coli,* 65% that were recovered contained the mutation.

> Wang *et al.* (2013) used the CRISPR-Cas system for the one-step generation of mice carrying mutations in multiple genes which were traditionally generated in multiple steps by sequential recombination in embryonic stem cells and/or time-consuming intercrossing of mice with a single mutation. The CRISPR-Cas system will greatly accelerate the *in vivo* study of functionally redundant genes and of epistatic gene interactions.

> Konermann *et al.* (2013) addressed the need in the art for versatile and robust technologies that enable optical and chemical modulation of DNA-binding domains based CRISPR Cas9 enzyme and also Transcriptional Activator Like Effectors

> Ran *et al.* (2013-A) described an approach that combined a Cas9 nickase mutant with paired guide RNAs to introduce targeted double-strand breaks. This addresses the issue of the Cas9 nuclease from the microbial CRISPR-Cas system being targeted to specific genomic loci by a guide sequence, which can tolerate certain mismatches to the DNA target and thereby promote undesired off-target mutagenesis. Because individual nicks in the genome are repaired with high fidelity, simultaneous nicking *via* appropriately offset guide RNAs is required for double-stranded breaks and extends the number of specifically recognized bases for target cleavage. The authors demonstrated that using paired nicking can reduce off-target activity by 50- to 1,500-fold in cell lines and to facilitate gene knockout in mouse zygotes without sacrificing on-target cleavage efficiency. This versatile strategy enables a wide variety of genome editing applications that require high specificity.

> Hsu *et al.* (2013) characterized SpCas9 targeting specificity in human cells to inform the selection of target sites and avoid off-target effects. The study evaluated >700 guide RNA variants and SpCas9-induced indel mutation levels at >100 predicted genomic off-target loci in 293T and 293FT cells. The authors that SpCas9 tolerates mismatches between guide RNA and target DNA at different positions in a sequence-dependent manner, sensitive to the number, position and distribution of mismatches. The authors further showed that SpCas9-mediated cleavage is unaffected by DNA methylation and that the dosage of SpCas9 and guide RNA can be titrated to minimize off-target modification. Additionally, to facilitate mammalian genome engineering applications, the authors reported providing a web-based software tool to guide the selection and validation of target sequences as well as off-target analyses.

> Ran *et al.* (2013-B) described a set of tools for Cas9-mediated genome editing *via* non-homologous end joining (NHEJ) or homology-directed repair (HDR) in mammalian cells, as well as generation of modified cell lines for downstream functional studies. To minimize off-target cleavage, the authors further described a double-nicking strategy using the Cas9 nickase mutant with paired guide RNAs. The protocol provided by the authors experimentally derived guidelines for the selection of target sites, evaluation of cleavage efficiency and analysis of off-target activity. The studies showed that beginning with target design, gene modifications can be achieved within as little as 1-2 weeks, and modified clonal cell lines can be derived within 2-3 weeks.

> Shalem *et al.* described a new way to interrogate gene function on a genome-wide scale. Their studies showed that delivery of a genome-scale CRISPR-Cas9 knockout (GeCKO) library targeted 18,080 genes with 64,751 unique guide sequences enabled both negative and positive selection screening in human cells. First, the authors showed use of the GeCKO library to identify genes essential for cell viability in cancer and pluripotent stem cells. Next, in a melanoma model, the authors screened for genes whose loss is involved in resistance to vemurafenib, a therapeutic that inhibits mutant protein kinase BRAF. Their studies showed that the highest-ranking candidates included previously validated genes NF1 and MED12 as well as novel hits NF2, CLTL3, TADA2B, and TADA1. The authors observed a high level of consistency between independent guide RNAs targeting the same gene and a high rate of hit confirmation, and thus demonstrated the promise of genome-scale screening with Cas9.

> Nishimasu *et al.* reported the crystal structure of *Streptococcus pyogenes* Cas9 in complex with sgRNA and its target DNA at 2.5 A° resolution. The structure revealed a bilobed architecture composed of target recognition and nuclease lobes, accommodating the sgRNA:DNA heteroduplex in a positively charged groove at their interface. Whereas the recognition lobe is essential for binding sgRNA and DNA, the nuclease lobe contains the HNH and RuvC nuclease domains, which are properly positioned for cleavage of the complementary and non-complementary strands of the target DNA, respectively. The nuclease lobe also contains a carboxyl-terminal domain responsible for the interaction with the protospacer adjacent motif (PAM). This high-resolution structure and accompanying functional analyses have revealed the molecular mechanism of RNA-guided DNA targeting by Cas9, thus paving the way for the rational design of new, versatile genome-editing technologies.

> Wu *et al.* mapped genome-wide binding sites of a catalytically inactive Cas9 (dCas9) from *Streptococcus pyogenes* loaded with single guide RNAs (sgRNAs) in mouse embryonic stem cells (mESCs). The authors showed that each of the four sgRNAs tested targets dCas9 to between tens and thousands of genomic sites, frequently characterized by a 5-nucleotide seed region in the sgRNA and an NGG protospacer adjacent motif (PAM). Chromatin inaccessibility decreases dCas9 binding to other sites with matching seed sequences; thus 70% of off-target sites are associated with genes. The authors showed that targeted sequencing of 295 dCas9 binding sites in mESCs transfected with catalytically active Cas9 identified only one site mutated above background levels. The authors proposed a two-state model for Cas9 binding and cleavage, in which a seed match triggers binding but extensive pairing with target DNA is required for cleavage.

> Platt *et al.* established a Cre-dependent Cas9 knockin mouse. The authors demonstrated *in vivo* as well as *ex vivo* genome editing using adeno-associated virus (AAV)-, lentivirus-, or particle-mediated delivery of guide RNA in neurons, immune cells, and endothelial cells.

> Hsu *et al.* (2014) is a review article that discusses generally CRISPR-Cas9 history from yogurt to genome editing, including genetic screening of cells.

> Wang *et al.* (2014) relates to a pooled, loss-of-function genetic screening approach suitable for both positive and negative selection that uses a genome-scale lentiviral single guide RNA (sgRNA) library.

> Doench *et al.* created a pool of sgRNAs, tiling across all possible target sites of a panel of six endogenous mouse and three endogenous human genes and quantitatively assessed their ability to produce null alleles of their target gene by antibody staining and flow cytometry. The authors showed that optimization of the PAM improved activity and also provided an on-line tool for designing sgRNAs.

> Swiech *et al.* demonstrate that AAV-mediated SpCas9 genome editing can enable reverse genetic studies of gene function in the brain.

> Konermann *et al.* (2015) discusses the ability to attach multiple effector domains, e.g., transcriptional activator, functional and epigenomic regulators at appropriate positions on the guide such as stem or tetraloop with and without linkers.

> Zetsche *et al.* demonstrates that the Cas9 enzyme can be split into two and hence the assembly of Cas9 for activation can be controlled.

> Chen *et al.* relates to multiplex screening by demonstrating that a genome-wide *in vivo* CRISPR-Cas9 screen in mice reveals genes regulating lung metastasis.

> Ran *et al.* (2015) relates to SaCas9 and its ability to edit genomes and demonstrates that one cannot extrapolate from biochemical assays.

> Shalem *et al.* (2015) described ways in which catalytically inactive Cas9 (dCas9) fusions are used to synthetically repress (CRISPRi) or activate (CRISPRa) expression, showing. advances using Cas9 for genome-scale screens, including arrayed and pooled screens, knockout approaches that inactivate genomic loci and strategies that modulate transcriptional activity.

> Xu *et al.* (2015) assessed the DNA sequence features that contribute to single guide RNA (sgRNA) efficiency in CRISPR-based screens. The authors explored efficiency of CRISPR-Cas9 knockout and nucleotide preference at the cleavage site. The authors also found that the sequence preference for CRISPRi/a is substantially different from that for CRISPR-Cas9 knockout.

> Parnas *et al.* (2015) introduced genome-wide pooled CRISPR-Cas9 libraries into dendritic cells (DCs) to identify genes that control the induction of tumor necrosis factor (Tnf) by bacterial lipopolysaccharide (LPS). Known regulators of Tlr4 signaling and previously unknown candidates were identified and classified into three functional modules with distinct effects on the canonical responses to LPS.

> Ramanan *et al* (2015) demonstrated cleavage of viral episomal DNA (cccDNA) in infected cells. The HBV genome exists in the nuclei of infected hepatocytes as a 3.2kb double-stranded episomal DNA species called covalently closed circular DNA (cccDNA), which is a key component in the HBV life cycle whose replication is not inhibited by current therapies. The authors showed that sgRNAs specifically targeting highly conserved regions of HBV robustly suppresses viral replication and depleted cccDNA.

> Nishimasu *et al.* (2015) reported the crystal structures of SaCas9 in complex with a single guide RNA (sgRNA) and its double-stranded DNA targets, containing the 5'-TTGAAT-3' PAM and the 5'-TTGGGT-3' PAM. A structural comparison of SaCas9 with SpCas9 highlighted both structural conservation and divergence, explaining their distinct PAM specificities and orthologous sgRNA recognition.

> Canver *et al.* (2015) demonstrated a CRISPR-Cas9-based functional investigation of non-coding genomic elements. The authors we developed pooled CRISPR-Cas9 guide RNA libraries to perform *in situ* saturating mutagenesis of the human and mouse BCL11A enhancers which revealed critical features of the enhancers.

> Zetsche et al. (2015) reported characterization of Cpf1, a class 2 CRISPR nuclease from Francisella novicida U112 having features distinct from Cas9. Cpf1 is a single RNA-guided endonuclease lacking tracrRNA, utilizes a T-rich protospacer-adjacent motif, and cleaves DNA via a staggered DNA double-stranded break.

> Shmakov et al. (2015) reported three distinct Class 2 CRISPR-Cas systems. Two system CRISPR enzymes (C2c1 and C2c3) contain RuvC-like endonuclease domains distantly related to Cpf1. Unlike Cpf1, C2c1 depends on both crRNA and tracrRNA for DNA cleavage. The third enzyme (C2c2) contains two predicted HEPN RNase domains and is tracrRNA independent.

> Slaymaker et al (2016) reported the use of structure-guided protein engineering to improve the specificity of Streptococcus pyogenes Cas9 (SpCas9). The authors developed "enhanced specificity" SpCas9 (eSpCas9) variants which maintained robust on-target cleavage with reduced off-target effects.

> Cox et al., (2017) reported the use of catalytically inactive Cas13 (dCas13) to direct adenosine-to-inosine deaminase activity by ADAR2 (adenosine deaminase acting on RNA type 2) to transcripts in mammalian cells. The system, referred to as RNA Editing for Programmable A to I Replacement (REPAIR), has no strict sequence constraints and can be used to edit full-length transcripts. The authors further engineered the system to create a high-specificity variant and minimized the system to facilitate viral delivery.

Also, "Dimeric CRISPR RNA-guided FokI nucleases for highly specific genome editing", Shengdar Q. Tsai, Nicolas Wyvekens, Cyd Khayter, Jennifer A. Foden, Vishal Thapar, Deepak Reyon, Mathew J. Goodwin, Martin J. Aryee, J. Keith Joung Nature Biotechnology 32(6): 569-77 (2014), relates to dimeric RNA-guided FokI Nucleases that recognize extended sequences and can edit endogenous genes with high efficiencies in human cells.

One type of programmable DNA-binding domain is provided by artificial zinc-finger (ZF) technology, which involves arrays of ZF modules to target new DNA-binding sites in the genome. Each finger module in a ZF array targets three DNA bases. A customized array of individual zinc finger domains is assembled into a ZF protein (ZFP).

ZFPs can comprise a functional domain. The first synthetic zinc finger nucleases (ZFNs) were developed by fusing a ZF protein to the catalytic domain of the Type IIS restriction enzyme FokI. (Kim, Y. G. et al., 1994, Chimeric restriction endonuclease, Proc. Natl. Acad. Sci. U.S.A. 91, 883-887; Kim, Y. G. et al., 1996, Hybrid restriction enzymes: zinc finger fusions to Fok I cleavage domain. Proc. Natl. Acad. Sci. U.S.A. 93, 1156-1160). Increased cleavage specificity can be attained with decreased off target activity by use of paired ZFN heterodimers, each targeting different nucleotide sequences separated by a short spacer. (Doyon, Y. et al., 2011, Enhancing zinc-finger-nuclease activity with improved obligate heterodimeric architectures. Nat. Methods 8, 74-79). ZFPs can also be designed as transcription activators and repressors and have been used to target many genes in a wide variety of organisms.

Naturally occurring TALEs or "wild type TALEs" are nucleic acid binding proteins secreted by numerous species of proteobacteria. TALE polypeptides contain a nucleic acid binding domain composed of tandem repeats of highly conserved monomer polypeptides that are predominantly 33, 34 or 35 amino acids in length and that differ from each other mainly in amino acid positions 12 and 13. In advantageous embodiments the nucleic acid is DNA. As used herein, the term "polypeptide monomers", "TALE monomers" or "monomers" will be used to refer to the highly conserved repetitive polypeptide sequences within the TALE nucleic acid binding domain and the term "repeat variable di-residues" or "RVD" will be used to refer to the highly variable amino acids at positions 12 and 13 of the polypeptide monomers. As provided throughout the disclosure, the amino acid residues of the RVD are depicted using the IUPAC single letter code for amino acids. A general representation of a TALE monomer which is comprised within the DNA binding domain is X1-11-(X12X13)-X14-33 or 34 or 35, where the subscript indicates the amino acid position and X represents any amino acid. X12X13 indicate the RVDs. In some polypeptide monomers, the variable amino acid at position 13 is missing or absent and in such monomers, the RVD consists of a single amino acid. In such cases the RVD may be alternatively represented as X*, where X represents X12 and (*) indicates that X13 is absent. The DNA binding domain comprises several repeats of TALE monomers and this may be represented as (X1-11-(X12X13)-X14-33 or 34 or 35)z, where in an advantageous embodiment, z is at least 5 to 40. In a further advantageous embodiment, z is at least 10 to 26.

The TALE monomers have a nucleotide binding affinity that is determined by the identity of the amino acids in its RVD. For example, polypeptide monomers with an RVD of NI preferentially bind to adenine (A), monomers with an RVD of NG preferentially bind to thymine (T), monomers with an RVD of HD preferentially bind to cytosine (C) and monomers with an RVD of NN preferentially bind to both adenine (A) and guanine (G). In yet another embodiment of the disclosure, monomers with an RVD of IG preferentially bind to T. Thus, the number and order of the polypeptide monomer repeats in the nucleic acid binding domain of a TALE determines its nucleic acid target specificity. In still further embodiments of the disclosure, monomers with an RVD of NS recognize all four base pairs and may bind to A, T, G or C. The structure and function of TALEs is further described in, for example, Moscou et al., Science 326:1501 (2009); Boch et al., Science 326:1509-1512 (2009); and Zhang et al., Nature Biotechnology 29:149-153 (2011).

## Computer Systems

The system, as described in the present technique or any of its components, may be embodied in the form of a computer system. Typical examples of a computer system includes a general-purpose computer, a programmed microprocessor, a micro-controller, a peripheral integrated circuit element, and other devices or arrangements of devices that are capable of implementing the steps that constitute the method of the present technique.

The computer system may comprise a computer, an input device, a display unit and/or the Internet. The computer further comprises a microprocessor. The microprocessor is connected to a communication bus. The computer also includes a memory. The memory may include Random Access Memory (RAM) and Read Only Memory (ROM). The computer system further comprises a storage device. The storage device can be a hard disk drive or a removable storage drive such as a floppy disk drive, optical disk drive, etc. The storage device can also be other similar means for loading computer programs or other instructions into the computer system. The computer system also includes a communication unit. The communication unit allows the computer to connect to other databases and the Internet through an I/O interface. The communication unit allows the transfer as well as reception of data from other databases. The communication unit may include a modem, an Ethernet card, or any similar device which enables the computer system to connect to databases and networks such as LAN, MAN, WAN and the Internet. The computer system facilitates inputs from a user through input device, accessible to the system through I/O interface.

The computer system executes a set of instructions that are stored in one or more storage elements, in order to process input data. The storage elements may also hold data or other information as desired. The storage element may be in the form of an information source or a physical memory element present in the processing machine.

The set of instructions may include various commands that instruct the processing machine to perform specific tasks such as the steps that constitute the method of the present technique.

The set of instructions may be in the form of a software program. Further, the software may be in the form of a collection of separate programs, a program module with a larger program or a portion of a program module, as in the present technique. The software may also include modular programming in the form of object-oriented programming. The processing of input data by the processing machine may be in response to user commands, results of previous processing or a request made by another processing machine.

The present invention advantageously provides several applications for the described methods. The present invention advantageously provides for identification and quantitative analysis of mutagenicity of different environmental exposures (physical, chemical, biological). The present invention advantageously provides for measuring the effect of drugs. This answer provides an unmet clinical need to determine the mutagenic effects of therapies prior to their widespread deployment, and can improve treatments to extend life expectancies for cancer patients. Using the described methods, it is feasible to quantify the effect of drugs on mutation accumulation phenotype and in addition to read the mutation specific signatures that might shed light on the affected mechanism. The present invention advantageously provides for characterization of mutation accumulation behavior of individuals, and measuring the drug response and DNA damage mechanism of individuals, by performing the method on specific patient derived stem cells or iPS.

The invention is further described in the following examples, which do not limit the scope of the invention described in the claims.

**EXAMPLES**

**Example 1** - **Lineage sequencing for precise assessment of somatic mutation flow in mammalian cells.**

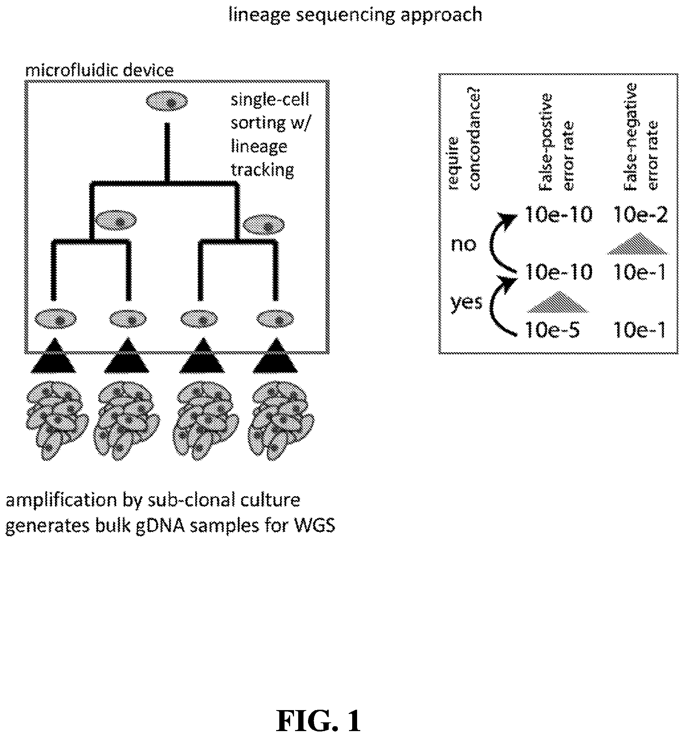

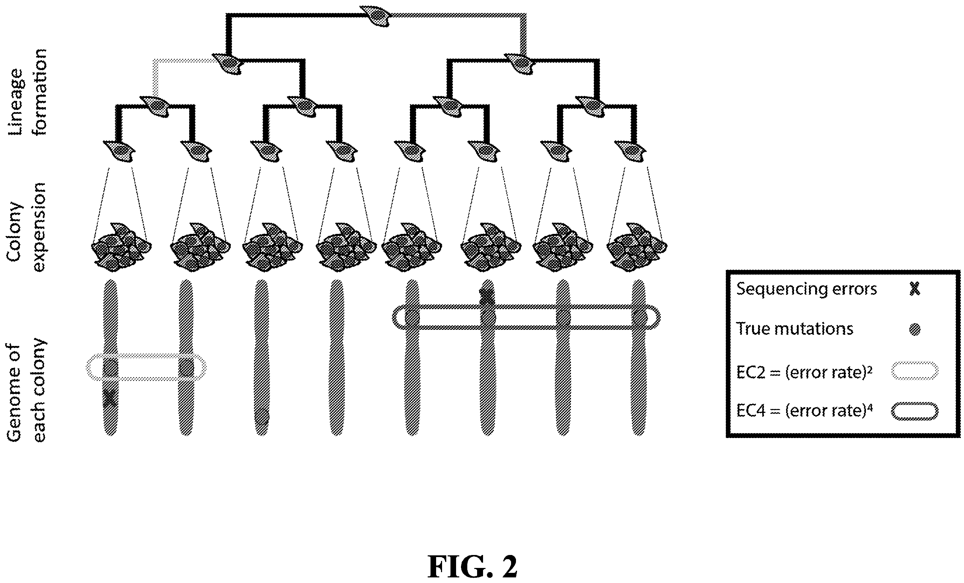

As described herein, somatic mutations steadily accumulate in our cells. While most somatic mutations are harmless, some lead to phenotypic consequences. Highly sensitive and specific detection of mutations by whole genome sequencing is desired. Figure 1 illustrates an overview of lineage sequencing in mammalian cells for the precise assessment of somatic mutation flow. Figure 1 also illustrates that by requiring concordance the false-positive rate (i.e., specificity) is increased from $10^{-5}$ to $10^{-10}$ and false-negative rate (i.e., sensitivity) remains $10^{-1}$. Figure 2 illustrates an overview of somatic mutation detection. Shown is the step of lineage formation from a single cell. In preferred embodiments, the cells are treated with a perturbation as described herein. Control experiments without perturbation are also encompassed by the present disclosure. After lineage formation, single cells are isolated as described herein. In preferred embodiments, single cells are separated by use of a microscope and either microfluidics or optical tweezer. Microfluidics coupled with imaging allows for each single cell in a lineage to be removed from the microfluidic device sequentially (e.g., one by one). An optical tweezer coupled with imaging may be used to move cells such that single cells may be expanded into single colonies. Upon colony expansion, bulk genomic DNA may be extracted using known methods in the art and the DNA analyzed by whole genome or whole exome sequencing without an amplification step. In preferred embodiments, whole genome sequencing is performed to detect mutations in coding and non-coding regions of DNA. In certain embodiments, mutations in only protein coding genes is desired, such as when interested in detecting the causes of non-silent mutations in protein coding genes. True mutations are observed in at least a pair of daughter cells.

The illustrated method provides dense sampling allowing reduced selection bias toward faster-dividing lineages that occurs when random single cells or sub-clones from samples of interest are obtained. The method allows known relationships between lineages because the cells are derived from a single cell over generations and the cells can be visualized over time. These known relationships allow the invention to have improved accuracy and/or sensitivity. The method allows the number of cell divisions relating to different sub-clones to be exactly known allowing for a quantitative mutation rate. Mutations that co-occurred in the same single-cell division event can be identified thereby allowing mutations to be spatially correlated. Mutation sets occurring in sequential single-cell division events can be compared thereby allowing mutations to be temporally correlated. Continuous microscopic observation enables recording of phenotypic correlates to mutations. The method also allows for the determination of the causes of mutations (e.g., effective repair, failed repair, distribution of fitness effects (DFE)).

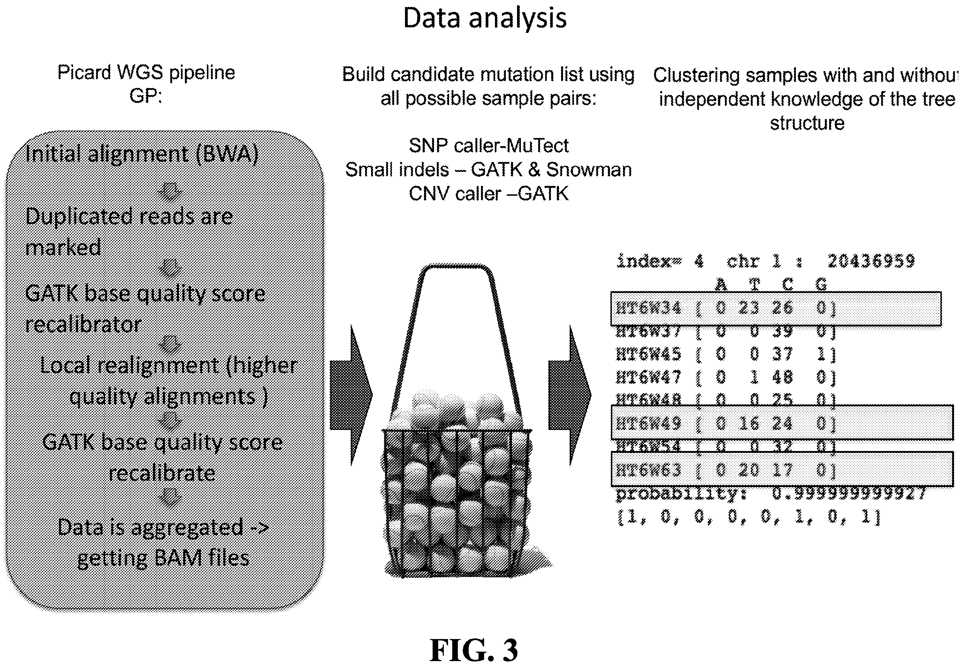

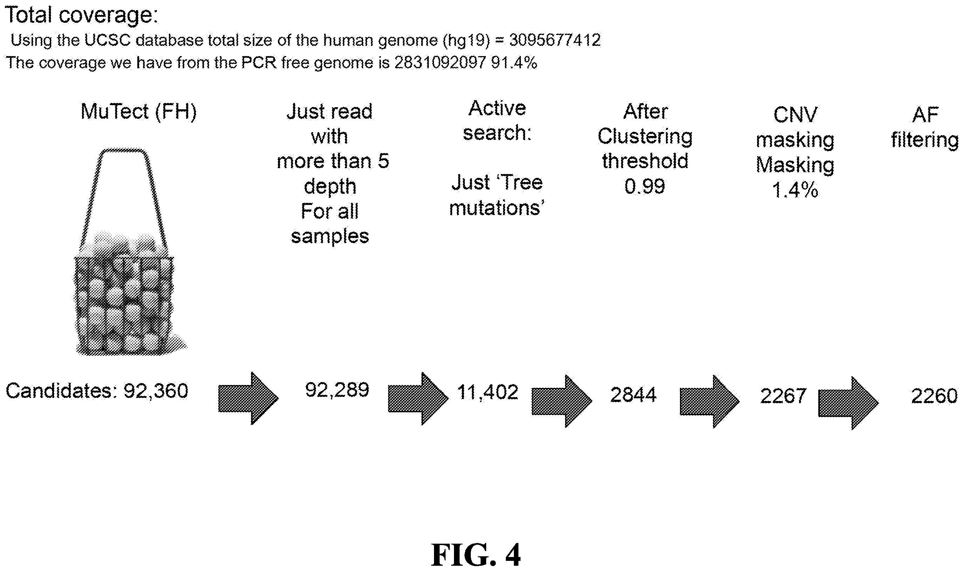

Figure 3 and 4 show flow charts describing data analysis once sequencing is performed. Applicants use common tools for generating 'candidates' of events including SNPs, insertions, deletions, and genomic structural variants, followed by filtering based on 'siblings' clustering over clonal mutation events. Software is available for use online. Local realignment and base recalibration were performed with GATK (www.broadinstitute.org/gatk/). Putative SNVs were called by comparing using MuTect (www.broadinstitute.org/cancer/cga/mutect). Structural variation and indel analysis by assembly (SvABA, formerly Snowman) can be used for small indels (github.com/walaj/svaba). Initial alignment of short-read sequences may be performed by any method known in the art. In an exemplary embodiment, alignment is performed by BWA, which uses a Burrows-Wheeler transform to create an index of the genome. This method provides accurate identification of genomic events that occurred inside the device during lineage development, and attributes each event to a specific known time frame. Using the UCSC database total size of the human genome (hg19) = 3095677412, the coverage Applicants obtained from the PCR free genome was 2831092097 (91.4%). The method only requires low read depths and can be performed with more than 5 depth for all samples. The filtering described (figure 4) resulted in 2260 candidates.

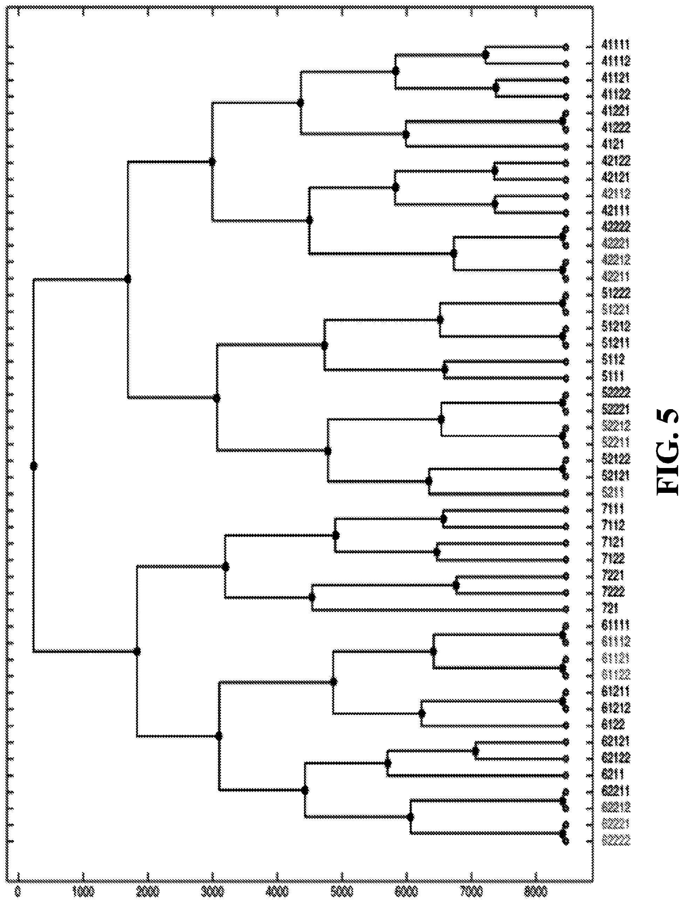

Figure 5 illustrates an example of mutation detection by lineage sequencing using a microfluidic device as described (Kimmerling et al., 2016). HT115 lineages marked by lighter shading were successfully sub-cloned. HT115 is a human colon epithelial cell line that has a polymerase epsilon mutation that increases the mutation rate in the leading strand resulting in a relatively high number of point mutation accumulates (V411L mutation in the exonuclease domain of POLE gene, MMR-proficient). The microfluidic platform enables direct observation of lineage growth that can be linked to the genomic data. PCR-free 30x whole human genome sequencing is performed for each sub-clonal lineage.

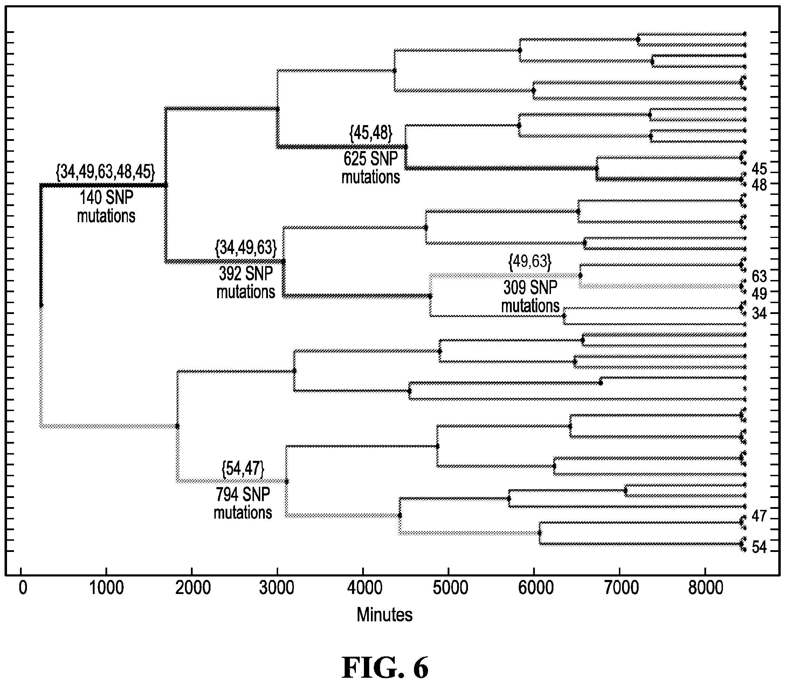

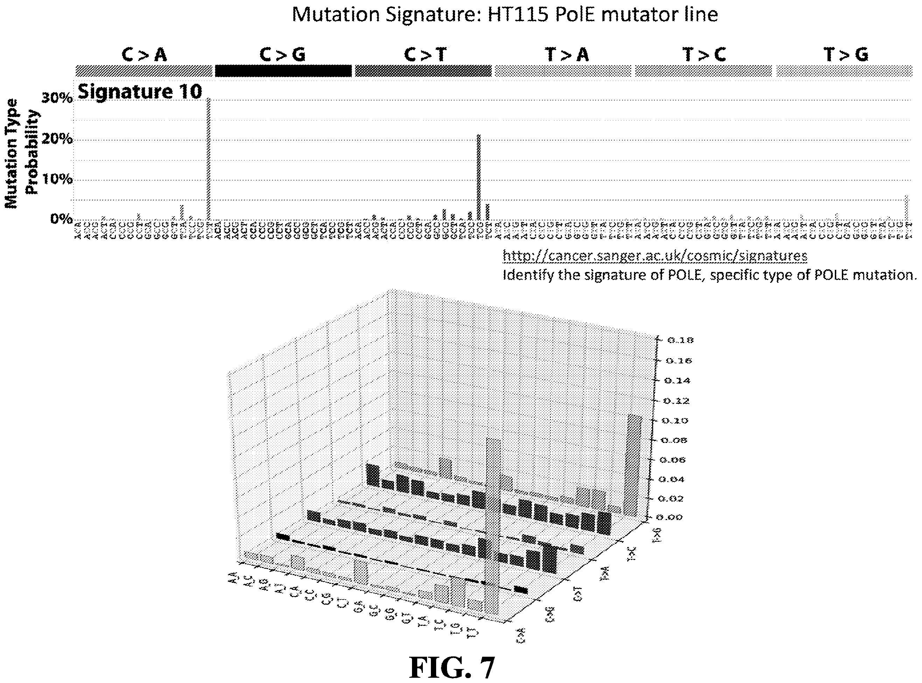

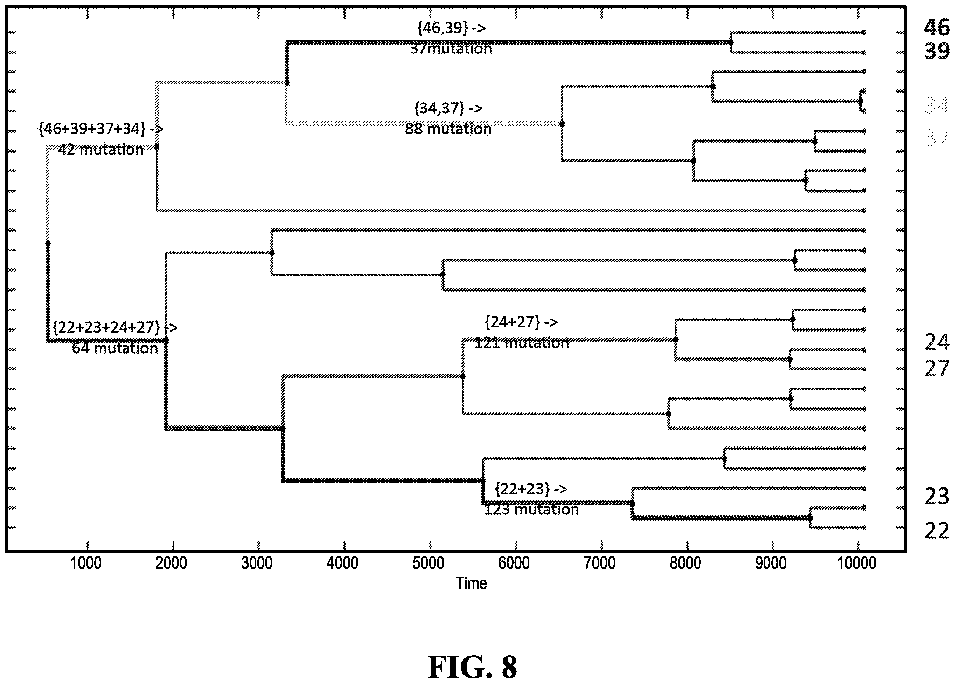

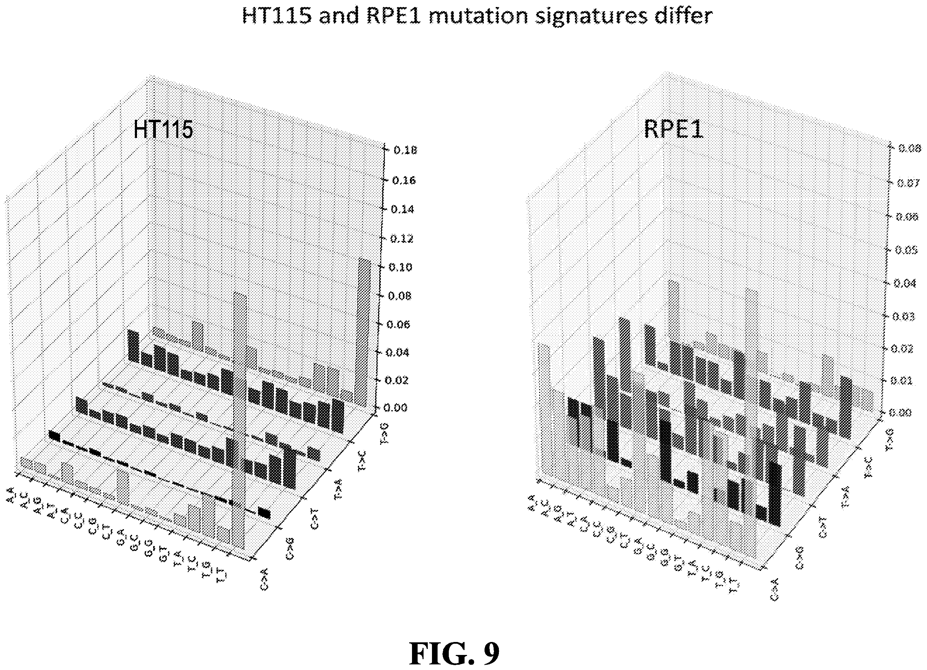

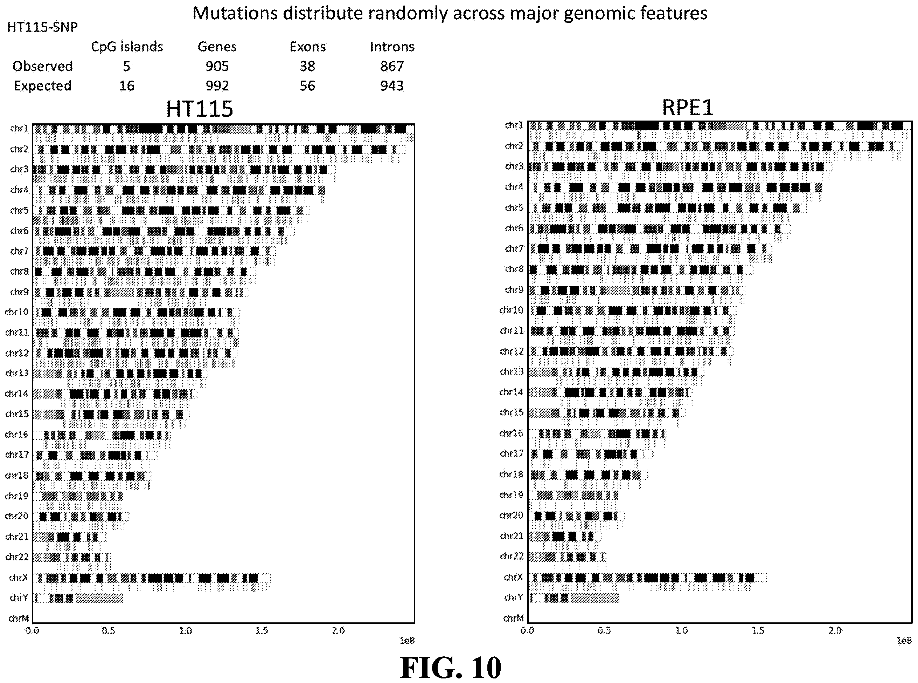

Figure 6 illustrates the flow of SNPs in the HT115 cell line that accumulate during 5-6 generations. Each number in the tree leafs is derived from a single cell expanded into a colony and whole genome sequenced. Each mutation can be traced back along the lineage. Figure 7 illustrates that a mutation signature can be determined. As used herein the term "mutation signature" refers to the type of mutations that occur in response to a cell line or perturbation, such as C to T mutations. The RPE1 cell line is a human retina epitheliala cell line that is a karyotypically normal non-mutator line. Figure 8 illustrates the flow of SNPs in the RPE1 cell line that accumulate during 5-6 generations. Applicants can also use the retained cultures and genomic DNA for validation. Figure 9 illustrates that the mutations signatures are different when comparing the two cell lines. Figure 10 illustrates that mutations distribute randomly across major genomic features in the two cell lines.

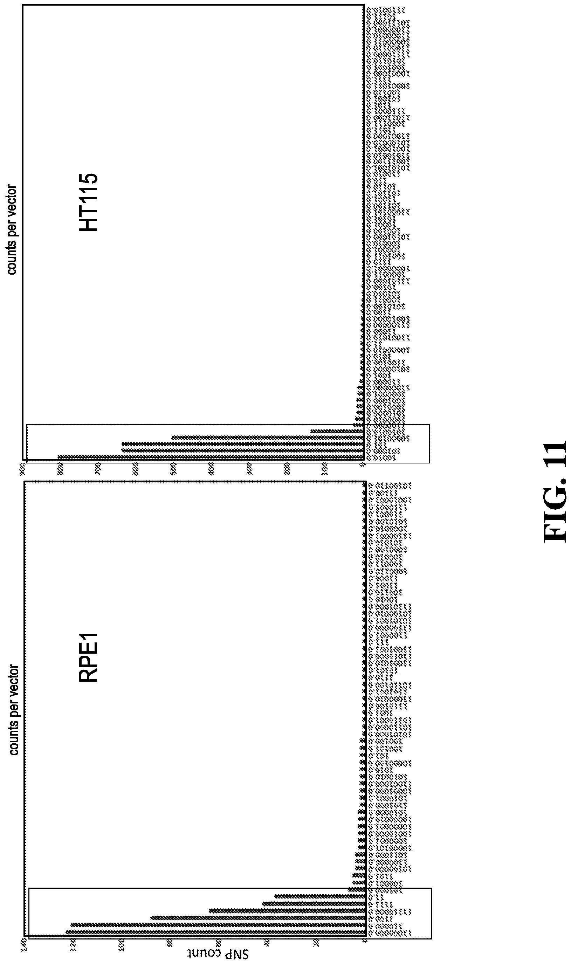

Figure 11 illustrates that lineage sequencing allows construction of trees without prior knowledge of lineage structure. Shown is data for HT115 and RPE1 cells. Variant clustering identified related sub-clone sets, even in the RPE1 cell line having few mutations. Complete sampling of cells enables perfect lineage reconstruction without tracking.

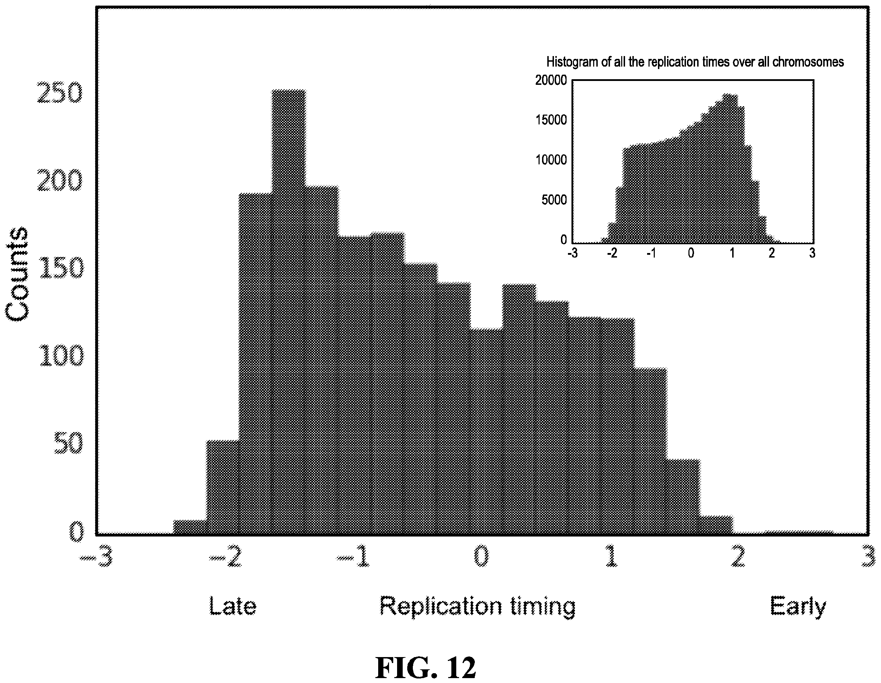

Figure 12 illustrates that late replication timing is associated with higher mutation rate in HT115 cells. This is consistent with prior studies (Koren et al. Cell 2014 Nov 20;159(5): 1015-26).

Overall, lineage sequencing enables accurate single-cell sequencing by replacing WGA with culture and applying scalable error correction. The lineage sequencing variant calls are consistent with sensitive and accurate detection of mutations with single-generation resolution.

**Example 2 - Lineage sequencing for precise assessment of mutation flow in bacterial cells.**

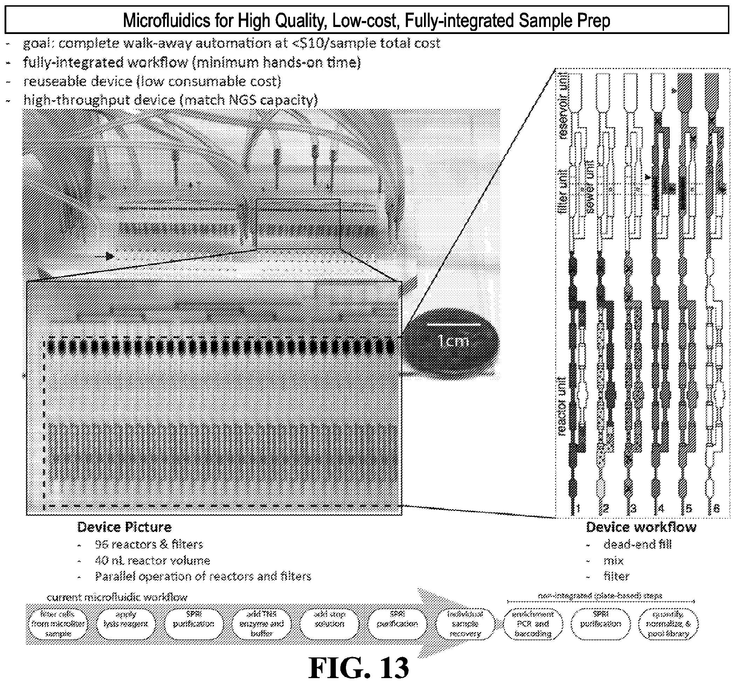

While low-cost DNA sequencing is transforming biological research and discovery, preparing large sample sets for sequencing with minuscule starting material is now the limiting factor in many applications. Applicants have developed two polydimethlysiloxane (PDMS) microfluidic devices that automates the key steps in whole genome sequencing (WGS) sample preparation, integrating lysis, fragmentation, adapter tagging, purification, and size selection of 96 samples in parallel (Fig. 13; see also, International Publication Numbers WO 2015/050998 A2 and WO 2016/138290). Applicants applied the first device to prepare about 500 WGS libraries in a study of 124 *Pseudomonas aeruginosa* clinical isolates, Mycobacterium tuberculosis, soil microbes, and human gut microbes using >100 times lower sample input and reagent quantities. These microfluidic libraries showed excellent coverage for variant calling, phylotyping, metabolic profiling, and metagenomic analysis performance from less than 10,000 cells (50 picograms of genomic DNA). The methods enable high-throughput processing of samples for shotgun sequencing with broad application to basic science and clinical medicine. Specifically, the methods and compositions may be used for sequencing of small samples of bacteria. In certain embodiments, the bacteria originate from a single cell and form a cell lineage or pedigree. Thus, the microfluidic systems may be used in bacterial lineage sequencing.

Figure 13 illustrates a fully integrated microfluidic device for high quality, low cost sample preparation. The device is reusable and has low consumable costs. The device shown has 96 reactors and filters and 40 nL reaction volumes. Furthermore, the device is configured for parallel operation of reactors and filters. The current microfluidic workflow is illustrated in the flow diagram. The device workflow is also illustrated.

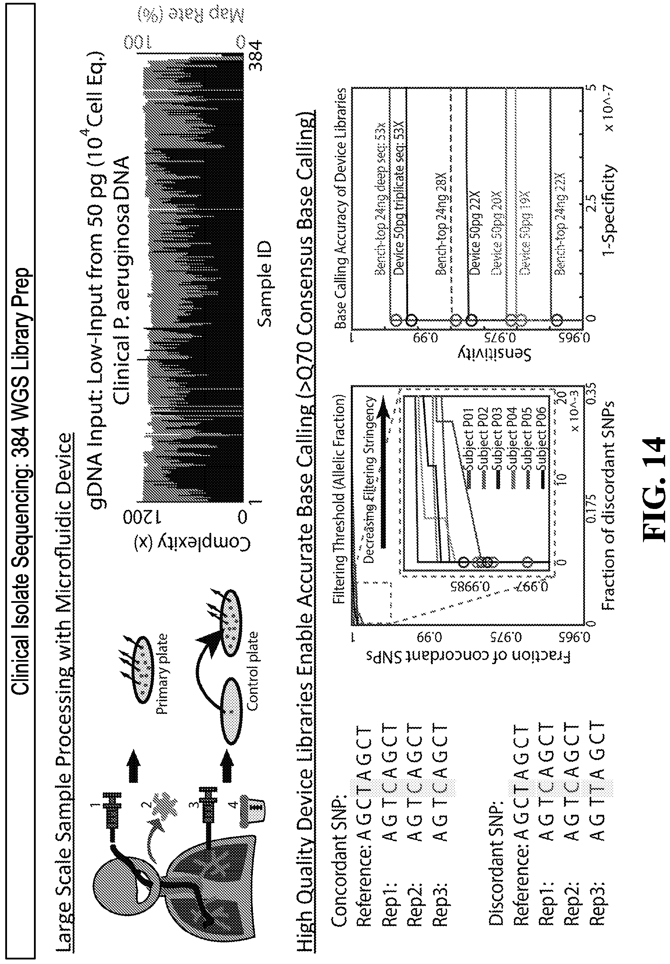

Figure 14 illustrates a comparison of the sequencing of clinical isolates using the microfluidic device. The methods and compositions may be used to analyze clinical samples obtained from various clinical specimens. Applicants analyzed 384 *P. aeruginosa* clinical samples. The genomic DNA input for each sample was 50 pg ($10^4$ Cells). The results show that each sample yielded a library with high complexity. Moreover, adjusting the filtering threshold provided a high fraction of concordant SNP's and low fraction of discordant SNP's. Compared to bench-top sequencing at different sequencing depths and different input gDNA concentrations the library preparation using the microfluidic device yielded similar sensitivity and specificity using 50 pg gDNA compared to 24 ng gDNA.

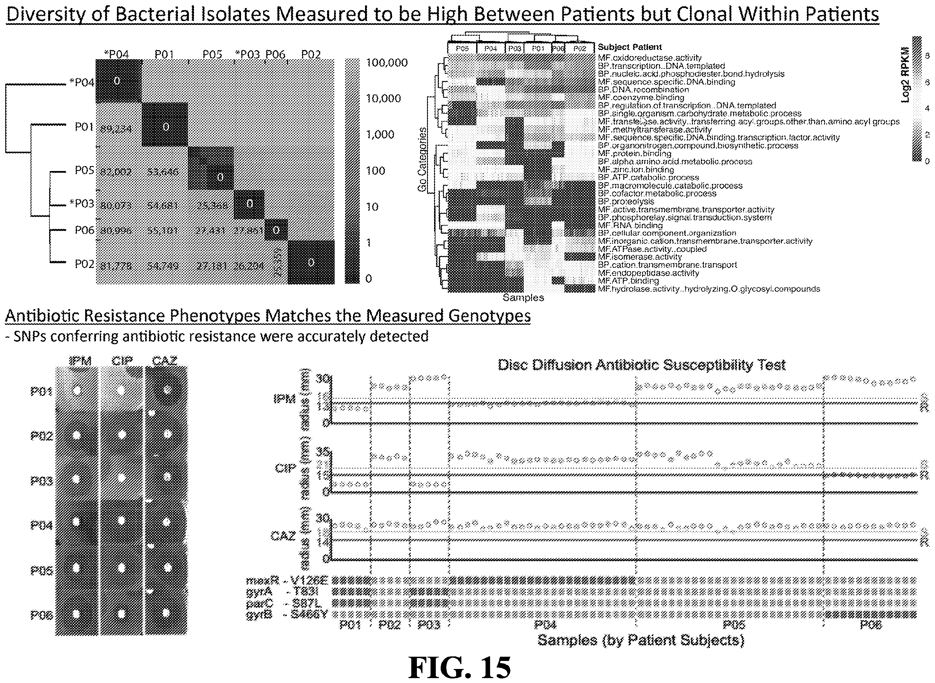

Figure 15 illustrates analysis of bacterial isolates using the microfluidic device and methods. Single bacterial isolates within patients were measured to be clonal, while diversity was high between patients. Thus, the microfluidic device may be used to compare single bacterial cells. Figure 15 also illustrates that SNP's conferring antibiotic resistance were accurately detected.

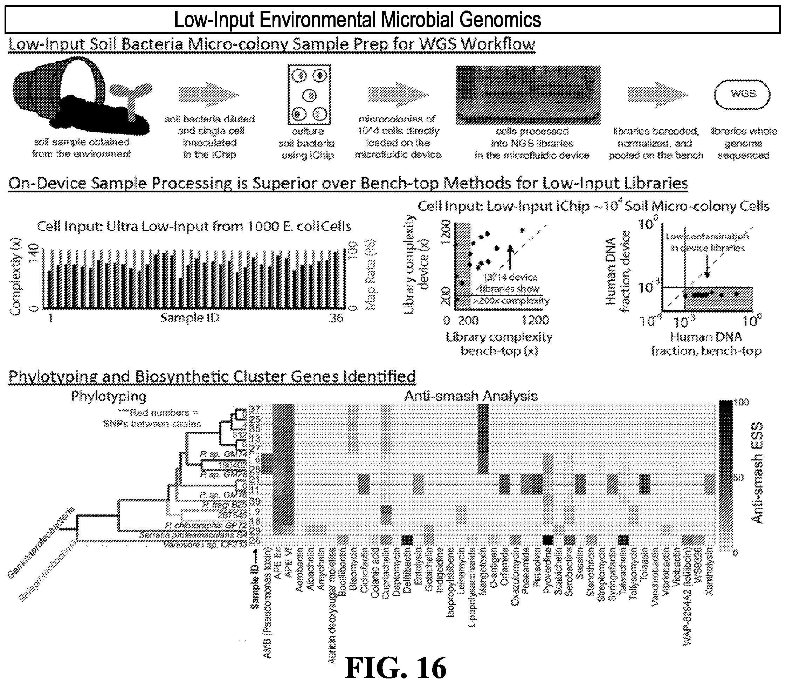

Figure 16 illustrates that low-input environmental samples may be analyzed using the microfluidic device and methods. Single bacteria were diluted to obtain single cells and the single cells were cultured using a chip to generate microcolonies (iChip). On -device sample processing was shown to be superior over bench-top methods for low-input libraries. The on-device method allowed phylotyping and clustering of biosynthetic genes identified of the soil bacteria.

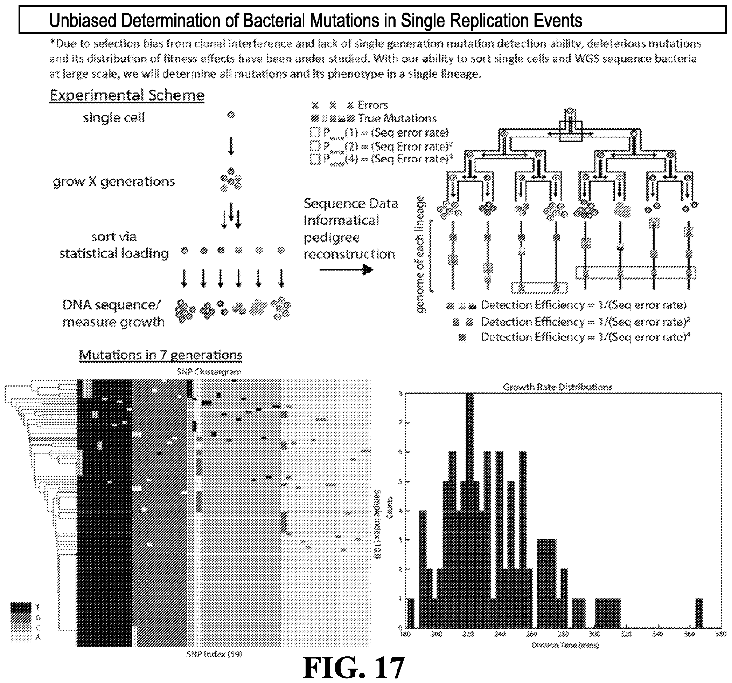

Figure 17 illustrates lineage sequencing (or pedigree sequencing) of bacteria for unbiased determination of bacterial mutations in single replication events. Due to selection bias from clonal interference and lack of single generation mutation detection ability, deleterious mutations and its distribution of fitness effects have been understudied. The present invention allows the ability to sort single cells and WGS sequence bacteria at large scale. This allows Applicants to determine all mutations and its phenotype in a single lineage. The experimental scheme shows that a single bacterial cell may be grown to allow 1 or more generations. The cells of the lineage may be sorted by statistical loading, whereby each cell of the lineage is segregated. The single cells of the lineage may be segregated on an iChip (Figure 16), microwells or microfluidic device as described herein. The cells of a lineage may be assayed for a phenotype (e.g., cell division, growth rate) and sequenced. The sequencing data may be used to generate a lineage or pedigree and mutations can be mapped precisely to cell division events across the lineage. The lineage sequencing allows for accurate detection of true mutations. Furthermore, the growth rate of the cells across the lineage can be correlated to mutations.

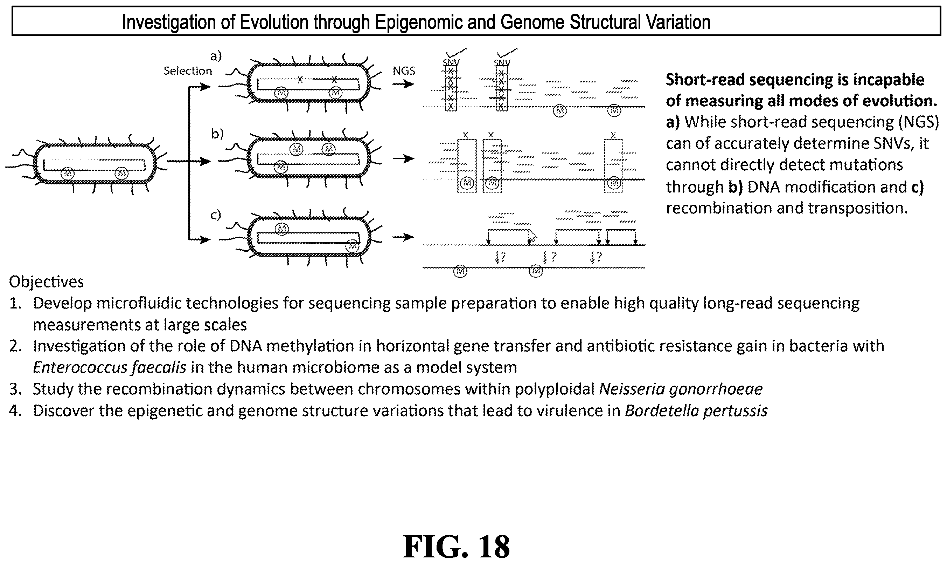

Figure 18 illustrates measuring bacterial evolution through epigenomic and genome structural variation. Short-read sequencing is incapable of measuring all modes of evolution. While short-read sequencing (NGS) can accurately determine SNV's, it cannot directly detect mutations that occur through DNA modification (e.g., methylation), recombination, and transposition. The Applicants have developed a microfluidic methodology for sequencing sample preparation to enable long-read sequencing measurements at large scales. Specific embodiments include the investigation of the role of DNA methylation in horizontal gene transfer and antibiotic resistance gain in bacteria with Enterococcus faecalis in the human microbiome as a model system, the study of recombination dynamics between chromosomes within polyploidal Neisseria gonorrhoeae, and discovery of the epigenetic and genome structure variations that lead to virulence in Bordetella pertussis.

In conclusion, using the microfluidic device, Applicants built 500 WGS libraries from clinical and environmental microbial samples. The low sample prep cost enabled Applicants to process each sample in triplicate to achieve >Q70 consensus base calling accuracy that revealed clonality between the Pseudomonas isolates within the patients, while isolates between patients were vastly diverse. Despite >100X reduction in sample and reagent input, the device☐made libraries displayed superior quality in complexity and orders of magnitude lower contamination over conventional bench-☐top methodologies. With this advantage, Applicants processed soil micro-colonies (~$10^3$ cells/colony) into WGS libraries to phylotype and predict biosynthetic gene clusters. With this platform, Applicants are processing 3000+ clinical isolates of MRSA and PSSA. Applicants are also expanding this technology for other application such as transcriptomics, metagenomics, and long-read DNA sequencing. The microfluidic system may also be used for lineage sequencing.

**Example 3 - Quantification of somatic mutations in individual cell division events by 'Lineage sequencing'**

Here Applicants further describe *lineage sequencing* (**Figure 19**) and its application to clonal cell populations cultured *in vitro.* In lineage sequencing, one collects specific cells representing different lineages, sub clones these cells, and shotgun sequences each sub-clonal population. *De novo* variants arising during the growth of the original population are shared between sub clones and identified with high sensitivity and specificity by identifying somatic variants across the sub clones that are consistent with the estimated lineage structure of the population. In regions of the lineage that are well-covered by sub clones, the origin of variants can be pinpointed to an individual cell division.

In this proof-of-concept demonstration, Applicants precisely controlled cells in the population using a microfluidic device (Kimmerling et al. 2016) and used time-lapse microscopy to provide independent knowledge of the relationships between sub clones (**Figure 24**). Initially, Applicants used the lineage structure known from the images to search for somatic variants (lineage -> called variants). Subsequently, in order to test the accuracy and sensitivity of lineage sequencing in the absence of cell tracking results, Applicants blinded themselves to the imaging data and generated a prior estimate of the lineage structure using only the sequence data (raw variants -> lineage -> called variants). This sequencing-only implementation resulted in equivalent somatic variant detection performance as the imaging-informed approach but compromises the ability to correlate observed single-cell phenotypes with mutational outcomes. By resolving the lineage at high, even single-cell, resolution, lineage sequencing is better-positioned than alternative methods to probe intra-lineage heterogeneity of mutational processes and tie mutational outcomes to specific events occurring in particular cells. For example, Applicants apply lineage sequencing to compare mutation rates across different lineage segments and to identify multiple mutations likely resulting from the same genomic lesion.

**Example 4 - Application of *Lineage sequencing* to cultured human cells**

To demonstrate the lineage sequencing concept (**Figure 19**), Applicants selected two cell lines expected to exhibit different mutation rates and spectra. The human colon carcinoma cell line HT115 possesses a missense mutation in one copy of the polymerase epsilon (POLE) gene, specifically a V411L mutation in the exonuclease domain which has been associated with hyper-mutated cancer phenotypes (Forbes et al. 2015)'(Barretina et al. 2012). POLE exonuclease ('proofreading') deficiency has been identified in a subset of human tumors and results in high mutation burdens and a unique mutational signature (Shinbrot et al. 2014; Lange et al. 2011; Albertson et al. 2009). The human retinal pigmented epithelium cell line RPE1 was immortalized by hTERT overexpression, and has not been annotated as exhibiting a mutator phenotype (Bodnar 1998). For each cell line, Applicants grew lineages to five or six generations starting from a single founding cell. This culture step was carried out in a custom microfluidic device able to track and manipulate small populations of cells arising from a single founding cell (**Figure 24**)(Kimmerling et al. 2016). The device contains an array of hydrodynamic trap structures that enable long-term growth with the entire population under continuous observation by time-lapse imaging. The device is also configured to release cells one at a time, a capability Applicants utilized to collect cells from the population and establish sub-clonal cultures representing individual lineages. Applicants extracted genomic DNA from sub clones and prepared PCR-free shotgun sequence libraries, which were sequenced to 35-fold coverage (Methods).

**Example 5 - Lineage structure information can be utilized in variant calling across multiple datasets**

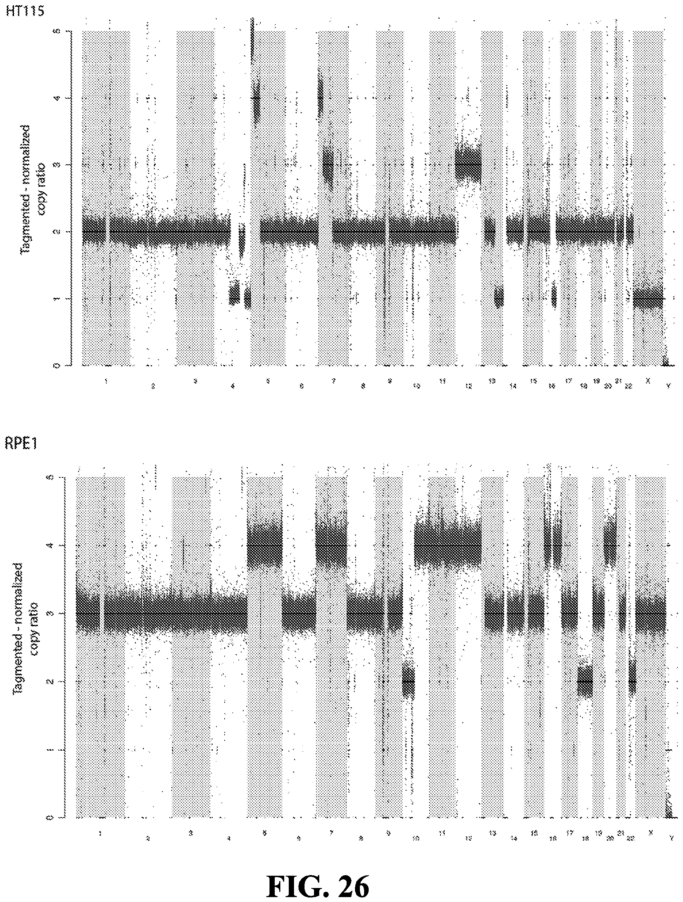

In order to produce a list of provisional SNVs for each lineage experiment, sequence data from all pairs of sub clones were analyzed with MuTect (Cibulskis et al. 2013). SNVs that arose *de novo* during the lineage experiment were identified by filtering for groups of SNVs produced by the MuTect analysis that occurred at the same locus in two or more (but not all) sub clones. Such groups of matching SNVs that coincide at the same genomic locus in multiple sub clones putatively represent *de novo* somatic mutations that occurred during generations 1-5 in the lineage experiments. Applicants term these SNVs "branch variants" (**Figure 20a-c**). By contrast, SNVs shared by all sub clones were most likely present in the founding cell. SNVs appearing in only one sub clone are termed "leaf variants", and likely represent variants that either appeared in the last round of cell division, appeared early in sub-clonal culture (or later on if strongly selected), or represent technical errors in sequencing or variant calling. Variants arising during sub-clonal culture are excluded from the branch variant call set, which only accepts variants present in at least two sub clones. Using the branch variants, which represent *de novo* somatic mutations that appeared in generations 1-5 of the lineage experiments, Applicants quantitatively reconstructed mutational events and the flow of mutations through the lineages (**Figure 20b** for HT115 and **Figure 20c** for RPE1). Branch variants are expected to appear as fully-penetrant clonal variants in the affected sub-clonal populations because they occur before the sub cloning step. In HT115, such coincident SNV sets constituting branch variants were enriched at allele fractions close to 0.5, as expected for clonal mutations in a predominantly diploid genome **(Figure 20d;** corresponding RPE1 allelic fraction results are shown in **Figure 25**). The allele fraction distribution of clonal branch variants is concordant with the copy number variation analysis for both cell lines **(Figure 26**).

By contrast, non-coincident SNVs representing true variants arising within or after the last (sixth) generation of the HT115 lineage - the leaf variants - had to be identified within individual samples without the extra statistical power lent by joint variant calling across multiple samples. The leaf variants showed an allele fraction distribution distinct from the branch variants with most values lower than 0.5 and ranging down to uncertain instances of candidate variants with low allele fraction that are filtered out by MuTect **(Figure 20d)**.

The knowledge that branch variants are clonal is valuable in variant detection. For example, Applicants can easily segment mutations by ploidy of the genomic locus using the read depth in the standard-depth PCR-free data since variant alleles are fully represented. Leaf variants in the data include sub-clonal variants, and their detection is fraught by challenging tradeoffs in read depth and variant allele fraction cutoffs (**Figure 20d** and for RPE1 figure 26b). The ability to apply relaxed thresholds in calling branch variants with a low chance of false-positive detections makes lineage sequencing more sensitive and quantitative than other approaches, which suffer from significant allele dropout when controlling false-positive detections.

**Example 6 - Lineage sequencing recapitulates mutational characteristics of POLE proofreading deficiency**

Mutations are driven by the chemistry of nucleotides, DNA, and the biology of their metabolism, including synthesis and repair. Each mutagenic pathway has particular characteristics and results in a distinct mutational spectrum (Alexandrov et al. 2013a, 2013b). As a result, observed mutational spectra provide clues about the mutational processes operating in cells. The spectrum of mutations caused by POLE proofreading deficiency has been previously characterized (Shinbrot et al. 2014). Applicants compared the mutational spectrum of the aggregated HT115 branch variant SNV call set with mutational spectra derived from tumor-normal whole genome sequencing of group A POLE colon tumors (Shinbrot et al. 2014) generated by the TCGA Research Network and found high similarity (**Figure 21a**). The highest similarity was found with a previously described colorectal tumor sample annotated with stop codon (R1371*) and a missense mutation L1235I in the POLE gene (cosine similarity = 0.97 +/-0.01) (**Figure 21b** and **Figure 38**). The RPE1 spectrum differs markedly from the HT115 spectrum (cosine similarity = 0.67+/- 0.015) and the POLE mutant clinical tumor spectra (**Figure 21a** and COSMIC mutational signature analysis **Figure 27**).

Activity of the DNA mismatch repair (MMR) pathway is known to be coordinated with DNA replication and to be most active during S phase, particularly in euchromatic early-replicating regions (Supek and Lehner 2015; Kunkel and Erie 2015). Using high-resolution genomic replication timing data, Applicants compared the frequency of SNVs and replication timing across the genome. SNVs were markedly suppressed in early-replicating regions relative to late-replicating regions (HT115 in **Figure 21c**,d, and RPE1 in **Figure 28**). Applicants also observed significantly fewer SNVs in genic regions than in intergenic regions and an even higher skew against variants in exons (**Figure 21d** and **Figure 28**)(Frigola et al. 2017; Dulak et al. 2013; Li et al. 2015). These effects were observed in both the branch and leaf variant sets and are likely attributable to differential epigenomic status and repair efficacy across different classes of genomic loci (Frigola et al. 2017; Schwartz et al. 2009; Li et al. 2013; Huff et al. 2010; Smith et al. 2017). Such local variation in mutation rates across the genome underscores the need for mutation rates to be assessed on a genome-wide basis, as targeted approaches are likely to be biased by sampling any particular subset of genomic loci.

POLE mutations are also associated with a particular type of strand asymmetry called replication-class (R-class) asymmetry (Haradhvala et al. 2016), which arises due to POLE's specific role in synthesis and proofreading of the leading strand during DNA replication and the stereotyped locations of replication origins in much of the human genome. POLE-driven mutations appear in these regions in a polarized fashion with respect to the two DNA strands. For example, Applicants expect a high proportion of C>A relative to G>T in the DNA strand being synthesized as the leading strand (C>A in left replicating regions and G>T in right replicating regions relative to the genomic reference). Indeed, Applicants observed the predicted POLE R-class asymmetry among HT115 branch variant SNVs at the same level previously quantified in TCGA POLE mutant samples (Haradhvala et al. 2016) (**Figure 29**).

**Example 7** - **Lineage sequencing is accurate and sensitive**

Applicants tested whether lineage structures could be estimated from the genomic data alone by blinding themselves to the time-lapse imaging data from the HT115 and RPE1 experiments. Applicants first filtered the raw SNV calls from MuTect to identify coincident SNV calls (**Figure 22a**). Applicants then counted the number of coincident SNVs for each set of sub clones in which coincident SNVs occurred, and plotted the ranked counts for the HT115 and the RPE1 experiments (**Figure 22b**). A small number of sub clone sets contained high counts of coincident SNVs (HT115: >100; RPE1: >10), while other sets contained very low counts (HT115: <10; RPE1: <5). The groups of sub clones with high frequencies of coincident SNVs comprised a consistent set of relationships that predicted a single lineage structure for each experiment (**Figure 22c,** dendrograms plotted based on total set of high quality coincident SNVs). Using these lineage structure estimates, Applicants then recovered nearly the same sets of branch variant calls identified earlier using the time-lapse imaging data to track cells (SNV recovery versus the time-lapse imaging-informed approach: HT115, 99.4%; RPE1, 97.1%).

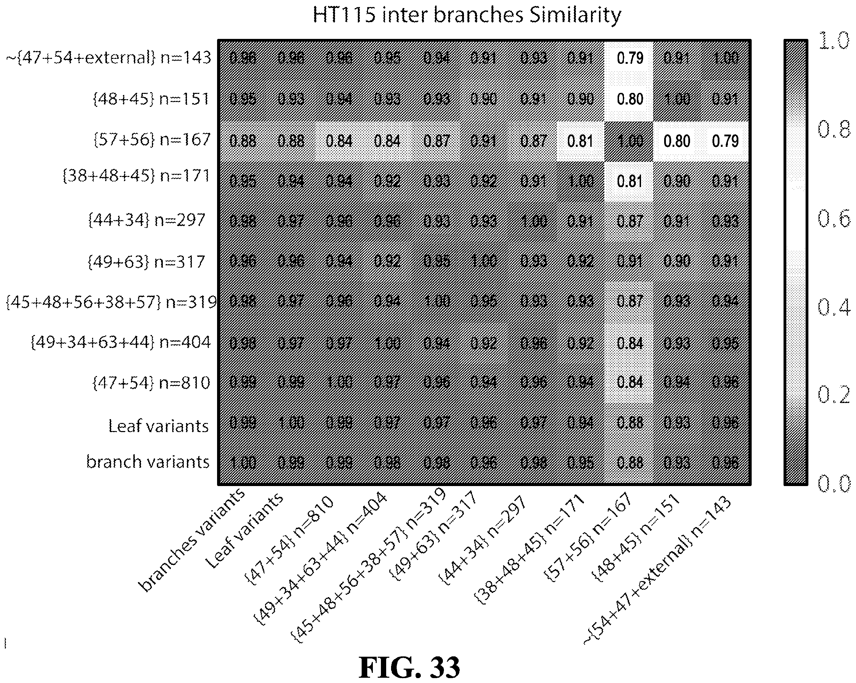

Lineage sequencing also allows data quality to be tested by quantifying variants that do not agree with the consensus lineage structure. Applicants estimated the specificity and sensitivity of the branch SNV calls as a function of the quality threshold for coincident variant calls, similar to the construction of a receiver operator characteristic (ROC) (**Figure 30**). For HT115 branch variant SNVs, sensitivity and specificity were estimated to be 96.4% and 99.9% (HT115) and 91.9% and 99.9% (RPE1) based on the selected quality threshold of 0.99 applied to filter coincident SNVs when branch variants are called (**Figure 31**). The mutational spectra of the SNVs that do not agree with the consensus lineage structure differ from the spectrum for the lineage structure-concordant set for each cell line, consistent with the hypothesis that SNVs in disagreement with the called lineage structure represent errors that were correctly filtered out by the data analysis procedure (**Figure 32**). In contrast, mutational spectra of branch variant SNVs show strong similarity across sub-lineages, indicating the high specificity of these variant calls (**Figure 33**).

**Example 8** - **Lineage sequencing enables mutation rate determination with certainty in the generation number**

Applicants constructed a null statistical model for mutation accrual based on the assumptions that each somatic mutation event was independent of the others and that the number of mutations appearing in each daughter cell followed the same (Poisson) distribution. Applicants estimated the average mutation rate for HT115 cells as 173 SNVs per cell division with the 95% confidence interval (CI) [147,203] (using the model), which corresponds to a rate of $3.0 *10^{-8}$, 95% CI [$2.5*10^{-8}$, $3.5*10^{-8}$] SNV per bp per cell division (SNV/bp/division). The error in this estimate is driven principally by the variant counting statistics since Applicants have precise knowledge of the number generations over which mutations accrued in each lineage segment. At haploid loci, leaf variant data can also be used to independently estimate the mutation rate for SNVs at haploid loci with allele fraction of one. These SNVs must occur prior to sub clone expansion to appear clonal, elsewise the SNV would be diluted in the population. Applicants calculated the mutation rate for branch variants, $2.9*10^{-8}$ CI [$1.3*10^{-8}$, $5.2*10^{-8}$] SNV/bp/division, and leaf variants, $4.2*10^{-8}$ CI [$2.2*10^{-8}$, $6.5*10^{-8}$] SNV/bp/division, at HT115 haploid sites, and found rates similar to those calculated for diploid/triploid sites, suggesting that homology-directed repair makes a minor impact in the HT115 experiment (**Figure 34**). Consensus estimates of spontaneous mutation rates during nuclear DNA replication in normal cells are close to $5*10^{-10}$ SNV/bp/division (Blokzijl et al. 2016), indicating that the mutation rate Applicants measured in HT115 cells is about two orders of magnitude higher than expected in normal cells and similar to previous estimates of the rate in cells with compromised POLE proofreading activity (Shinbrot et al. 2014). In contrast to the rapidly mutating HT115 cells, RPE1 cells showed an average mutation rate of 39 CI [27,52] SNVs per cell division, or $4.3*10^{-9}$ CI [$2.9*10^{-9}$ $5.7*10^{-9}$] SNV/bp/division, seven times lower than observed in HT115 cells. With time-to-division data available from the time-lapse imaging, Applicants could analyze the dependence between mutation accumulation and generation time (**Figure 35**) but found no evidence for accrual of mutations during interphase. This could be explained by limited statistical power in analysis of the small datasets, by the dominance of POLE substitution errors at replication, or compensation of accruing mutations by greater repair in slow-cycling cells.

## Example 9 - The mutation rate is heterogeneous across sub-lineages

Applicants next tested the assumption that mutations accrue according to a Poisson process, or equivalently, that the cells in the lineage experiment growing under similar conditions at the same time, would exhibit the same average mutation rate. Applicants produced quantile-quantile (QQ) plots of p-value quantiles to compare the observed distribution of branch variant SNV counts across each lineage segment with the theoretical Poisson process model based on a constant rate of mutation. The QQ plots show poor concordance of the experimental data with the theoretical Poisson process model (**Figure 23a**). This result indicates that mutations accrued across lineages in HT115 and RPE1 experiments by a more heterogeneous process than the Poisson model and that new models for mutation accrual with additional parameters to account for variable mutation rates are likely justified when analyzing mutational processes and interpreting mutational data.

## Example 10 - Persistent lesions cause mutations in multiple cells

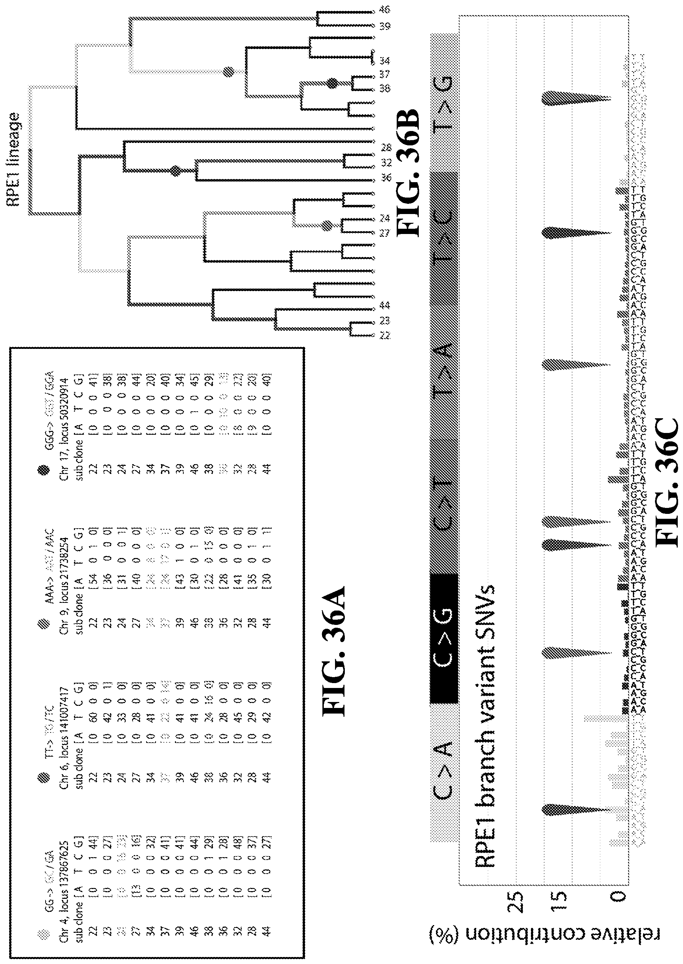

Applicants observed eleven instances where two SNVs occurred at identical genomic positions in related sub-lineages (HT115: 7 instances, **Figure 23b;** RPE1: 4 instances, **Figure 36**). All of these instances were consistent with the lineage structures. In the dataset, it is extremely unlikely that multiple variants coincide (for HT115 $P<2.05 \times 10^{-20}$ and for RPE1 $P<2.8*10^{-16}$, considering uniform probably along the genome), and this observation further challenges models such as the Poisson process that assume independent mutations. Applicants hypothesize that each of these events results from a DNA lesion that persists through multiple rounds of DNA replication and cause multiple, different, substitution errors through translesion DNA synthesis opposite the same DNA lesion (Sale et al. 2012; Makridakis and Reichardt 2012; Sale 2013; Bi 2015; Ziv et al. 2014). Lesion persistence could result from common DNA lesions that escape repair by chance, other types of lesions that are repaired only slowly by cells, or damage-tolerant cell states (Geacintov and Broyde 2017). These events can be effectively identified only when somatic variants are detected with high sensitivity and single cell cycle resolution. The multiple mutation events also seem to show a different mutational spectrum than the complete branch variant sets, lending additional support for the idea that the multiple mutation phenomenon results from one or more unique molecular sub processes. Linking multiple mutations to lesion persistence exemplifies how detailed analysis of high-quality variant data can be used to refine mutational signatures and parse mutation signatures for distinct biochemical processes.

## Example 11 - Discussion

Here Applicants describe lineage sequencing, a new genome-wide technique that utilizes knowledge of the cell lineage structure to reconstruct mutational events occurring during lineage formation with high resolution, high accuracy, high precision, and minimal bias. Lineage sequencing is based on standard short-read next generation sequencing methods applied at standard shotgun sequencing coverage depth for each sequence library and utilizes joint variant calling across the set of sequence libraries informed by an estimate of the lineage structure to enhance statistical power. The proof of concept implementation achieves partial resolution of individual cell cycles with neither whole-genome amplification nor polymerase chain reaction amplification steps, and as a result, produces sequence libraries with highly uniform and accurate coverage of the genome. By applying lineage sequencing, Applicants measure the accumulation of SNVs by identifying >90% of SNVs that evolved during lineage formation and mapping these onto cells in the lineage with very high resolution (1 - 4 cell divisions, with little uncertainty in the number of cell divisions in each lineage segment where SNVs were assigned). Altogether, the method provides accurate and nearly complete estimates of cellular genotypes within the lineage, suggesting that lineage sequencing may emerge as a gold standard for somatic mutation quantification.

Applicants were able to distinguish two types of SNV call sets: high-confidence branch variants that occur during lineage formation and leaf variants which occur in the last round of cell divisions or during sub-clonal culture. Leaf variants can be compared to variants identified by the existing "double bottleneck" methodology (Szikriszt et al. 2016; Blokzijl et al. 2016; Drost et al. 2017). Leaf variants cannot be assigned to a specific cell division in the lineage with high confidence and suffer from low specificity, preventing precise quantification for mutation rate assessment.

By contrast, the branch variant analysis shows improved performance over alternative methods by providing a nearly complete and almost perfectly accurate list of somatic variants that accumulate within a specific number of cell divisions thanks to the support of each branch variant by multiple sequencing libraries. The use of the prior estimate of inter-sample relationships here is analogous to the use of known family relationships in human germline trio and quartet sequencing (Roach et al., Science. 2010 April 30; 328(5978): 636-639). In addition to the internal consistency of the branch variant analysis (**Figure 33**), these results are validated independently by agreement with the lineage structure established by time-lapse microscopy and the consistency of the aggregated *de novo* somatic variant sets with the known mutational spectra, the expected impact of replication timing on mutation, and observed R-class asymmetry in POLE mutant samples. Applicants also gained key insights into the short-term mutational dynamics of human cells including indications that the measured mutation counts were over-dispersed relative to a Poisson process, which showed that the mutation rate varied across the lineage on top of the mutation rate variability observed across different genomic loci. Applicants found strong similarity of HT115 SNV spectrum with tumor samples sharing POLE deficiency, and highest similarity to the COSMIC POLE signature based on whole exome analysis (**Figure 27**). These observations highlight the need for quantitative genome-wide signature assessment to take full advantage of somatic mutations to discriminate mechanisms of mutagenesis.

While the lineage structures Applicants derived from sequence data alone enabled recovery of the same set of relationships among the sub clones as observed in the time-lapse microscopy, they lack independent information about number of cell divisions and duration of each cell cycle in the lineage that was available from the imaging data. In principle, the observed time-to-replication for each cell in the imaging data can be used to determine whether the number of cell cycles, or alternatively, the elapsed time, is a better predictor of the number of somatic mutations, and further, to constrain likely mechanisms of mutagenesis in these cells. The sample size provided insufficient power for this task, although the analysis illustrates the framework for cell-specific phenotype-mutation set correlative analysis (**Figure 35**). Where observations or perturbations are made of the cells during the development of the lineage, these can be directly related to genomic variants that appeared at the same times in the lineage. Targeting specific lineages in which intracellular events of interest were observed would enrich the variant set for mutations linked to these events (**Figure 37**). Much can be learned by studying real-time cellular phenotype data with linked genomic data from lineage sequencing, for example, the effects of different exogenous insults to cells, checkpoint activation, DNA repair capacity or stochastic dynamics, cell division defects, and more. Another feature of the approach is that the sub-clonal cultures can be grown to different population sizes and sampled at different times. This flexibility means that multiple genotyping methods with differing biomass requirements can be deployed optimally on the same sub clone, and that live cultures representing sub-lineages will typically be available for further experimentation.

Here, Applicants showed two different ways that information about the lineage structure can be obtained prior to joint variant calling to support lineage sequencing: 1) by time-lapse microscopy, and 2) using raw SNVs from whole genome sequence data (provided at least one detectable variant per cell to achieve full resolution of the lineage). In principle, other approaches for tracking/estimating lineage structures could also support lineage sequencing (Woodworth et al. 2017). For example, recently reported self-editing genomic barcoding approaches (Frieda et al. 2017)'(McKenna et al. 2016). With appropriate methods for cell sampling and sub-clonal culture, Applicants expect that the lineage sequencing concept can be applied to study mutations that occur in solid tissues or whole organisms.

Lineage sequencing facilitates in-line data quality evaluation by enabling candidate variants that do not agree with the consensus lineage structure to be quantified. Data quality assessments can in turn be used to optimize the biochemical and computational protocols for analysis of genomic aberrations occurring during lineage formation. Technical errors are introduced independently across sub clones and are uncorrelated, resulting in dramatically lower false positive error rates in the branch variants calls where uncorrelated errors are excluded. The branch variant error probabilities are expected to fall by the square (or higher power for variants supported by data from more than two sub clones) of the nominal consensus error rate, *e.g.* $(10^{-6})^2 = 10^{-12}$. The extra statistical power lent by joint variant calling in lineage sequencing could conceivably ensure accurate and more sensitive mutation detection for direct single-cell readout that depends on noisy whole-genome amplification. In addition, sampling strategies for lineage sequencing can be tailored to optimize statistical power for different estimation tasks: for example, more sparse sampling of cells from the lineage could improve mutation rate determination at constant sequencing effort for cells with few mutations per generation.

These results show that mutations do not occur independently with uniform probability, but rather are heterogeneous across the genome and across closely related cells, even when environmental conditions are uniform. Applicants observed that mutations are not independent, rather that different mutations can occur at some genomic locus in different cells. Lesion persistence probably explains this and serves as an example of one particular mechanism that causes strong correlations among somatic mutations.

In summary, lineage sequencing allows precise assessment of the rate and spectrum of somatic mutations with intra-lineage resolution as high as individual cell division events. Lineage sequencing can be combined with real-time phenotypic observation and/or perturbation to link cellular activity and responses with mutational events at the single-cell level. This detailed level of analysis enables the detection of individual biochemical events in cells and the parsing of mutational spectra with enhanced resolution. Applicants imagine that lineage sequencing could be applied widely to study spontaneous and exposure- or therapy-associated mutational processes in remarkable detail and to help identify the molecular mechanisms and genomic consequences of many mutation types.

**Example 12** - **Lineage sequencing 2.0**

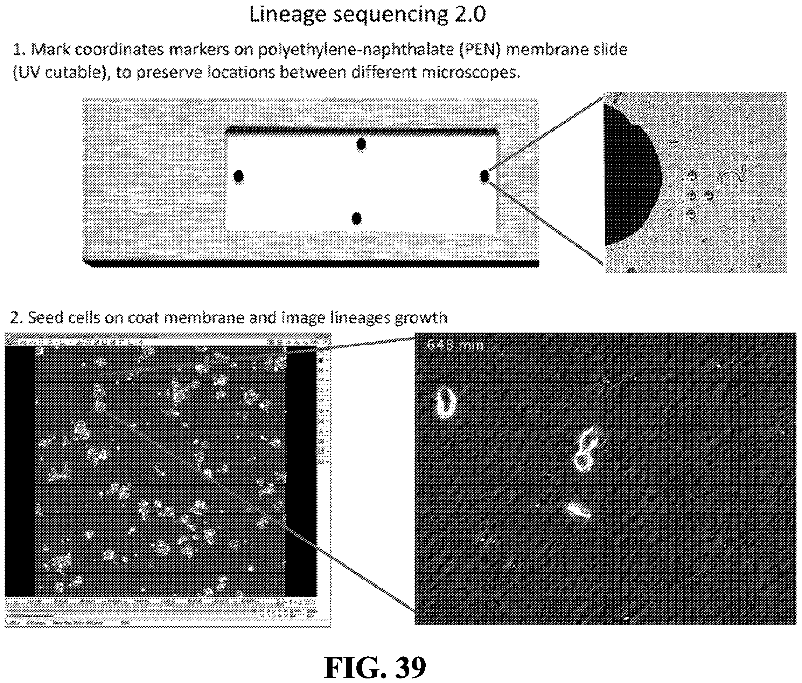

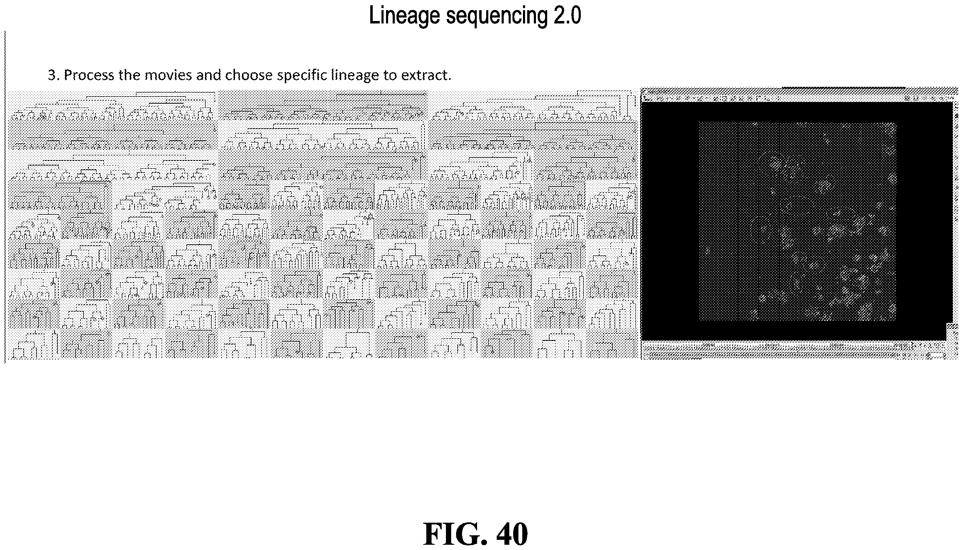

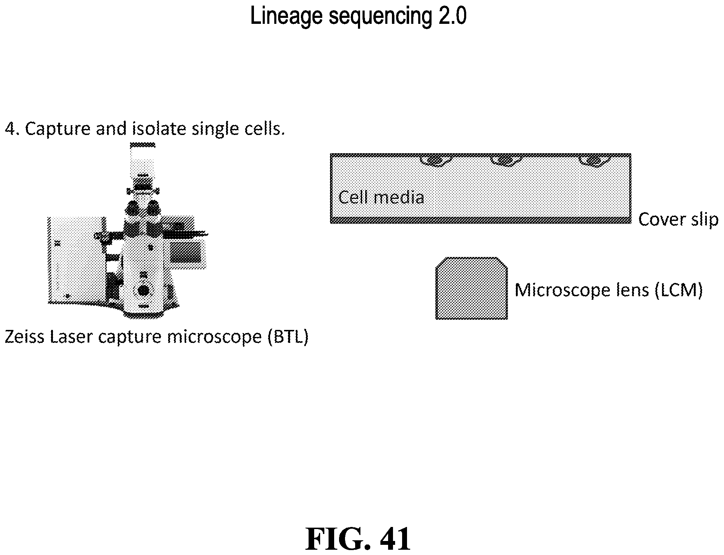

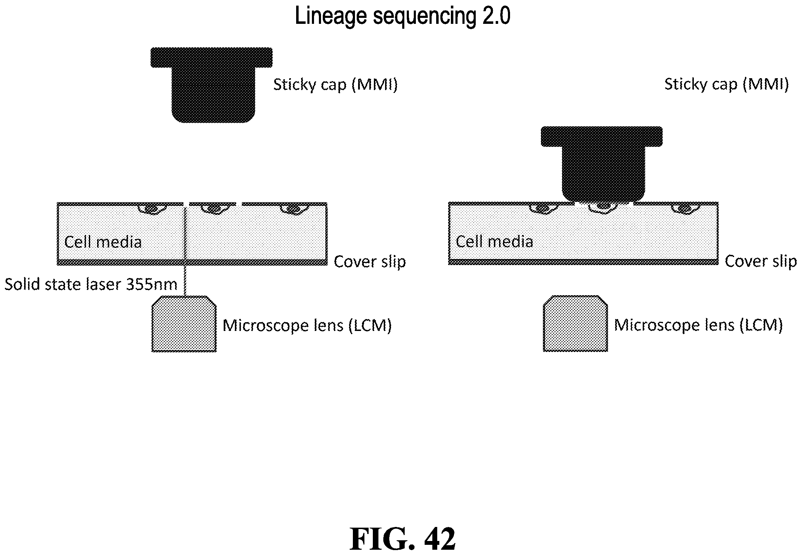

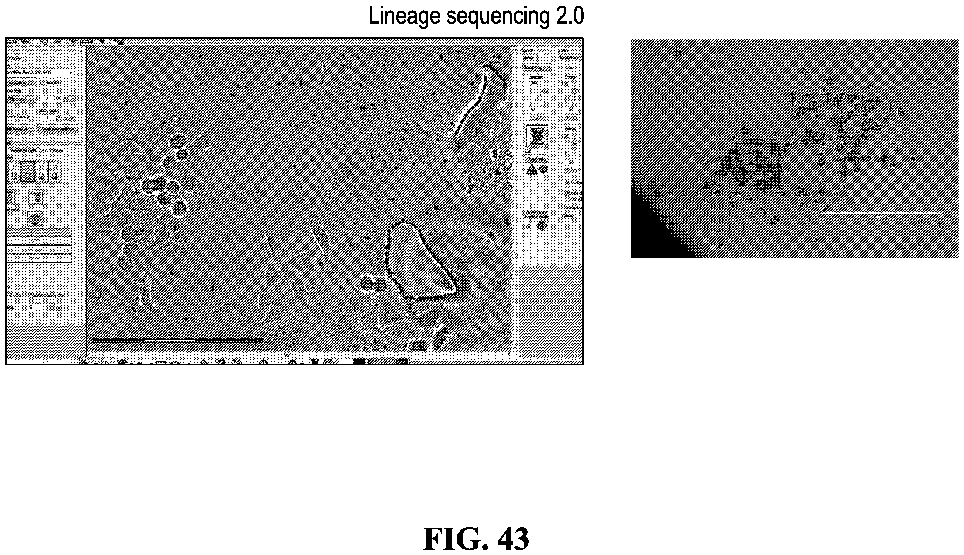

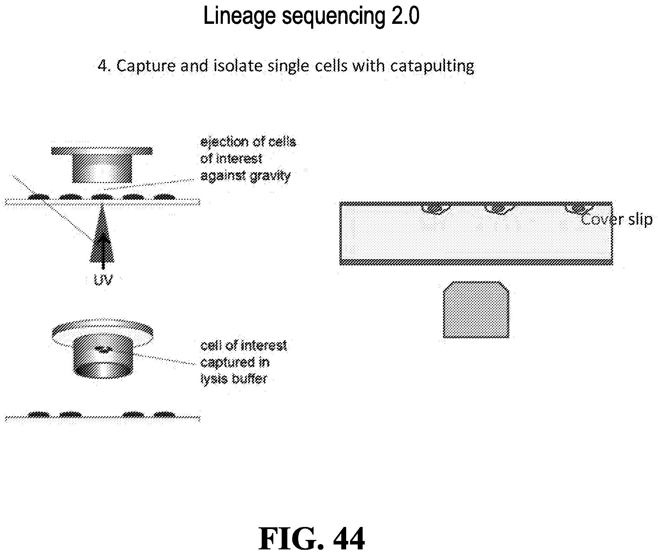

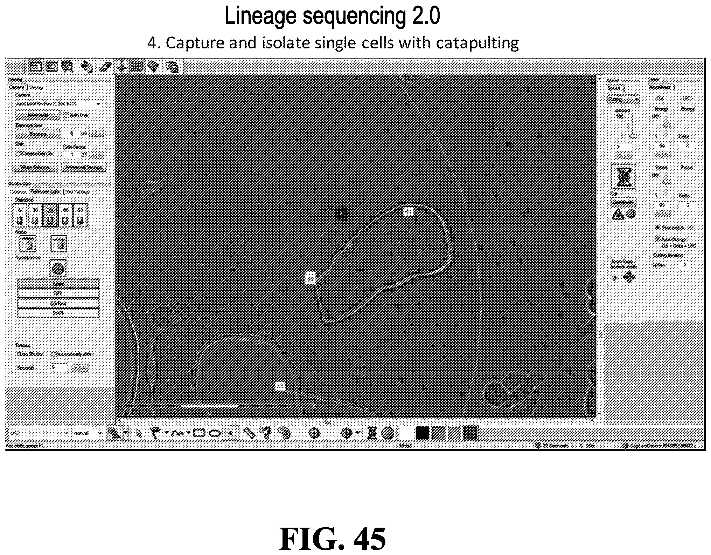

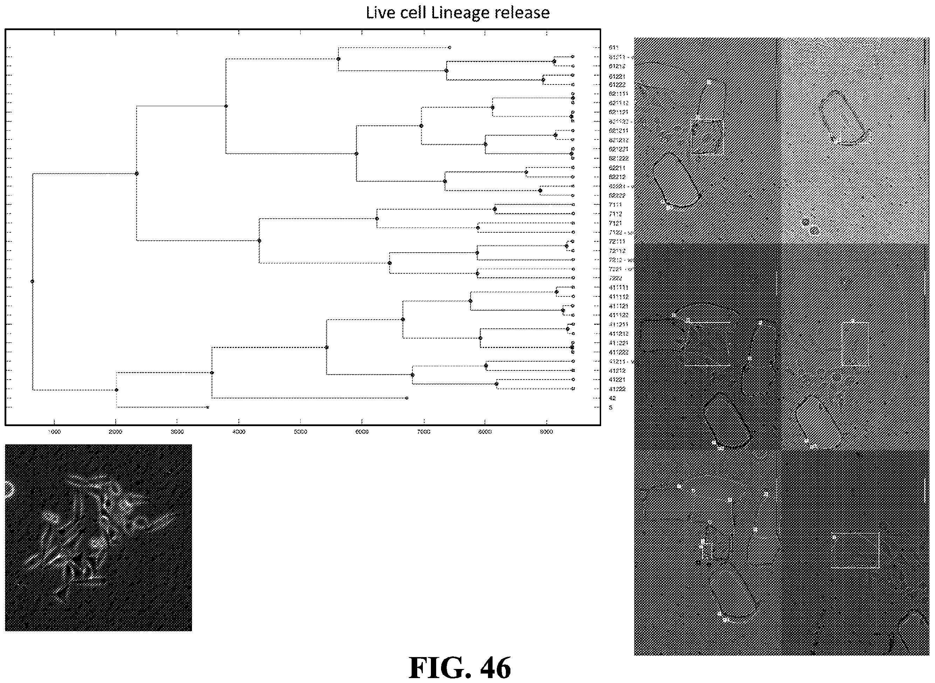

Use of the microfluidic devices described herein for lineage sequences represent only single embodiments for practicing the invention. Lineage sequencing can also be performed using live cell imaging and laser capture methods (**Figures 39 to 47**). In certain embodiments, live imaging is used to construct movies of cells grown on slides. The slides are configured such that cells can be grown and the surface can be cut with a UV light (e.g., laser) (**Figure 39**). In certain embodiments, the slide is marked, such that the slide orientation and location of cells can be determined when transferring slides from one microscope to the next (**Figure 39** top panel). The movies allow specific lineages to be chosen (**Figure 39** bottom panel). In certain embodiments, a live cell imaging microscope is used to capture the movies. Once, specific movies are chosen, lineages can be extracted for processing (e.g., sub cloning cells and shotgun sequencing each sub-clonal population) (**Figure 40**). In certain embodiments, single cells are captured and isolated using a laser capture microscope. Advantages of this method are that it prevents the growth bias cause by single cell loading, it is easier to perform, it is more robust for adherent cells, and it enables visualization of whole mutagenesis affects. In certain exemplary embodiments, steps for lineage sequencing may include: 1. Mark coordinates markers on polyethylene-naphthalate (PEN) membrane slide (able to be cut by UV), to preserve locations between different microscopes; 2. Seed cells on coat membrane and image lineages growth; 3. Process the movies and choose specific lineage to extract; and 4. Capture and isolate single cells. Capturing may be performed using a Laser Microdissection system (see, e.g., Vandewoestyne et al., 2013) (**Figures 41, 42 and 43**). In certain embodiments, a laser is used to cut out cells from the coverslip and the cell captured using the sticky cap of an Eppendorf tube (**Figure 42**). Figure 43 shows an image of selected cells cut out (left) and a zoomed-out image showing the coverslip with many cells cut out (right). Capturing may also be performed using catapulting (**Figures 44 and 45**)(see, e.g., Horneffer et al., 2007; and Vogel et al., 2007). Live cells captured using the live cell imaging and microdissection can be used to perform lineage sequencing as described herein **(Figure 46**).

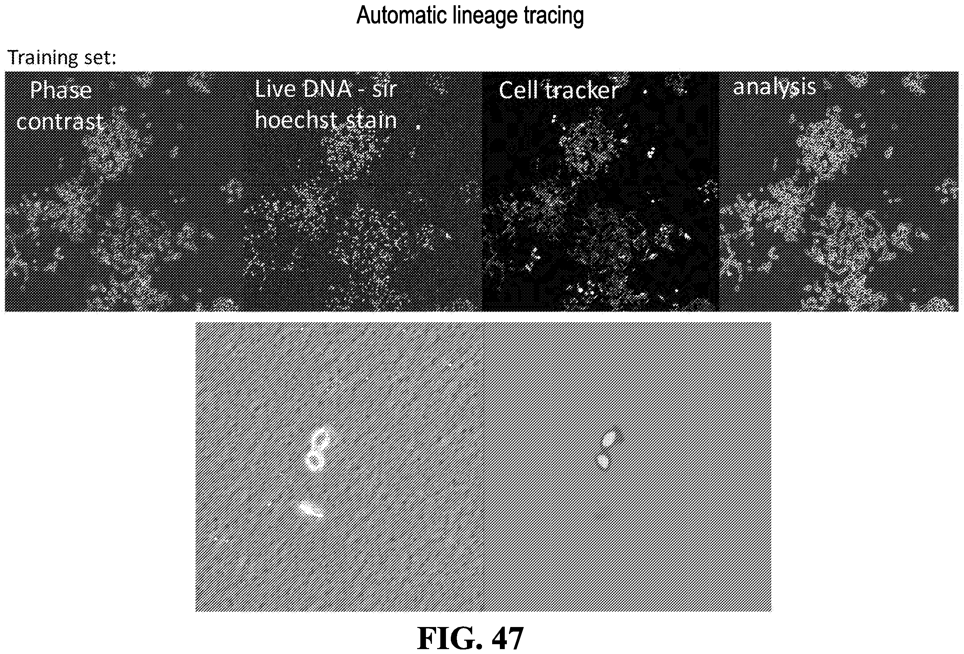

In certain embodiments, the lineage tracing is automated. Applicants can train the computer to select cells that are part of a lineage with training sets and live cell imaging (**Figure 47**). Applicants use a computer to select dividing cells and capture them for downstream processing. In certain embodiments, the method uses a network and training strategy that relies on the strong use of data augmentation to use the available annotated samples more efficiently (see, e.g., Ronneberger et al., 2015).

**Example 13** - **Further Applications of Lineage Sequencing**

In certain embodiments, lineage sequencing may be used for genotoxicity testing or in exposure models. In certain embodiments, primary cells are exposed to a toxin. The primary cells may be small lung airway epithelial cells. The toxin may be cigarette extract. Cell lines may include, but are not limited to human lung cell lines (e.g., A549 and BEAS-2B), small airway primary cells (e.g., for purchase from Lonza), or immortalized small airway epithelial cells (e.g., HSAEC1-KT). In certain embodiments, the toxin is a chemotherapy (e.g., cisplatin). In certain embodiments, lineage sequencing is used to quantify the genotoxicity damage from exposure to a drug or toxin and identify repair resistant DNA lesions and tolerance pathways.

In certain embodiments, an epigenetic mutational rate is measured. The present invention can be used to calculate lineages with single-cell epigenomics to enable charting of tumor evolution with therapy. In certain embodiments, cell lineages are isolated and DNA methylation patterns across the lineage are determined. DNA methylation may be determined using bisulfite sequencing methods. Bisulfite sequencing may be performed on single cells isolated from a lineage.

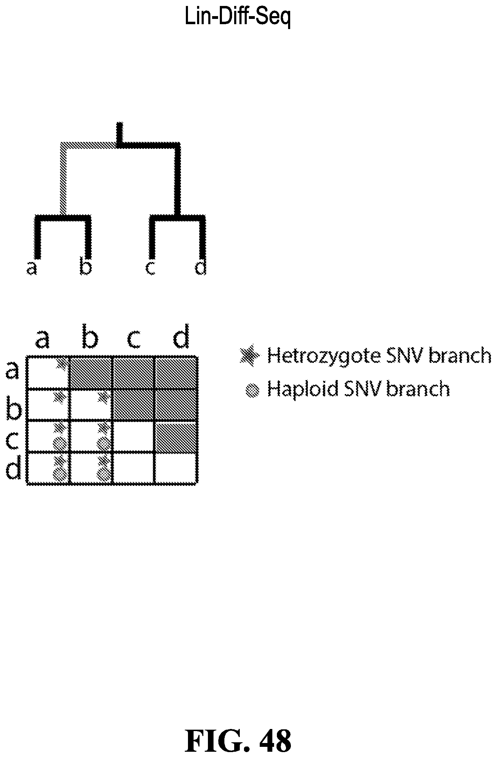

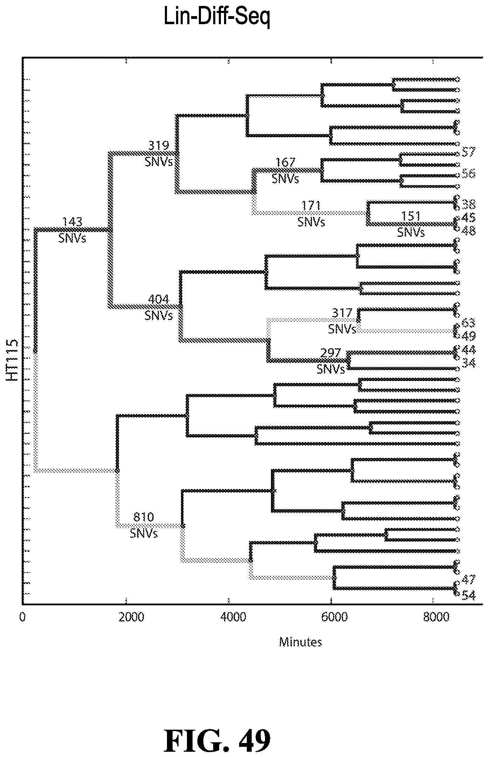

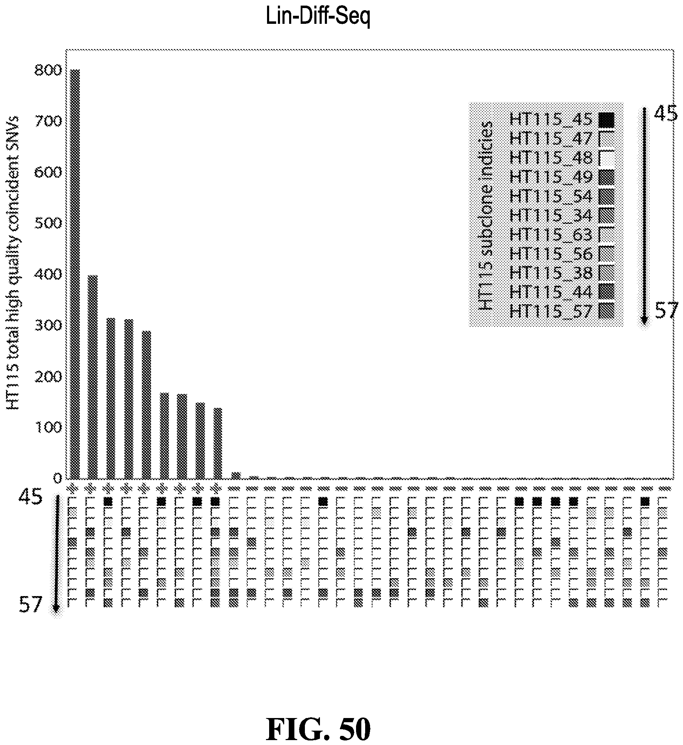

In certain embodiments, lineage sequencing can be combined with Diff-seq (Lin-Diff-seq) (see e.g., Aggeli et al., Diff-seq: A high throughput sequencing-based mismatch detection assay for DNA variant enrichment and discovery, Nucleic Acids Res. 2018 Apr 20; 46(7): e42). Diff-seq leverages the Surveyor endonuclease to cleave mismatched DNA molecules that are generated after cross-annealing of a complex pool of DNA fragments. Sequencing libraries enriched for Surveyor-cleaved molecules result in increased coverage at the variant sites. In certain embodiments, Diff-seq is applied on DNA molecules obtained from captured lineages. Applicants applied Lin-Diff-seq on HT115 cells (**Figures 48, 49 and 50**).

In certain embodiments, lineage sequencing is used to determine conditions that decrease the mutation rate (e.g., slower division rate) (see, e.g., Tomasetti and Vogelstein, Variation in cancer risk among tissues can be explained by the number of stem cell divisions, Science. 2015 Jan 2; 347(6217): 78-81). In certain embodiments, lineage sequencing is used for functional genomics, such that specific phenotypes can be detected and associated with somatic mutation. For example, a branch variant or leaf variant may occur that is associated with growth rate or drug resistance. In certain embodiments, a phenotype may be detected in a movie generated by live cell imaging. In certain embodiments, lineage sequencing can be used to screen for agents or conditions that decrease the mutation rate in a population of cells. In certain embodiments, lineage sequencing can be used to screen for conditions that increase the mutation rate in a population of cells (e.g., ageing). In certain embodiments, lineage sequencing can be used to compare the mutation rate between different cells.

## Example 14 - Materials and Methods

**Cell Culture and conditioned media preparation.** Human HT115 Epithelial colon carcinoma cells from the Cancer Cell Line Encyclopedia (CCLE), (Barretina et al. 2012)), were maintained in High Glucose Dulbecco's modified Eagle's medium (DMEM, Life Technologies, Inc.) supplemented with 15% fetal bovine serum (Mediatech #35-015-CV). The telomerase-immortalized RPE1 cells (ATCC) were maintained in DMEM/F12 medium (ThermoFisher) with 10% fetal bovine serum. Conditioned media for use with the microfluidic device was collected from the stock cell flask 24 hours and no longer than 48 hours after fresh medium was added. The medium was centrifuged (4700g for 5 min) and filtered (0.2um) before use.

**Microfluidic Device.** Hydrodynamic trap array devices were fabricated in silicon and glass as described previously (Kimmerling et al. 2016). Prior to cell culture in the device, the system was flushed with 10% bleach for ten minutes for sterilization and cleaning, rinsed with water, and then flushed with conditioned cell culture media overnight to fully rinse and prime the system for cell culture. Finally, devices were flushed with a 0.1% poly-L-lysine solution (Sigma) for ten minutes to coat the channel surfaces and promote cell adhesion and growth, followed by a short wash of a few seconds with conditioned cell culture media just before cell loading.

**Single-cell culture in the hydrodynamic trap array.** A single-cell suspension was introduced at the downstream port of the system to load cells into the device (port P3 in Supplemental Figure S1). In order to introduce cells into the device, the pressures P2 and P3 were increased equivalently (P2 = P3) and the pressure P1 was decreased (P2 > P1) such that there was equal flow from the downstream (P2, P3) to upstream (P1) ends of the bypass channels on either side of the hydrodynamic trap array. Once the dead volume of the system was purged and cells were within view in the device, single cells were manually loaded into each lane of the trap array. This cell loading was achieved by decreasing the pressure P2 relative to P3 (P2 < P3) to introduce reversed flow in the trap array (Figure S1). With this sustained flow into the trap array, the upstream pressure (P1) was periodically toggled between a higher pressure than P3 (P1 > P3) and atmospheric pressure (P1 < P3) to flow cells upstream and downstream near the entrance of the trap array to allow them to slowly drift into each lane. Once a single cell had been loaded into each lane, the bypass channels were flushed with conditional cell growth media and the pressures were set for long term culture such that media was constantly perfused along the bypass channels (P1 > P2, P3) and very slightly across the trap array (P2 > P3). This reverse-side loading approach ensured that only cells of interest entered the trap array and no cells or debris accumulated in the bypass channel on the left side of the device (channel connecting P1 and P2). Therefore, when cells were released from the trap array and flushed from the left bypass channel there was significantly lower risk of collecting a contaminating cell or piece of debris that had been caught in the channels or tubing during cell loading.

**Trypsinization for cellular re-seeding or release.** Following multigenerational growth in the device, cells were detached from the channel surfaces by flowing in a solution of 0.25% Trypsin and EDTA (Gibco). In order to achieve rapid fluidic exchange while minimizing shear stress on cells within the trap array the pressures were set to have significant flow rate along the bypass channels (P1 ≫ P2, P3) while maintaining only slight flow across the trap lanes (P2 > P3). Fully disassociated cells were either re-seeded in the device for continued culture or released one at a time for downstream collection and sub-clonal outgrowth. Cellular re-seeding was carried out both across and within individual lanes of traps in the array. For instance, for longer-term lineage tracking, a single cell was loaded into one lane of the trap array and allowed to divide for two generations. After trypsin treatment, these cells were released into the left bypass channel (P3 > P2) where the pressures were set to have no flow (P1 = P2) and subsequently re-captured one cell per lane by maintaining slight flow across the array (P2 > P3) and periodically increasing the upstream pressure (P1 > P2) or setting it to atmospheric pressure (P1 < P2) to move cells along the length of the left bypass channel and position them for capture within each lane of the trap array. Following subsequent rounds of cell divisions, cells were re-seeded within each trap lane by gently flowing forward across the trap array (P2 > P3) after detachment with trypsin. In both cases, the cellular detachment and re-capture processes were recorded in order to maintain lineage information collected via time lapse imaging. For single cell collection downstream of the device, the pressures were set to have substantial flow rate along the bypass channels (P1 ≫ P2, P3) after detachment with trypsin - these pressures were set such that the volumetric flow rate along the bypass channel was approximately 15 μl per minute. To release individual cells to the bypass channel, the flow direction was periodically reversed (P3 > P2) until a cell reached the bypass channel at which point flow into the traps was reestablished (P2 > P3) to ensure no other cells were released. Each cell was flushed for 30 seconds (approximately 7.5 μl total volume - in order to clear the dead volume of the system) and collected directly into 70 μl of conditioned cell culture medium in a glass-bottom 384 well plate for continued sub-clonal outgrowth in a standard tissue culture incubator (37° C, 5% $CO_2$).

**Cells identity tracking and lineage reconstruction by time lapse image analysis.** Time-lapse imaging was conducted with a custom LabView program (National Instruments) which drove a TTL-triggered white LED light-source (ThorLabs) for illumination as well as two automated stages (Newport), which traversed the x and y axes to capture multiple fields of view for each frame. Images were taken every 3 minutes (using 10X magnification lens). Lineage structure and time to division measurements were determined by manually tracking the recorded image series using ImageJ software (Example image series shown in Supp. movie 1). The trypsin release and re-seeding and single cell release image series were captured continuously using a lower magnification (4X) lens that captured the entire device image in a single field of view. These image series were analyzed with assistance of custom Python code based on the openCV library package that pre-analyzed the movies and marked each cell with a different color to ease human analysis, which was performed with iMovie software (Apple, Inc.).

**Cell growth measurements.** Single cells released into separate wells on glass bottom plate for sub-clonal culture were immediately imaged upon collection to validate the presence of a single cell per well. The growth of sub clones was monitored every 24 - 48 hours, and fresh conditional medium was introduced every 48 hours. For HT115 lineage, Applicants isolated 37 cells (out of 45 cells in the channel), of which 11 grew as sub-clonal cultures and processed for sequencing. For RPE1, Applicants isolated 22 single cells (out of 26 cells in the channel), of which 15 grew as sub-clonal cultures and 13 were processed for sequencing.

**gDNA extraction and Library Construction.** Colonies were grown to at least $10^6$ cells. Genomic DNA was extracted from each sample using the QIAamp DNA Mini kit (Qiagen). PCR-free library construction was performed by the Genomics Platform at the Broad Institute using their standard process. All sample information and tracking was performed by automated LIMS messaging. Samples undergo fragmentation by means of acoustic shearing using Covaris focused ultrasound shearing instrument to provide fragments of approximately 385 bp. Following fragmentation, size selection was performed using a SPRI cleanup. Library preparation was performed using a commercially available kit (product KK8202, KAPALIBPREPKT, KAPA Biosystems) that entailed palindromic forked adapters with unique 8 base index sequences embedded within the adapter (the DNA oligonucleotide adaptors were purchased from IDT). Following library construction, each library was quantified using quantitative PCR (qPCR; KAPA Library Quantification kit (ABI Prism) from KAPA Biosystems). This qPCR quantification assay was automated using Agilent's Bravo liquid handling platform. Based on the qPCR quantification, libraries were normalized to 1.7 nanomolar. Library samples were then pooled into groups of 24 samples, and the 24-plex pools were once again quantified by qPCR. Library pools were then combined with HiSeq X Cluster Amp Mix 1, 2 and 3 in a tube strip using the Hamilton Starlet Liquid Handling system. Cluster amplification of the templates was performed according to Illumina's protocol using the cBot instrument (Illumina). Clustered flow cells were then shotgun sequenced on HiSeqX to approximately 35-fold coverage of the genome using proprietary sequencing by synthesis (SBS) reagents (Illumina HiSeqX), then analyzed using RTA2.

**Primary analysis of genomic data.** Sequence read alignment, data aggregation, preliminary production analysis and quality control proceeds after sequencing using the automated Picard pipeline (Broad Institute Genomics Platform, (picard.sourceforge.net/). The Picard pipeline produces high quality recalibrated sample level BAM files using the following procedure. Reads were extracted from sequencing instruments and aligned using BWA against the GRCh3 7/hg 19 reference. Duplicate reads were marked for downstream interpretation in the analysis pipeline. Reads around known indel sites were realigned to produce improved alignments. Quality scores were recalibrated using the GATK base quality score recalibrator to increase the accuracy of reported base quality scores. Cell line identity was verified against reference genotype fingerprints. Data were aggregated per sample in BAM format including base calls, quality scores, and alignment data. Finally, summary metrics were generated to allow quality assessment.

**Copy number variation analysis.** Copy number variation (CNV) analysis for each cell line (Figure S3) was carried out by the procedure outlined below and showed that HT115 cells were largely diploid as expected. However, CNV analysis showed that the RPE1 cells were predominantly triploid, which might be related to genomic instability caused by telomerase/telomere dysfunction(Garbe et al. 2014), as the RPE1 line was originally immortalized by telomerase overexpression. For CNV analysis, reads were counted for each sample in 10,000-base bins using the GATK 4 Tool SparkGenomeReadCounts function and divided by the median bin coverage. By exploiting the fact that each sample came from a pure sub-clonal population, coverage was scaled by a factor of $x$ where $x$ minimized the objective sum{bins}(abs($x$ coverage(bin) - nearest integer($x$ coverage(bin))). Coverage scores were scaled so that they lined up with integers as well as possible. The absolute (but noisy) coverage level values were input into a Hidden Markov Model (HMM) with integer copy number states 0, 1, 2, 3, 4, 5. This HMM learned transition matrix elements between states via an expectation-maximization (EM) algorithm. The observed coverage was modeled as a Cauchy distribution, centered at the integer copy number values and the model learned the width parameter of this emission distribution via the EM algorithm. The parameters were updated by an M step consisting of numerical optimization while the E step consisted of obtaining posteriors from the forward-backward algorithm. Once the model had converged, the Viterbi algorithm was used to obtain segments of constant copy number.

**SNV analysis.** Raw single nucleotide variants (SNVs) were identified using MuTect (Cibulskis et al. 2013) within the "Firehose" pipeline, developed at the Broad Institute (www.broadinstitute.org/cancer/cga). MuTect was run for every pair of sub clones twice, such that each sample acted as a "tumor" sample in one run for a sub clone pair, and as a "normal" sample in another run of the same sub clone pair. The SNV lists from the MuTect runs for each lineage experiment were combined and de-duplicated using a custom Python script.

**Lineage sequencing analysis pipeline.** Raw SNVs for each lineage experiment were extracted from the combined MuTect output list. Candidate coincident SNVs, identical SNVs appearing at matching genomic loci in two or more but not all sub clones from a lineage experiment were identified. DNA base counts were extracted for all sub clones at the locus for each coincident SNV. SNVs that contained low coverage (<5 reads) of alternate alleles (compared with the reference) in one of the variant samples were not pursued further. Coincident SNVs were called where the quality of coincident SNV classification was assessed by calculating the probability for each sample to belong to its consensus-assigned group ('reference' or 'alternative') considering the base content in the read alignment at the locus in question for each sample. The probability was calculated using a binomial distribution test for each of the samples. Assuming each sample can belong to the assigned reference or variant group (H0) or to another group (H1). The calculated probability serves a heuristic score to rank the quality of coincident SNV groups.

H0: P = P1, the probability weight that the sample belongs to group1, the assigned reference or variant group.

H1: P = P2, the probability weight that the sample belongs to group 2, a different group than assigned.

$$P1 = P(alt_{allele} = x \in group1) = \binom{\#alt}{total\_nucleotides} P1^{\#alt}(1 - P1)^{\#!alt}$$

$$P2 = P(alt_{allele} = x \in group2) = \binom{\#alt}{total\_nucleotides} P2^{\#alt}(1 - P2)^{\#!alt}$$

$$P(H0 \mid \text{alternative allele} = x) = \frac{P1}{P1+P2}$$

For each classified SNV Applicants calculated this probability for every sample ($\frac{P1}{P1+P2}$) and choose the minimal probability to represent the quality of this coincident classification option to the SNV. Applicants depleted all SNVs with quality of this coincident classification less than 0.99 (Figure 30).

Two approaches were used to obtain a prior estimate of the lineage structure for use in calling the final branch variant sets:

Method 1: In the "lineage -> called variants" approach, the lineage structure was determined by analysis of time lapse imaging of cells in the microfluidic trap array device. This lineage structure was subsequently used for calling branch variants. SNVs were grouped as coincident SNVs and branch variants called by evaluating the coincident SNV quality score for the highest scoring group of sub clones over all the groups of sub clones consistent with the lineage structure determined by time lapse imaging, including the option that an SNV is only truly present in a single sub clone and represents a leaf variant.

$$\text{Coincident SNV Quality Score} = (\text{MAX P}_{\text{clusters}} (\text{Min P}_{\text{sample}} (\frac{P1}{P1+P2}))).$$

Method 2: In the "raw variants -> lineage -> called variants" approach, Applicants used the shotgun sequence data to estimate the most likely lineage structure in each lineage experiment. This was accomplished by identifying coincident SNVs by analysis of raw SNV calls from the sub clones generated by MuTect (see methods section on Lineage sequencing analysis pipeline). Sub clones sharing the same SNVs were grouped without restrictions by hierarchical clustering and for each coincident SNV, the quality score was calculated Coincident SNV Quality Score = $(\text{Min P}_{\text{samples}} (\frac{P1}{P1+P2}))$. The frequency of coincident SNVs for each group of sub clones was tabulated and ranked to generate the plots in Figure 22b. For the experiments with both cell lines, the groups of sub clones sharing a large number of high-scoring coincident SNVs nested in such a way as to indicate a single lineage structure for each of the experiments. This lineage structure was subsequently used for calling branch variants. The prior estimated lineage structure was used to filter the high-quality coincident SNV list for coincident SNVs that were consistent with the lineage structure and produce the final branch variant call set.

**Classifying SNVs in the first cell division of the lineage.** By grouping the samples in each branch variant SNV and comparing to the base call at the same locus in a sister lineage, Applicants could determine which was the reference allele, which was the alternative, and calculate the variant allele fraction without depending on an external reference allele. However, for the first cell division, no such sister lineage exists within the dataset, so Applicants do need an external reference to identify the reference genotype for the cell that founded each lineage experiment. In the HT115 lineage, this involved the split across sub clones {{47,54}; {34,44,49,63,48,45,38,56,57}} and in the RPE1 lineage, the split across sub clones {{46,39,34,37,38}; {28,32,36,24,27,44,23,22}}. In order to resolve this issue, Applicants compared each branch variant SNV in these groups to the reference genome (hg19) for RPE1 cells, and assigned alleles matching hg19 as reference. Applicants were less confident in using hg19 to identify reference alleles in the fast-mutating HT115 cells, so instead Applicants used base calls from an additional HT115 sub clone Applicants sequenced from a different HT115 lineage experiment (seeded from the same HT115 cell stock) to identify reference alleles at the top of the HT115 lineage presented in this study. An additional consistency check to determine if the correct alleles after the first cell division were assigned as reference was to check that the majority of the SNVs represent homozygote to heterozygous changes as expected, which was indeed found to be the case.

**Cell line authentication.** Cell identity was authenticated by the Broad Genomics Platform for HT115 using previously stored fingerprint genotypes. The fingerprint consists of the genotype at 82 loci from the query sample which were compared against fingerprints of all the cancer cell line encyclopedia (CCLE) cell lines using the farthest neighbor graph (FNG) algorithm. The highest correlation (0.83) was found between the HT115 sample and the CCLE HT115 sample. For RPE1 (which is not a CCLE cell line), Applicants used the ATCC short tandem repeat (STR)-based authentication service. The STR profile of the RPE1 sample validated with 100% similarity to hTERT RPE1.

**POLE tumor WGS datasets.** Applicants assembled a collection of 10 whole genome tumor datasets that were published and annotated with POLE coding mutations and mutator phenotype from the Cancer Genome Atlas (TCGA; dbGAP: phs000178.v1.p1)(Haradhvala et al. 2016).

**Mutation spectrum calculation.** Mutational spectra were defined using standard approaches and reported in the standard format(Alexandrov et al. 2013b). Each SNV was classified into one of six subtypes; C:G>A:T, C:G>G:C, C:G>T:A, T:A>A:T, T:A>C:G, and T:A>G:C. and further refined by including the sequence context of each mutated base (one base 3' and one base 5'). For example, a C:G>T:A mutation can be characterized as TpCpG>TpTpG (mutated base underlined and presented as the pyrimidine partner of the mutated base pair) generating 96 possible mutation types (6 types of substitution * 4 types of 5' base * 4 types of 3' base). Applicants compared the mutational spectra from the HT115 and RPE1 branch variant and leaf variant SNV call sets with patterns extracted from POLE mutant clinical and cell line (ATCC) samples (Figure 3a and table S1). The similarity between two mutational patterns A and B, defined as a non-negative vector with 96 mutation types, was computed by cosine similarity:

$$sim(A,B) = \frac{\sum_{i=1}^{n} A_i B_i}{\sqrt{\sum_{i=1}^{n} A_i^2} \sqrt{\sum_{i=1}^{n} B_i^2}}$$

The cosine similarity value indicates mutational spectra are more similar to one another. 95% confidence intervals were calculated by bootstrapping the SNV list $10^4$ times.

**Replication timing analysis.** DNA replication timing for HT115 cells was measured according to a previously described method(Koren et al. 2012). Briefly, 50 million cells were fixed with EtOH, treated with RNaseA, and stained with Propidium Iodide (PI) for DNA content. G1 and S phase cells were sorted using the FACSAria cell sorter (Beckton Dickinson; 1 million cells per fraction), and genomic DNA was extracted and whole-genome sequenced. Replication timing was calculated by counting the number of S phase reads in consecutive windows containing 200 G1 reads along each chromosome, filtering outlier data points and smoothing the data with a cubic smoothing spline. Applicants arbitrarily divided the genome into early- ($\geq$60), intermediate- (>33 & <60) and late ($\leq$33) replicating bins. The 95% confidence intervals (presented as error bars) were calculated by bootstrapping the SNV list $10^4$ times. Replication timing data for RPE1 dataset were obtained from a general previously published dataset(Koren et al. 2012).

**Branch variant SNV occurrence in genes and exons.** Genomic regions consisting of genes, exons, and introns were determined from RefSeq gene tables(Pruitt et al. 2007). The total fraction of these regions in the genome were counted and normalized with attention to the locus-wise CNV calls. The log2(observed/expected) ratios of the branch variant SNV count values in each genomic region type were calculated. One-tailed binomial tests were performed to calculate the statistical significance of deviations by the observed counts from the expected number of mutations (based on the average genome-wide count for each lineage experiment and the size of each genomic region type considered) using binomial statistics (custom Python code); P<0.01 was considered significant. The 95% confidence intervals (presented as error bars) were calculated by bootstrapping the SNV list $10^4$ times, recalculating results for each list, and determining the 0.025th and 0.975th quantile values.

**Analysis of replication-class (R class) asymmetry.** Left-and right-replicating regions were calculated from replication timing measurements as described previously(Haradhvala et al. 2016). Regions with 0.1<= slope <=0.3 units/interval were designated right-replicating, and regions with -0.3<= slope <=-0.1 were designated left-replicating. In order to determine the reference strand asymmetries (a control for other types of asymmetry), each of the twelve possible substitutions with respect to the genomic reference strand (six base pair changes x two orientations) were counted and normalized by the number of corresponding bases in the genome to measure mutations/Mb. Rates of complementary mutations (e.g. C>A and G>T) were then compared. To measure replicative strand asymmetries, all SNV were counted with respect to the leading strand template as described(Haradhvala et al. 2016) using the left- and right-replicating regions defined above. For example, C>A mutations in the leading strand reference are considered to be genomic reference strand C>A mutations in left-replicating regions and genomic reference strand G>T mutations in right-replicating regions. Mutation rates were again calculated by normalizing for the number of corresponding bases in the genome within the intervals with defined replication direction and the complementary mutations were compared. Error bars for mutation rates represent a 95% confidence interval for the underlying binomial probability of a given base being mutated, calculated from the beta distribution parameterized as Beta(n+1,N-n+1), where n is the number of a given mutation and N is the size of the genomic territory of the mutated base. P-values represent the binomial probability of seeing a given distribution of complementary mutations, assuming the probability of a given mutation is determined solely by the base composition within an interval (e.g. the probability of seeing a C>A instead of a G>T is the proportion of C:G base-pairs of with a C on the strand of reference. This will be very near a value of 0.5). Strand asymmetries were calculated as log2 of the ratio of complementary mutation counts. Error bars represent a 95% confidence interval on the log2 quotient of the underlying binomial probabilities above. These confidence intervals were determined empirically by taking 1000 pairs of samples from the beta distribution above for both complementary mutation sets, taking the log2 quotient of each sampled pair and determining the 0.025th and 0.975th quantiles of the resulting distribution.

**Calculation of branch variant SNV sensitivity and specificity in lineage sequencing.** False positive and false negative SNV call rates were estimated by applying the "raw variants -> lineage -> called variants" approach to generate calls and analyzing the results in the context of the microscopic derived lineage structure. The consensus lineage structure estimated agreed with the lineage structure determined by time lapse imaging and was assumed to be the true lineage structure for this analysis. In the following, coincident SNVs were considered to be true positives when in agreement with the consensus lineage structure, and false positives when conflicting with the consensus lineage structure.

Next, Applicants prepared a scrambled version of the dataset where sub clone labels were randomly scrambled for each SNV call, and false positive SNV call rates were calculated by summing the count of coincident SNVs that agreed with the consensus lineage in the scrambled dataset. The data were scrambled by building a list of 10,000 alternative scrambling patterns. Each pattern was checked to verify that each real sub clone set representing the known true lineage structure was indeed disrupted by the scrambling operation and would not create bias. For each SNV Applicants randomly picked a scrambling pattern from the list and reassigned SNVs to the new sub clone identities. The scrambled data set was reanalyzed and the false positive coincident SNV rate found to be 5 SNVs for the HT115 lineage experiment and 24 SNVs for the RPE1 lineage experiment where the quality threshold for accepting coincident SNVs was set to >= 0.99 (Figure S8).

False negative SNV counts were estimated by counting the number of true SNVs that were filtered out as a result of their failure to surpass the required quality threshold for accepting coincident SNVs. Applicants recognize that these "missing" SNVs could likely be imputed to improve the sensitivity of the branch variant SNV call sets, but Applicants have not performed such an imputation in this study and seek here to report the "raw" false negative rate. Applicants start with the true positive branch variant SNVs, then look in sub clones where the consensus lineage structure would have predicted the same SNV to exist but none was called due to the "missing" SNV falling short of the quality threshold applied (0.99). From the counts of these "missing" SNVs, Applicants subtract the estimated false positive counts separately estimated at this quality threshold to arrive at the estimated false negative SNV count.

FN = [#branch_variants < threshold] - [#scrambled_branch_variants < threshold]

With True Negative (TN), False Positive (FP), True Positive (TP), and False Negative (FN) counts, Applicants could estimate the specificity and sensitivity of lineage sequencing for the HT115 and RPE1 lineage sequencing datasets with the coincident SNV quality threshold at 0.99:

$$Specificity = TN/(TN+FP) = 0.999 \text{ (HT115 and RPE1)}$$

$$Sensitivity = TP/(TP+FN) = 0.964 \text{ (HT115) and } 0.918 \text{ (RPE1)}.$$

Applicants then estimated the sensitivity and specificity of SNV calls using the above estimation approach as a function of the coincident SNV quality threshold value to produce the plots in Figure 30. An additional possible source of false negative branch variant SNV calls are coincident SNV calls that surpass the quality threshold but only partially agree with the consensus lineage structure and were excluded. For example, coincident SNVs that agree with 2 out of 3 samples in a clade of the consensus lineage structure. These cases were not found to be higher than expected based on background raw SNV noise.

**Generation of lineage dendrograms from a sequence distance metric.** Dendrograms representing cell lineage relations between samples were measured by counting the number of high-quality coincident SNVs between every pair of samples normalized by the total number of high-quality coincident SNVs. One minus this matrix of similarity scores results matrix of distance scores between every pair of samples. This pairwise distance matrix was used to render dendrograms using the MATLAB seqlinkage tool.

**Statistical analysis of mutation rate and quantile-quantile plotting.** The counts of branch variants (Figure 2) were assumed to follow independent Poisson distributions(Rubin 2013) with a constant rate proportional to the number of cell divisions (=generations).

Let yi be the number of mutation per branch, and $y_i \mid \lambda \sim$ Poisson($\lambda$*# of generations).

Applicants assume the non-informative prior distribution of $\lambda \mid$Gamma($\alpha,\beta$) with $\alpha$=0, $\beta$=0. So, the posterior distribution is then:

$$\lambda|y \sim \text{Gamma}(\alpha+n*y_{\text{mean}},\beta+n) \, ,$$

Applicants then simulated the model (using custom R code) $10^6$ times, and the number mutations per generation and determined as well as the 0.025th and 0.975th quantiles of the resulting distribution. These counts were normalized by dividing out the total size of the genome taking into account the regional copy number variation of each line (for HT115, total bp of $5.7*10^9$, and for RPE1, total bp of $9.15*10^9$) to get the mutation rate with units of SNV/bp/cell division.

The P-value calculations for branch variant counts in each lineage segment were done by simulation of the Poisson model $10^6$ times with dependence on the segment length. The quantile-quantile plot produced by correlating the observed Log10 of the sorted P-values, against the log10 of the expected P-values.

**Haploid mutation count validation.** The full list of branch and leaf variants in the HT115 lineage experiment was reduced to those SNVs that occur in haploid regions by filtering allele fraction SNVs where both the reference allele fraction was greater than 0.9 and the alternative allele fraction was also greater than 0.9. Leaf variant SNVs in this set are likely to be true SNVs that occurred in the last generation of the lineage experiment. The total length of haploid regions was calculated in consideration of measured copy number variation and totaled $3.05 \times 10^8$ bp. Applicants estimated the mutation rate in these regions in conjunction with the branch and leaf variant counts. Applicants then simulated the Poisson counting statistics as described before $10^6$ times and determined the 0.025th and 0.975th quantiles of the resulting distribution.

**Detection of different SNVs that occurred at identical genomic positions:** In order to identify SNVs that occurred at identical genomic positions, Applicants modified the "raw variants -> lineage -> called variants" approach by allowing initial clustering into three groups ('reference', 'alternative1' or 'alternative 2'). Initial coincident SNVs were then determined by hierarchical clustering of three groups for each SNV (Scipy hierarchy fcluster). The center of every cluster was calculated and the quality of coincident SNV classification was calculated similarly as above, by the probability of each sample to belong to its consensus-assigned group:

$$P(\text{sample} \in \text{assigned group}) = \frac{P1}{P1+P2+P3}$$

The final quality of the multiple event coincident mutations was determined by taking the minimum probability sample as describing each sample

$$\text{Coincident SNV Quality Score} = (\text{Min P}_{\text{sample}} (\frac{P1}{P1+P2+P3})).$$

The estimate of the probability of getting multiple variants coincide for uniform probability along the genome was calculated by simulating $10^6$ times the chance of obtaining 7 cases of multiple variants coincide from 3000 events for HT115 (or 4 cases in 700 events for RPE1) in $3 \times 10^9$ sites. This analysis results in an estimate of the upper limit of the p-value because the procedure for calling multiple mutation events further requires that the events co-occur within a sub-lineage.

**References**

Albertson TM, Ogawa M, Bugni JM, Hays LE, Chen Y, Wang Y, Treuting PM, Heddle J a, Goldsby RE, Preston BD. 2009. DNA polymerase epsilon and delta proofreading suppress discrete mutator and cancer phenotypes in mice. Proc Natl Acad Sci USA 106: 171014. www.ncbi.nlm.nih.gov/pubmed/19805137%5Cnwww.pubmedcentral.nih.gov/articlerender.fcgi? artid=PMC2761330.

Alexandrov LB, Nik-Zainal S, Wedge DC, Aparicio SAJR, Behjati S, Biankin A V, Bignell GR, Bolli N, Borg A, Borresen-Dale A-L, et al. 2013a. Signatures of mutational processes in human cancer. Nature 500: 415-21. www.ncbi.nlm.nih.gov/pubmed/23945592.

Alexandrov LB, Nik-Zainal S, Wedge DC, Campbell PJ, Stratton MR. 2013b. Deciphering Signatures of Mutational Processes Operative in Human Cancer. Cell Rep 3: 246-259. dx.doi.org/10.1016/j.celrep.2012.12.008.

Araten DJ, Golde DW, Zhang RH, Thaler HT, Gargiulo L, Notaro R, Luzzatto L. 2005. A quantitative measurement of the human somatic mutation rate. Cancer Res 65: 8111-7. www.ncbi.nlm.nih.gov/pubmed/16166284.

Barretina J, Caponigro G, Stransky N, Venkatesan K, Margolin AA, Kim S, Wilson CJ, Lehar J, Kryukov G V, Sonkin D, et al. 2012. The Cancer Cell Line Encyclopedia enables predictive modelling of anticancer drug sensitivity. Nat (London, UK) 483: 603-607.

Baslan T, Hicks J. 2017. Unravelling biology and shifting paradigms in cancer with single-cell sequencing. Nat Rev Cancer 17: 557-569. dx.doi.org/10.1038/nrc.2017.58.

Bi X. 2015. Mechanism of DNA damage tolerance. World J Biol Chem 6: 48. www.wjgnet.com/1949-8454/full/v6/i3/48.htm.

Blokzijl F, de Ligt J, Jager M, Sasselli V, Roerink S, Sasaki N, Huch M, Boymans S, Kuijk E, Prins P, et al. 2016. Tissue-specific mutation accumulation in human adult stem cells during life. Nature 538: 260-264. dx.doi.org/10.1038/nature19768.

Bodnar AG. 1998. Extension of Life-Span by Introduction of Telomerase into Normal Human Cells. Science (80- ) 279: 349-352. www.sciencemag.org/cgi/doi/10.1126/science.279.5349.349.

Campbell PJ, Martinocorena I, Campbell PJ. 2015. Somatic mutation in cancer and normal cells. Science (80-) 349: 1483-1489.

Chen C, Xing D, Tan L, Li H, Zhou G, Huang L, Xie XS. 2017. Single-cell whole-genome analyses by Linear Amplification via Transposon Insertion (LIANTI). Science (80- ) 356: 189-194. science.sciencemag.org/content/356/6334/189.

Cibulskis K, Lawrence MS, Carter SL, Sivachenko A, Jaffe D, Sougnez C, Gabriel S, Meyerson M, Lander ES, Getz G. 2013. Sensitive detection of somatic point mutations in impure and heterogeneous cancer samples. Nat Biotechnol 31: 213-219. www.nature.com/doifinder/10.1038/nbt2514.

Drost J, van Boxtel R, Blokzijl F, Mizutani T, Sasaki N, Sasselli V, de Ligt J, Behjati S, Grolleman JE, van Wezel T, et al. 2017. Use of CRISPR-modified human stem cell organoids to study the origin of mutational signatures in cancer. Science 3130: eaao3130. www.ncbi.nlm.nih.gov/pubmed/28912133.

Dulak AM, Stojanov P, Peng S, Lawrence MS, Fox C, Stewart C, Bandla S, Imamura Y, Schumacher SE, Shefler E, et al. 2013. Exome and whole-genome sequencing of esophageal adenocarcinoma identifies recurrent driver events and mutational complexity. Nat Genet 45: 478-486. www.nature.com/doifinder/10.1038/ng.2591.

Fernández LC, Torres M, Real FX. 2016. Somatic mosaicism: on the road to cancer. Nat Rev Cancer 16: 43-55. www.ncbi.nlm.nih.gov/pubmed/26678315.

Forbes SA, Beare D, Gunasekaran P, Leung K, Bindal N, Boutselakis H, Ding M, Bamford S, Cole C, Ward S, et al. 2015. COSMIC: Exploring the world's knowledge of somatic mutations in human cancer. Nucleic Acids Res 43: D805-D811. www.ncbi.nlm.nih.gov/pubmed/25355519 (Accessed June 8, 2017).

Frieda KL, Linton JM, Hormoz S, Choi J, Chow KHK, Singer ZS, Budde MW, Elowitz MB, Cai L. 2017. Synthetic recording and in situ readout of lineage information in single cells. Nature 541: 107-111. dx.doi.org/10.1038/nature20777.

Frigola J, Sabarinathan R, Mularoni L, Muinõs F, Gonzalez-Perez A, Lopez-Bigas N. 2017. Reduced mutation rate in exons due to differential mismatch repair. Nat Genet 49: 1684-1692.

Garbe JC, Vrba L, Sputova K, Fuchs L, Novak P, Brothman AR, Jackson M, Chin K, La Barge MA, Watts G, et al. 2014. Immortalization of normal human mammary epithelial cells in two steps by direct targeting of senescence barriers does not require gross genomic alterations. Cell Cycle 13: 3423-3435.

Gawad C, Koh W, Quake SR. 2016. Single-cell genome sequencing: Current state of the science. Nat Rev Genet 17: 175-188. dx.doi.org/10.1038/nrg.2015.16.

Geacintov NE, Broyde S. 2017. Repair-Resistant DNA Lesions. Chem Res Toxicol 30: 1517-1548.

Haradhvala NJ, Polak P, Stojanov P, Covington KR, Shinbrot E, Hess JM, Rheinbay E, Kim J, Maruvka YE, Braunstein LZ, et al. 2016. Mutational Strand Asymmetries in Cancer Genomes Reveal Mechanisms of DNA Damage and Repair. Cell 164: 538-549. dx.doi.org/10.1016/j.cell.2015.12.050.

Holstege H, Pfeiffer W, Sie D, Hulsman M, Nicholas TJ, Lee CC, Ross T, Lin J, Miller M a, Ylstra B, et al. 2014. Somatic mutations found in the healthy blood compartment of a 115-yr-old woman demonstrate oligoclonal hematopoiesis. Genome Res 24: 733-42. www.pubmedcentral.nih.gov/articlerender.fcgi?artid=4009603&tool=pmcentrez&rendertype=ab stract (Accessed May 25, 2014).

Huff JT, Plocik AM, Guthrie C, Yamamoto KR. 2010. Reciprocal intronic and exonic histone modification regions in humans. Nat Struct Mol Biol 17: 1495-1499. www.nature.com/doifinder/10.1038/nsmb.1924.

Ju YS, Martincorena I, Gerstung M, Petljak M, Alexandrov LB, Rahbari R, Wedge DC, Davies HR, Ramakrishna M, Fullam A, et al. 2017. Somatic mutations reveal asymmetric cellular dynamics in the early human embryo. Nature 543: 714-718. www.nature.com/doifinder/10.1038/nature21703.

Kimmerling RJ, Lee Szeto G, Li JW, Genshaft AS, Kazer SW, Payer KR, de Riba Borrajo J, Blainey PC, Irvine DJ, Shalek AK, et al. 2016. A microfluidic platform enabling single-cell RNA-seq of multigenerational lineages. Nat Commun 7: 10220. www.pubmedcentral.nih.gov/articlerender.fcgi?artid=4729820&tool=pmcentrez&rendertype=ab stract.

Koren A, Polak P, Nemesh J, Michaelson JJ, Sebat J, Sunyaev SR, McCarroll SA. 2012. Differential relationship of DNA replication timing to different forms of human mutation and variation. Am J Hum Genet 91: 1033-1040. dx.doi.org/10.1016/j.ajhg.2012.10.018.

Kunkel TA, Erie DA. 2015. Eukaryotic Mismatch Repair in Relation to DNA Replication. Annu Rev Genet 49: 291-313. www.annualreviews.org/doi/10.1146/annurev-genet-112414-054722.

Lange SS, Takata K, Wood RD. 2011. DNA polymerases and cancer. Nat Rev Cancer 11: 96-110. dx.doi.org/10.1038/nrc2998.

Li F, Mao G, Tong D, Huang J, Gu L, Yang W, Li GM. 2013. The histone mark H3K36me3 regulates human DNA mismatch repair through its interaction with MutSα. Cell 153: 590-600. dx.doi.org/10.1016/j.cell.2013.03.025.

Li J, Poursat MA, Drubay D, Motz A, Saci Z, Morillon A, Michiels S, Gautheret D. 2015. A Dual Model for Prioritizing Cancer Mutations in the Non-coding Genome Based on Germline and Somatic Events. PLoS Comput Biol 11: 1-17.

Li R, Montpetit A, Rousseau M, Wu SYM, Greenwood CMT, Spector TD, Pollak M, Polychronakos C, Richards JB. 2014. Somatic point mutations occurring early in development: a monozygotic twin study. J Med Genet 51: 28-34. www.ncbi.nlm.nih.gov/pubmed/24123875.

Lodato MA, Rodin RE, Bohrson CL, Coulter ME, Barton AR, Kwon M, Sherman MA, Vitzthum CM, Luquette LJ, Yandava C, et al. 2017. Aging and neurodegeneration are associated with increased mutations in single human neurons. Science. www.ncbi.nlm.nih.gov/pubmed/29217584.

Lodato MA, Woodworth MB, Lee S, Evrony GD, Mehta BK, Karger A, Lee S, Chittenden TW, D'Gama AM, Cai X, et al. 2015. Somatic mutation in single human neurons tracks developmental and transcriptional history. Science (80- ) 350: 94-98. www.sciencemag.org/cgi/doi/10.1126/science.aab1785.

Lynch M. 2010. Rate, molecular spectrum, and consequences of human mutation. Proc Natl Acad Sci USA 107: 961-8. www.pubmedcentral.nih.gov/articlerender.fcgi?artid=2824313&tool=pmcentrez&rendertype=ab stract (Accessed July 19, 2014).

Makridakis NM, Reichardt JK V. 2012. Translesion DNA polymerases and cancer. Front Genet 3: 1-8.

Martincorena I, Roshan A, Gerstung M, Ellis P, Van Loo P, McLaren S, Wedge DC, Fullam A, Alexandrov LB, Tubio JM, et al. 2015. High burden and pervasive positive selection of somatic mutations in normal human skin. Science (80- ) 348: 880-886. www.ncbi.nlm.nih.gov/pubmed/25999502.

McGranahan N, Swanton C. 2017. Clonal Heterogeneity and Tumor Evolution: Past, Present, and the Future. Cell 168: 613-628. dx.doi.org/10.1016/j.cell.2017.01.018.

McKenna A, Findlay GM, Gagnon JA, Horwitz MS, Schier AF, Shendure J. 2016. Whole-organism lineage tracing by combinatorial and cumulative genome editing. Science (80-) 353: aaf7907. www.sciencemag.org/lookup/doi/10.1126/science.aaf7907.

Merkle FT, Ghosh S, Kamitaki N, Mitchell J, Avior Y, Mello C, Kashin S, Mekhoubad S, Ilic D, Charlton M, et al. 2017. Human pluripotent stem cells recurrently acquire and expand dominant negative P53 mutations. Nature 545: 229-233. www.nature.com/doifinder/10.1038/nature22312.

Milholland B, Dong X, Zhang L, Hao X, Suh Y, Vijg J. 2017. Differences between germline and somatic mutation rates in humans and mice. Nat Commun 8: 1-8. dx.doi.org/10.1038/ncomms15183.

Petljak M, Alexandrov LB. 2016. Understanding mutagenesis through delineation of mutational signatures in human cancer. Carcinogenesis 37: 531-540.

Pruitt KD, Tatusova T, Maglott DR. 2007. NCBI reference sequences (RefSeq): A curated non-redundant sequence database of genomes, transcripts and proteins. Nucleic Acids Res 35: 61-65.

Roach JC, Glusman G, Smit AFA, Huff CD, Hubley R, Shannon PT, Rowen L, Pant KP, Goodman N, Bamshad M, et al. 2010. Analysis of genetic inheritance in a family quartet by whole-genome sequencing. Science 328: 636-9.

www.ncbi.nlm.nih.gov/pubmed/20220176.

Rossi DJ, Jamieson CHM, Weissman IL. 2008. Stems Cells and the Pathways to Aging and Cancer. Cell 132: 681-696.

Rubin AG et al. 2013. Bayesian Data Analysis, Third Edition, 3rd Edition. CRC Press.

Sale JE. 2013. Translesion DNA synthesis and mutagenesis in eukaryotes. Cold Spring Harb Perspect Biol 5.

Sale JE, Lehmann AR, Woodgate R. 2012. Y-family DNA polymerases and their role in tolerance of cellular DNA damage. Nat Rev Mol Cell Biol 13: 141-152. dx.doi.org/10.1038/nrm3289.

Schwartz S, Meshorer E, Ast G. 2009. Chromatin organization marks exon-intron structure. Hum Mol Genet.

Shinbrot E, Henninger EE, Weinhold N, Covington KR, Göksenin AY, Schultz N, Chao H, Doddapaneni H, Muzny DM, Gibbs RA, et al. 2014. Exonuclease mutations in DNA polymerase epsilon reveal replication strand specific mutation patterns and human origins of replication. Genome Res 24: 1740-50. www.ncbi.nlm.nih.gov/pubmed/25228659.

Smith KS, Liu LL, Ganesan S, Michor F, De S. 2017. Nuclear topology modulates the mutational landscapes of cancer genomes. Nat Struct Mol Biol 24: 1000-1006. www.ncbi.nlm.nih.gov/pubmed/28967881.

Supek F, Lehner B. 2015. Differential DNA mismatch repair underlies mutation rate variation across the human genome. Nature 521: 81-84. www.nature.com/doifinder/10.1038/nature14173.

Szikriszt B, Poti Á, Pipek O, Krzystanek M, Kanu N, Molnár J, Ribli D, Szeltner Z, Tusnády GE, Csabai I, et al. 2016. A comprehensive survey of the mutagenic impact of common cancer cytotoxics. Genome Biol 17: 99. genomebiology.biomedcentral.com/articles/10.1186/s13059-016-0963-7.

Tenaillon O, Barrick JE, Ribeck N, Deatherage DE, Blanchard JL, Dasgupta A, Wu GC, Wielgoss S, Cruveiller S, Médigue C, et al. 2016. Tempo and mode of genome evolution in a 50,000-generation experiment. Nature 536: 165-170. www.nature.com/doifinder/10.1038/nature18959.

Tomasetti C, Durrett R, Kimmel M, Lambert A, Parmigiani G, Zauber A, Vogelstein B. 2017. Role of stem-cell divisions in cancer risk. Nature 548: E13-E14. www.nature.com/doifinder/10.1038/nature23302.

Vijg J, Dong X, Milholland B, Zhang L. 2017. Genome instability: a conserved mechanism of ageing? Essays Biochem 61: 305-315. www.ncbi.nlm.nih.gov/pubmed/28550046.

Woodworth MB, Girskis KM, Walsh CA. 2017. Building a lineage from single cells: genetic techniques for cell lineage tracking. Nat Rev Genet 18: 230-244. www.ncbi.nlm.nih.gov/pubmed/28111472.

Wu S, Powers S, Zhu W, Hannun YA. 2016. Substantial contribution of extrinsic risk factors to cancer development. Nature 529: 43-7. www.nature.com/doifinder/10.1038/nature16166.

Ziv O, Zeisel A, Mirlas-Neisberg N, Swain U, Nevo R, Ben-Chetrit N, Martelli MP, Rossi R, Schiesser S, Canman CE, et al. 2014. Identification of novel DNA-damage tolerance genes reveals regulation of translesion DNA synthesis by nucleophosmin. Nat Commun 5.

The invention is defined by the appended claims.

**Claims**

1. A method of detecting somatic mutations across single cell lineages comprising:

   a) isolating single cells from a clonal population, each single cell representing a lineage segment of the clonal population;
   b) sequencing genomic DNA representing each single cell; and
   c) determining true somatic mutations along the single cell lineage of the clonal population based, at least in part, on the sequencing of the genomic DNA representing each single cell, wherein true somatic mutations are determined by tracing the mutations across the cell lineage for each generation and wherein each mutation is observed in at least two cells in the lineage.

2. The method according to claim 1, further comprising culturing a sub-clonal cell population from each single cell representing a lineage segment and sequencing genomic DNA from each sub-clonal cell population; or

   further comprising whole genome amplification (WGA) and sequencing amplified DNA from each single cell; and / or

   wherein the determining step comprises generating a lineage structure of the clonal population that identifies and maps the origin of variants within the clonal population.

3. The method according to claim 1 or 2, wherein the clonal population is derived from expanding a single parent cell;

wherein, optionally, the single parent cell is expanded for at least 2 generations, more preferably, between 5 and 6 generations; or

wherein, optionally, the single parent cell is expanded into 2 to 10 generations; and / or

wherein the method further comprises detecting a mutation signature; and / or

wherein the method further comprises determining a mutation rate; and / or

wherein the method further comprises determining the set of mutations that occurred in individual cell cycles in the lineage; and / or

wherein the method further comprises clustering of mutations in the genome in an individual cell cycle; and / or

wherein the method further comprises determining relationships in the number, type, spectrum, clustering and/or overlap of mutations in mother and daughter cells and/or along a sub-lineage.

4. The method according to any of claims 1 to 3, wherein the clonal population is derived from expanding a single parent cell exposed to or under exposure to one or more perturbations;

wherein, optionally, the cell(s) are exposed to the one or more perturbations prior to expanding and/or during the step of expanding.

5. The method according to claim 4, wherein the perturbation is an environmental condition, a drug, or an agent capable of modulating expression of a gene;

wherein, optionally, the environmental condition is physical, chemical, or biological; or

wherein, optionally, the agent capable of modulating expression of a gene is a CRISPR system, RNAi, TALE, or zinc finger protein;

wherein, further optionally, the agent is inducible.

6. The method according to any of claims 1 to 5, wherein the step of isolating single cells from a clonal population is performed in an automated device; and / or wherein the step of expanding a single parent cell is performed in an automated device.

7. The method according to claim 6, wherein the device is operably linked to a computing system.

8. The method according to any of claims 3 to 7, wherein the single parent cell is loaded into a microfluidic device configured for segregation of single cells across the cell lineage.

9. The method according to any of claims 1 to 8, wherein the single cells are isolated with an optical tweezer; or wherein the single cells are segregated into separate wells.

10. The method according to any of claims 3 to 7, wherein expanding a single parent cell is performed on a microscope slide;

wherein, optionally, the step of isolating single cells from a clonal population is performed on the microscope slide;

wherein, further optionally, the slide is configured for cutting with UV light;

wherein, still further optionally, the slide is a polyethylene-naphthalate (PEN) membrane slide.

11. The method according to claim 10, wherein isolating single cells from the clonal population comprises laser microdissection;

wherein, optionally, the cells are captured on a sticky cap tube; or

wherein, optionally, cells are captured with catapulting.

12. The method according to any of claims 1 to 11, wherein sequencing is whole genome or whole exome sequencing.

13. The method according to any of claims 3 to 11, wherein expanding a single parent cell is visually recorded, whereby single cells across the lineage are tracked; or

wherein the single parent cell is a eukaryotic cell; and / or

wherein the single parent cell is obtained from a subject in need thereof;

wherein, optionally, the single parent cell is a stem cell or induced pluripotent stem cell (iPS); and / or

further comprising detecting phenotypes during the step of expanding a single parent cell;

wherein, optionally, more than one parent cell is expanded and the method further comprises selecting specific cells and/or sub-lineages for genome sequencing based on the observed phenotypes.

14. A method of detecting mutations in bacteria during single replication events comprising:

a) expanding a single bacterial cell into 2 or more generations;
b) segregating single cells from the expanded cells;
c) culturing the single cells, whereby a colony for each cell is obtained;
d) DNA sequencing each colony; and
e) determining true mutations along the single cell lineage, wherein true mutations are determined by tracing the mutations across the cell lineage for each generation and wherein each mutation is observed in at least one pair of daughter cells.

15. The method according to claim 14, wherein the cell is expanded into 2 to 10 generations; and / or

wherein the single cell is exposed to a perturbation during the step of expanding into 2 or more generations; wherein, optionally, the perturbation is an environmental condition, a drug, or an agent capable of modulating expression of a gene;
wherein, further optionally, the environmental condition is physical, chemical, or biological; or
wherein, further optionally, the drug comprises an antibiotic, whereby mutations in response to the antibiotic are detected in single replication events; and / or
wherein the method further comprises detecting a mutation signature; and / or
wherein the single bacterial cell is obtained by diluting a sample of bacteria; or
wherein the single bacterial cell is obtained by sorting a sample of bacteria; or
wherein the single bacterial cell is obtained by separation with an optical tweezer and live single cell microscopy; and / or
wherein the single bacterial cell is loaded into a microfluidic device configured for segregation and recovery of single cells across the cell lineage; and / or
wherein single bacterial cells across a lineage are segregated on a chip and expanded; or
wherein the single cells across a lineage are segregated into separate wells and expanded; and / or
further comprising determining the growth rate of the isolated single cells across a lineage;
wherein, optionally, the growth rate is determined in the presence of an antibiotic; and / or
wherein DNA sequencing comprises loading the bacterial cells from a cell lineage into a microfluidic device capable of segregating each colony and generating a sequencing library for each colony; and / or
wherein the single bacterial cell is obtained from a subject in need thereof; or
wherein the single bacterial cell is obtained from an environmental sample.

**Patentansprüche**

1. Verfahren zum Nachweis von somatischen Mutationen über Einzelzelllinien, das Folgendes umfasst:

a) Isolieren von Einzelzellen aus einer klonalen Population, wobei jede Einzelzelle ein Liniensegment der klonalen Population repräsentiert;
b) Sequenzieren von genomischer DNA, die jede Einzelzelle repräsentiert; und
c) Bestimmen von wahren somatischen Mutationen entlang der Einzelzelllinie der klonalen Population basierend, zumindest teilweise, auf der Sequenzierung der genomischen DNA, die jede Einzelzelle repräsentiert, wobei wahre somatische Mutationen durch Verfolgen der Mutationen über die Zelllinie für jede Generation bestimmt werden und wobei jede Mutation in mindestens zwei Zellen in der Linie beobachtet wird.

2. Verfahren nach Anspruch 1, das weiter das Kultivieren einer subklonalen Zellpopulation aus jeder Einzelzelle, die ein Liniensegment repräsentiert, und die Sequenzierung von genomischer DNA aus jeder subklonalen Zellpopulation umfasst; oder

weiter Gesamtgenom-Amplifikation (WGA) und Sequenzierung von amplifizierter DNA aus jeder Einzelzelle umfasst; und/oder
wobei der Bestimmungsschritt die Erzeugung einer Linienstruktur der klonalen Population umfasst, die den Ursprung von Varianten innerhalb der klonalen Population identifiziert und kartiert.

3. Verfahren nach Anspruch 1 oder 2, wobei die klonale Population aus der Expansion einer einzelnen Mutterzelle stammt;

> wobei optional die einzelne Mutterzelle für mindestens 2 Generationen, bevorzugter zwischen 5 und 6 Generationen expandiert wird; oder
> wobei optional die einzelne Mutterzelle in 2 bis 10 Generationen expandiert wird; und/oder
> wobei das Verfahren weiter das Nachweisen einer Mutationssignatur umfasst; und/oder
> wobei das Verfahren weiter das Bestimmen einer Mutationsrate umfasst; und/oder
> wobei das Verfahren weiter das Bestimmen des Satzes von Mutationen umfasst, die in individuellen Zellzyklen in der Linie aufgetreten sind; und/oder
> wobei das Verfahren weiter das Clustering von Mutationen in dem Genom in einem individuellen Zellzyklus umfasst; und/oder
> wobei das Verfahren weiter das Bestimmen von Beziehungen der Anzahl, des Typs, Spektrums, Clusterings und/oder der Überlappung von Mutationen in Mutter- und Tochterzellen und/oder entlang einer Sublinie umfasst.

4. Verfahren nach einem der Ansprüche 1 bis 3, wobei die klonale Population aus der Expansion einer einzelnen Mutterzelle stammt, die einer oder mehreren Störungen ausgesetzt wurde oder unter Aussetzung stand;
wobei optional die Zelle(n) der einen oder den mehreren Störungen vor der Expansion und/oder während des Schritts der Expansion ausgesetzt werden.

5. Verfahren nach Anspruch 4, wobei die Störung eine Umgebungsbedingung, ein Arzneimittel oder ein Mittel, das die Expression eines Gens modulieren kann, ist;

> wobei optional die Umgebungsbedingung physikalisch, chemisch oder biologisch ist; oder
> wobei optional das Mittel, das die Expression eines Gens modulieren kann, ein CRISPR-System, RNAi, TALE oder Zinkfingerprotein ist;
> wobei weiter optional das Mittel induzierbar ist.

6. Verfahren nach einem der Ansprüche 1 bis 5, wobei der Schritt des Isolierens von Einzelzellen aus einer klonalen Population in einem automatisierten Gerät durchgeführt wird; und/oder
wobei der Schritt der Expansion einer einzelnen Mutterzelle in einem automatisierten Gerät durchgeführt wird.

7. Verfahren nach Anspruch 6, wobei das Gerät funktionsfähig mit einem Rechnersystem verbunden ist.

8. Verfahren nach einem der Ansprüche 3 bis 7, wobei die einzelne Mutterzelle in eine Mikrofluidik-Vorrichtung gegeben wird, die zur Aufteilung von Einzelzellen über die Zelllinie konfiguriert ist.

9. Verfahren nach einem der Ansprüche 1 bis 8, wobei die Einzelzellen mit einer optischen Pinzette isoliert werden; oder
wobei die Einzelzellen in separate Wells aufgeteilt werden.

10. Verfahren nach einem der Ansprüche 3 bis 7, wobei die Expansion einer einzelnen Mutterzelle auf einem Mikroskop-Objektträger durchgeführt wird;

> wobei optional der Schritt des Isolierens von Einzelzellen aus einer klonalen Population auf dem Mikroskop-Objektträger durchgeführt wird;
> wobei weiter optional der Objektträger zum Schneiden mit UV-Licht konfiguriert ist;
> wobei noch weiter optional der Objektträger ein Polyethylennaphthalat (PEN)-Membran-Objektträger ist.

11. Verfahren nach Anspruch 10, wobei das Isolieren von Einzelzellen aus der klonalen Population Lasermikrodissektion umfasst;

> wobei optional die Zellen auf einem Haftdeckelröhrchen eingefangen werden; oder
> wobei optional Zellen durch Katapultieren eingefangen werden.

12. Verfahren nach einem der Ansprüche 1 bis 11, wobei die Sequenzierung Gesamtgenom- oder Gesamtexom-Sequenzierung ist.

13. Verfahren nach einem der Ansprüche 3 bis 11, wobei die Expansion einer einzelnen Mutterzelle visuell erfasst wird, wodurch Einzelzellen über die Linie verfolgt werden; oder

wobei die einzelne Mutterzelle eine eukaryotische Zelle ist; und/oder

wobei die einzelne Mutterzelle von einem Subjekt, das dieses benötigt, erhalten wird;

wobei optional die einzelne Mutterzelle eine Stammzelle oder induzierte pluripotente Stammzelle (iPS) ist; und/oder

das weiter das Nachweisen von Phänotypen während des Schritts der Expansion einer einzelnen Mutterzelle umfasst;

wobei optional mehr als eine Mutterzelle expandiert wird und das Verfahren weiter das Selektieren von spezifischen Zellen und/oder Sublinien für die Genom-Sequenzierung basierend auf den beobachteten Phänotypen umfasst.

14. Verfahren zum Nachweis von Mutationen in Bakterien während einzelnen Replikationsereignissen, das Folgendes umfasst:

a) Expansion einer einzelnen Bakterienzelle in 2 oder mehr Generationen;
b) Aufteilen von Einzelzellen aus den expandierten Zellen;
c) Kultivieren der Einzelzellen, wodurch eine Kolonie für jede Zelle erhalten wird;
d) DNA-Sequenzierung von jeder Kolonie; und
e) Bestimmen von wahren Mutationen entlang der Einzelzelllinie, wobei wahre Mutationen durch Verfolgen der Mutationen über die Zelllinie für jede Generation bestimmt werden und wobei jede Mutation in mindestens einem Paar von Tochterzellen beobachtet wird.

15. Verfahren nach Anspruch 14, wobei die Zelle in 2 bis 10 Generationen expandiert wird; und/oder

wobei die Einzelzelle einer Störung während des Schritts der Expansion in 2 oder mehr Generationen ausgesetzt wird;

wobei optional die Störung eine Umgebungsbedingung, ein Arzneimittel oder ein Mittel, das die Expression eines Gens modulieren kann, ist;

wobei weiter optional die Umgebungsbedingung physikalisch, chemisch oder biologisch ist; oder

wobei weiter optional das Arzneimittel ein Antibiotikum umfasst, wodurch Mutationen als Reaktion auf das Antibiotikum in einzelnen Replikationsereignissen nachgewiesen werden; und/oder

wobei das Verfahren weiter das Nachweisen einer Mutationssignatur umfasst; und/oder

wobei die einzelne Bakterienzelle durch Verdünnen einer Probe von Bakterien erhalten wird; oder

wobei die einzelne Bakterienzelle durch Sortieren einer Probe von Bakterien erhalten wird; oder

wobei die einzelne Bakterienzelle durch Abtrennung mit einer optischen Pinzette und Mikroskopie von lebenden Einzelzellen erhalten wird; und/oder

wobei die einzelne Bakterienzelle in eine Mikrofluidik-Vorrichtung gegeben wird, die zur Aufteilung und Gewinnung von Einzelzellen über die Zelllinie konfiguriert ist; und/oder

wobei einzelne Bakterienzellen über eine Linie auf einem Chip aufgeteilt und expandiert werden; oder

wobei die Einzelzellen über eine Linie in separate Wells aufgeteilt und expandiert werden; und/oder

das weiter das Bestimmen der Wachstumsrate der isolierten Einzelzellen über eine Linie umfasst;

wobei optional die Wachstumsrate in der Gegenwart eines Antibiotikums bestimmt wird; und/oder

wobei DNA-Sequenzierung das Eingeben der Bakterienzellen aus einer Zelllinie in eine Mikrofluidik-Vorrichtung umfasst, die jede Kolonie aufteilen kann und eine Sequenzierungsbibliothek für jede Kolonie erzeugen kann; und/oder

wobei die einzelne Bakterienzelle von einem Subjekt, das dieses benötigt, erhalten wird; oder

wobei die einzelne Bakterienzelle aus einer Umweltprobe erhalten wird.

## Revendications

1. Méthode de détection de mutations somatiques sur l'ensemble de lignées cellulaires uniques, comprenant

a) l'isolement de cellules uniques à partir d'une population clonale, chaque cellule unique représentant un segment de lignée de la population clonale ;
b) le séquençage de l'ADN génomique représentant chaque cellule unique ; et
c) la détermination des mutations somatiques réelles sur l'ensemble de la lignée cellulaire unique de la population clonale en se basant, au moins en partie, sur le séquençage de l'ADN génomique représentant chaque cellule unique, les mutations somatiques réelles étant déterminées en traçant les mutations sur l'ensemble de la lignée cellulaire pour chaque génération, chaque mutation étant observée dans au moins deux cellules de la lignée.

2. Méthode selon la revendication 1, comprenant en outre la culture d'une population cellulaire sous-clonale à partir de chaque cellule unique représentant un segment de lignée et le séquençage de l'ADN génomique de chaque population cellulaire sous-clonale ; ou comprenant en outre l'amplification du génome entier (WGA) et le séquençage de l'ADN amplifié à partir de chaque cellule unique ; et/ou
où l'étape de détermination comprend la génération d'une structure de lignée de la population clonale qui identifie et cartographie l'origine des variants au sein de la population clonale.

3. Méthode selon la revendication 1 ou 2, dans laquelle la population clonale est dérivée de l'expansion d'une cellule mère unique ;

où, éventuellement, la cellule mère unique est développée sur au moins 2 générations, plus préférablement entre 5 et 6 générations ; ou
où, éventuellement, la cellule mère unique est développée sur de 2 à 10 générations ; et/ou
la méthode comprenant en outre la détection d'une signature de mutation ; et/ou
la méthode comprenant en outre la détermination d'un taux de mutation ; et/ou
la méthode comprenant en outre la détermination du set des mutations qui se sont produites dans des cycles cellulaires individuels dans la lignée ; et/ou
la méthode comprenant en outre le regroupement de mutations dans le génome dans un cycle cellulaire individuel ; et/ou
la méthode comprenant en outre la détermination des relations du nombre, du type, du spectre, du regroupement et/ou du chevauchement de mutations dans les cellules mères et filles et/ou sur l'ensemble d'une sous-lignée.

4. Méthode selon l'une quelconque des revendications 1 à 3, dans laquelle la population clonale est dérivée de l'expansion d'une cellule mère unique exposée à une ou plusieurs perturbations ou sous exposition à celles-ci ;
où, éventuellement, la ou les cellules sont exposées aux une ou plusieurs perturbations avant l'expansion et/ou au cours de l'étape d'expansion.

5. Méthode selon la revendication 4, dans laquelle la perturbation est une condition environnementale, un médicament ou un agent capable de moduler l'expression d'un gène ; où, optionnellement, la condition environnementale est physique, chimique ou biologique ; ou

où, optionnellement, l'agent capable de moduler l'expression d'un gène est un système CRISPR, un ARNi, un TALE, ou une protéine à doigt de zinc ;
où, en outre éventuellement, l'agent est inductible.

6. Méthode selon l'une quelconque des revendications 1 à 5, dans laquelle l'étape d'isolement de cellules uniques à partir d'une population clonale est réalisée dans un dispositif automatisé ; et/ou
où l'étape d'expansion d'une cellule mère unique est réalisée dans un dispositif automatisé.

7. Méthode selon la revendication 6, dans laquelle le dispositif est relié de manière opérationnelle à un système informatique.

8. Méthode selon l'une quelconque des revendications 3 à 7, dans laquelle la cellule mère unique est chargée dans un dispositif microfluidique configuré pour la ségrégation de cellules uniques sur l'ensemble de la lignée cellulaire.

9. Méthode selon l'une quelconque des revendications 1 à 8, dans laquelle les cellules uniques sont isolées à l'aide d'une pince optique ; ou
où les cellules uniques sont séparées dans des puits distincts.

10. Méthode selon l'une quelconque des revendications 3 à 7, dans laquelle l'expansion d'une cellule mère unique est réalisée sur une lame de microscope ;

où, éventuellement, l'étape d'isolement de cellules uniques à partir d'une population clonale est réalisée sur la lame de microscope ;

où, en outre éventuellement, la lame est configurée pour être découpée par une lumière UV ; où, encore en outre éventuellement, la lame est une lame à membrane en polyéthylène-naphtalate (PEN).

11. Méthode selon la revendication 10, dans laquelle l'isolement de cellules uniques à partir de la population clonale comprend une microdissection au laser ;

où, éventuellement, les cellules sont piégées sur un tube à bouchon collant ; ou
où, éventuellement, les cellules sont piégées par catapultage.

12. Méthode selon l'une quelconque des revendications 1 à 11, dans laquelle le séquençage est un séquençage du génome entier ou de l'exome entier.

13. Méthode selon l'une quelconque des revendications 3 à 11, dans laquelle l'expansion d'une cellule mère unique est enregistrée visuellement, ce qui permet de suivre les cellules uniques sur l'ensemble de la lignée ; ou

où la cellule mère unique est une cellule eucaryote ; et/ou
où la cellule mère unique est obtenue à partir d'un sujet en ayant besoin ;
où, éventuellement, la cellule mère unique est une cellule souche ou une cellule souche pluripotente induite (iPS) ; et/ou
comprenant en outre la détection de phénotypes au cours de l'étape d'expansion d'une cellule mère unique ;
où, éventuellement, plus d'une cellule mère est développée et la méthode comprend en outre la sélection de cellules et/ou sous-lignées spécifiques pour le séquençage du génome sur la base des phénotypes observés.

14. Méthode de détection de mutations chez les bactéries au cours d'événements de réplication uniques comprenant :

a) l'expansion d'une cellule bactérienne unique sur 2 générations ou plus ;
b) la ségrégation de cellules uniques à partir des cellules développées ;
c) la mise en culture des cellules uniques, ce qui permet d'obtenir une colonie pour chaque cellule ;
d) le séquençage de l'ADN de chaque colonie ; et
e) la détermination des mutations réelles sur l'ensemble de la lignée cellulaire unique, les mutations réelles étant déterminées en traçant les mutations sur l'ensemble de la lignée cellulaire pour chaque génération, et chaque mutation étant observée dans au moins une paire de cellules filles.

15. Méthode selon la revendication 14, dans laquelle la cellule est développée sur de 2 à 10 générations ; et/ou

où la cellule unique est exposée à une perturbation au cours de l'étape d'expansion sur 2 générations ou plus ;
où, éventuellement, la perturbation est une condition environnementale, un médicament ou un agent capable de moduler l'expression d'un gène ;
où en outre éventuellement, la condition environnementale est physique, chimique ou biologique ; ou
où, en outre éventuellement, le médicament comprend un antibiotique, les mutations en réponse à l'antibiotique étant détectées dans des événements de réplication unique ; et/ou la méthode comprenant en outre la détection d'une signature de mutation ; et/ou
où la cellule bactérienne unique est obtenue par dilution d'un échantillon de bactéries ; ou
où la cellule bactérienne unique est obtenue en triant un échantillon de bactéries ; ou
où la cellule bactérienne unique est obtenue par séparation à l'aide d'une pince optique et par microscopie de cellules uniques vivantes ; et/ou
où la cellule bactérienne unique est chargée dans un dispositif microfluidique configuré pour la ségrégation et la récupération de cellules uniques sur l'ensemble de la lignée cellulaire ; et/ou
où les cellules bactériennes uniques sur l'ensemble d'une lignée sont séparées sur une puce et développées ; ou
où les cellules uniques sur l'ensemble d'une lignée sont séparées dans des puits distincts et développées ; et/ou
comprenant en outre la détermination du taux de croissance des cellules uniques isolées sur l'ensemble d'une lignée ;
où, éventuellement, le taux de croissance est déterminé en présence d'un antibiotique ; et/ou
où le séquençage de l'ADN comprend le chargement des cellules bactériennes issues d'une lignée cellulaire dans un dispositif microfluidique capable de séparer chaque colonie et de générer une bibliothèque de séquençage pour chaque colonie ; et/ou
où la cellule bactérienne unique est obtenue à partir d'un sujet en ayant besoin ; ou
où la cellule bactérienne unique est obtenue à partir d'un échantillon environnemental.

lineage sequencing approach

microfluidic device

single-cell sorting w/ lineage tracking

require concordance?

False-postive error rate | False-negative error rate

no
10e-10 | 10e-2
10e-10 | 10e-1

yes
10e-10 | 10e-1
10e-5 | 10e-1

amplification by sub-clonal culture generates bulk gDNA samples for WGS

**FIG. 1**

FIG. 2

# Data analysis

Picard WGS pipeline GP:

Initial alignment (BWA)

Duplicated reads are marked

GATK base quality score recalibrator

Local realignment (higher quality alignments )

GATK base quality score recalibrate

Data is aggregated -> getting BAM files

Build candidate mutation list using all possible sample pairs:

SNP caller-MuTect
Small indels – GATK & Snowman
CNV caller –GATK

Clustering samples with and withou independent knowledge of the tree structure

```
index= 4   chr 1 :   20436959
                A  T  C  G
HT6W34 [ 0 23 26  0]
HT6W37 [ 0  0 39  0]
HT6W45 [ 0  0 37  1]
HT6W47 [ 0  1 48  0]
HT6W48 [ 0  0 25  0]
HT6W49 [ 0 16 24  0]
HT6W54 [ 0  0 32  0]
HT6W63 [ 0 20 17  0]
probability:  0.999999999927
[1, 0, 0, 0, 0, 1, 0, 1]
```

**FIG. 3**

## Total coverage:

Using the UCSC database total size of the human genome (hg19) = 3095677412
The coverage we have from the PCR free genome is 2831092097 91.4%

| MuTect (FH) | Just read with more than 5 depth For all samples | Active search: Just 'Tree mutations' | After Clustering threshold 0.99 | CNV masking Masking 1.4% | AF filtering |

Candidates: 92,360 ➡ 92,289 ➡ 11,402 ➡ 2844 ➡ 2267 ➡ 2260

**FIG. 4**

FIG. 5

EP 3 622 068 B1

**FIG. 6**

Mutation Signature: HT115 PolE mutator line

http://cancer.sanger.ac.uk/cosmic/signatures
Identify the signature of POLE, specific type of POLE mutation.

**FIG. 7**

**FIG. 8**

HT115 and RPE1 mutation signatures differ

FIG. 9

# Mutations distribute randomly across major genomic features

HT115-SNP

|  | CpG islands | Genes | Exons | Introns |
|---|---|---|---|---|
| Observed | 5 | 905 | 38 | 867 |
| Expected | 16 | 992 | 56 | 943 |

FIG. 10

**FIG. 11**

**FIG. 12**

## Microfluidics for High Quality, Low-cost, Fully-integrated Sample Prep

- goal: complete walk-away automation at <$10/sample total cost
- fully-integrated workflow (minimum hands-on time)
- reuseable device (low consumable cost)
- high-throughput device (match NGS capacity)

**Device Picture**
- 96 reactors & filters
- 40 nL reactor volume
- Parallel operation of reactors and filters

**Device workflow**
- dead-end fill
- mix
- filter

current microfluidic workflow

filter cells from microliter sample → apply lysis reagent → SPRI purification → add TN5 enzyme and buffer → add stop solution → SPRI purification → individual sample recovery

non-integrated (plate-based) steps

enrichment PCR and barcoding → SPRI purification → quantify, normalize, & pool library

## FIG. 13

## Clinical Isolate Sequencing: 384 WGS Library Prep

### Large Scale Sample Processing with Microfluidic Device

Primary plate

Control plate

gDNA Input: Low-Input from 50 pg ($10^4$ Cell Eq.)
Clinical P. aeruginosa DNA

Complexity (x) — Map Rate (%)

Sample ID

### High Quality Device Libraries Enable Accurate Base Calling (>Q70 Consensus Base Calling)

Concordant SNP:
Reference: A G C T A G C T
Rep1:    A G T C A G C T
Rep2:    A G T C A G C T
Rep3:    A G T C A G C T

Discordant SNP:
Reference: A G C T A G C T
Rep1:    A G T C A G C T
Rep2:    A G T C A G C T
Rep3:    A G T T A G C T

Filtering Threshold (Allelic Fraction)

Decreasing Filtering Stringency

Fraction of concordant SNPs

Subject P01
Subject P02
Subject P03
Subject P04
Subject P05
Subject P06

Fraction of discordant SNPs

Base Calling Accuracy of Device Libraries

Bench-top 24ng deep seq: 53x
Device 50pg triplicate seq: 53X
Bench-top 24ng 28X
Device 50pg 22X
Device 50pg 20X
Device 50pg 19X
Bench-top 24ng 22X

Sensitivity

1-Specificity    x 10^-7

## FIG. 14

# Diversity of Bacterial Isolates Measured to be High Between Patients but Clonal Within Patients

## Antibiotic Resistance Phenotypes Matches the Measured Genotypes
- SNPs conferring antibiotic resistance were accurately detected

# FIG. 15

EP 3 622 068 B1

EP 3 622 068 B1

## Low-Input Environmental Microbial Genomics

### Low-Input Soil Bacteria Micro-colony Sample Prep for WGS Workflow

soil sample obtained from the environment

soil bacteria diluted and single cell innoculated in the iChip

culture soil bacteria using iChip

microcolonies of 10^4 cells directly loaded on the microfluidic device

cells processed into NGS libraries in the microfluidic device

libraries barcoded, normalized, and pooled on the bench

libraries whole genome sequenced

WGS

### On-Device Sample Processing is Superior over Bench-top Methods for Low-Input Libraries

Cell Input: Ultra Low-Input from 1000 E. coli Cells

Complexity (x) / Map Rate (%) — Sample ID 1 to 36

Cell Input: Low-Input iChip ~10^4 Soil Micro-colony Cells

Library complexity device (x) — 13/14 device libraries show >200x complexity — Library complexity bench-top (x)

Human DNA fraction, device — Low contamination in device libraries — Human DNA fraction, bench-top

### Phylotyping and Biosynthetic Cluster Genes Identified

Phylotyping

***Red numbers = SNPs between strains

Anti-smash Analysis

Anti-smash ESS

**FIG. 16**

# Unbiased Determination of Bacterial Mutations in Single Replication Events

*Due to selection bias from clonal interference and lack of single generation mutation detection ability, deleterious mutations and its distribution of fitness effects have been under studied. With our ability to sort single cells and WGS sequence bacteria at large scale, we will determine all mutations and its phenotype in a single lineage.

## FIG. 17

**Investigation of Evolution through Epigenomic and Genome Structural Variation**

**Short-read sequencing is incapable of measuring all modes of evolution.** a) While short-read sequencing (NGS) can of accurately determine SNVs, it cannot directly detect mutations through b) DNA modification and c) recombination and transposition.

Objectives

1. Develop microfluidic technologies for sequencing sample preparation to enable high quality long-read sequencing measurements at large scales
2. Investigation of the role of DNA methylation in horizontal gene transfer and antibiotic resistance gain in bacteria with *Enterococcus faecalis* in the human microbiome as a model system
3. Study the recombination dynamics between chromosomes within polyploidal *Neisseria gonorrhoeae*
4. Discover the epigenetic and genome structure variations that lead to virulence in *Bordetella pertussis*

**FIG. 18**

1) single cell founds
a clonal population

2) clonal population expands with
optional inline lineage tracing

3) single cells are collected
from the population

4) recovered cells are grown
as sub-clonal cultures

5) genomic DNA is extracted
from sub-clonal cultures

6) gDNA is shotgun-sequenced

7) lineage structure of sub clones
is estimated from inline tracing
or raw variants

8) variants are called jointly across
sub clones using prior lineage
structure estimate. branch variants
with support from >= 2 subclones
have enhanced statistical power

technical error
leaf variant
branch variant

9) variants are mapped to lineage
structure, with single-cell resolution
achieved where coverage of sub
clones is dense

accurate reconstrucion of mutational events

# FIG. 19

| | MuTect raw SNVs | loci with read coverage >= 5 | lineage-consistent coincident SNVs | coincident SNV quality score >= 0.99 | SNVs not associated loss of heterozygosity |
|---|---|---|---|---|---|
| HT115 lineage | 64,770 | 64,628 | 18,533 | 3,027 | 2,779 |
| RPE1 lineage | 50,946 | 50,785 | 17,660 | 663 | 663 |
| | | | | | branch variant call set |

example branch variant
HT115 lineage GG -> GT
Chr 1, locus 111370246

| sub clone | [A | T | C | G] |
|---|---|---|---|---|
| 45 | [0 | 0 | 1 | 41] |
| 47 | [0 | 0 | 0 | 49] |
| 48 | [0 | 0 | 0 | 51] |
| 49 | [0 | 24 | 1 | 23] |
| 54 | [0 | 0 | 0 | 54] |
| 34 | [0 | 21 | 0 | 23] |
| 63 | [0 | 14 | 0 | 15] |
| 56 | [0 | 1 | 0 | 28] |
| 38 | [0 | 0 | 0 | 39] |
| 44 | [0 | 22 | 0 | 23] |
| 57 | [0 | 0 | 0 | 60] |
| out | [0 | 0 | 0 | 36] |

**FIG. 20A**

**FIG. 20B**

**FIG. 20C**

**FIG. 20D**

FIG. 21C

FIG. 21D

FIG. 21A

FIG. 21B

| | MuTect raw SNVs | loci with read coverage >= 5 | total coincident SNVs | coincident SNV quality score >= 0.99 | SNVs not associated loss of heterozygosity | SNVs consistent with prior lineage estimate |
|---|---|---|---|---|---|---|
| HT115 lineage | 64,770 | 64,628 | 41,434 | 3,515 | 3,009 | 2,765 |
| RPE1 lineage | 50,946 | 50,785 | 44,212 | 925 | 811 | 646 branch variant call set |

**FIG. 22A**

**FIG. 22B**

**FIG. 22C**

FIG. 23A

FIG. 23B

FIG. 23C

FIG. 24

**FIG. 25**

**FIG. 26**

FIG. 27A

FIG. 27B

FIG. 28

FIG. 29

**FIG. 30**

FIG. 31

**FIG. 32**

**FIG. 33**

## Haploid SNV counts per generation

**FIG. 34**

FIG. 35

**FIG. 36A**

**FIG. 36B**

**FIG. 36C**

**FIG. 37**

| Sample | Correlation | | Mutation | Type |
|---|---|---|---|---|
| HT115 | | Missense | p.V411L | CRC |
| | | Missense | p.L1235I | |
| COAD-A6-6141 | 0.91 | Missense | p.S297F | CRC |
| COAD-AA-3555 | 0.91 | Missense | p.P286H | CRC |
| COAD-AA-3977 | 0.93 | Missense | p.K777N | CRC |
| | | Missense | p.F367S | |
| COAD-AA-A00N | 0.91 | Missense | p.L1255V | CRC |
| | | Missense | p.V411L | |
| COAD-AZ-4315 | 0.93 | Missense | p.R1826W | CRC |
| | | Missense | p.R1556W | |
| | | Missense | p.V411L | |
| COAD-CA-6717 | 0.97 | Missense | p.L1235I | CRC |
| | | Nonsense | p.R1371* | |
| COAD-CA-6718 | 0.94 | Missense | p.P286R | CRC |
| READ-AG-A002 | 0.9 | Missense | p.S459F | CRC |
| | | Nonsense | p.R150* | |
| READ-EI-6917 | 0.85 | Missense | p.A426V | CRC |
| | | Missense | p.V411L | |
| READ-F5-6814 | 0.96 | Missense | p.P286R | CRC |

# FIG. 38

# Lineage sequencing 2.0

1. Mark coordinates markers on polyethylene-naphthalate (PEN) membrane slide (UV cutable), to preserve locations between different microscopes.

2. Seed cells on coat membrane and image lineages growth

**FIG. 39**

## Lineage sequencing 2.0

3. Process the movies and choose specific lineage to extract.

**FIG. 40**

Lineage sequencing 2.0

4. Capture and isolate single cells.

Zeiss Laser capture microscope (BTL)

Cell media

Cover slip

Microscope lens (LCM)

**FIG. 41**

Lineage sequencing 2.0

FIG. 42

Lineage sequencing 2.0

FIG. 43

# Lineage sequencing 2.0

## 4. Capture and isolate single cells with catapulting

# FIG. 44

## Lineage sequencing 2.0

4. Capture and isolate single cells with catapulting

**FIG. 45**

**FIG. 46**

FIG. 47

Lin-Diff-Seq

FIG. 48

Lin-Diff-Seq

**FIG. 49**

FIG. 50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2015013191 A
- WO 2015050998 A2
- WO 2016138290 A
- WO 2014159356 A1
- US 9115402 B

### Non-patent literature cited in the description

- **STRATTON et al.** *Nature,* 2009, vol. 458, 719-724
- **MARTINCORENA et al.** *Science,* 25 September 2015, vol. 349 (6255), 1483-9
- **SZIKRISZT et al.** *Genome Biology,* 2016, vol. 17, 99
- **CHEN et al.** *Science,* 14 April 2017, vol. 356 (6334), 189-194
- **KENNEDY et al.** *PLoS Genet,* 2015, vol. 11 (4), e1005151
- *Genome Biology,* 2016, vol. 17, 99
- **B. MURRAY.** *New Engl. J. Med.,* 1994, vol. 330, 1229-1230
- **SAMBROOK ; FRITSCH ; MANIATIS.** Molecular Cloning: A Laboratory Manual. 1989
- **GREEN ; SAMBROOK.** Molecular Cloning: A Laboratory Manual. 2012
- Current Protocols in Molecular Biology. 1987
- Methods in Enzymology. Academic Press, Inc,
- PCR 2: A Practical Approach. 1995
- Antibodies, A Laboraotry Manual. 1988
- Antibodies A Laboraotry Manual. 2013
- Animal Cell Culture. 1987
- **BENJAMIN LEWIN.** Genes IX. Jones and Bartlet, 2008
- The Encyclopedia of Molecular Biology. Blackwell Science Ltd, 1994
- Molecular Biology and Biotechnology: a Comprehensive Desk Reference. VCH Publishers, Inc, 1995
- **SINGLETON et al.** Dictionary of Microbiology and Molecular Biology. J. Wiley & Sons, 1994
- **MARCH.** Advanced Organic Chemistry Reactions, Mechanisms and Structure. John Wiley & Sons, 1992
- **MARTEN H. HOFKER ; JAN VAN DEURSEN.** Transgenic Mouse Methods and Protocols. 2011
- **KHODJAKOV, A. ; RIEDER, C. L.** Imaging the division process in living tissue culture cells. *Methods,* 2006, vol. 38, 2-16
- Live Cell Imaging: A Laboratory Manual. Cold Spring Harbor Press, 2005, 631
- **REES et al.** Nanoparticle vesicle encoding for imaging and tracking cell populations. *Nat Methods.,* 14 September 2014, vol. 11 (11), 1177-81
- **SCHOFFNER et al.** *Nucleic Acids Research,* 1996, vol. 24, 375-379
- **KIMMERLING et al.** A microfluidic platform enabling single-cell RNA-seq of multigenerational lineages. *Nat Commun.,* 06 January 2016, vol. 7, 10220
- **LANDRY et al.** Optofluidic cell selection from complex microbial communities for single-genome analysis. *Methods Enzymol.,* 2013, vol. 531, 61-90
- **VANDEWOESTYNE et al.** Laser capture microdissection: should an ultraviolet or infrared laser be used?. *Anal Biochem.,* 15 August 2013, vol. 439 (2), 88-98
- **ESPINA et al.** Laser-capture microdissection technology. *Expert. Rev. Mol. Diagn.,* vol. 7, 647-657
- **HORNEFFER et al.** Principles of laser-induced separation and transport of living cells. *J Biomed Opt.,* September 2007, vol. 12 (5), 054016
- **VOGEL et al.** Principles of laser microdissection and catapulting of histologic specimens and live cells. *Methods Cell Biol.,* 2007, vol. 82, 153-205
- U-Net: Convolutional Networks for Biomedical Image Segmentation. **RONNEBERGER O. ; FISCHER P. ; BROX T.** Medical Image Computing and Computer-Assisted Intervention - MICCAI 2015. Lecture Notes in Computer Science. Springer, 2015, vol. 9351
- **HU et al.** *Curr Opin Biotechnol,* 1997, vol. 8, 148
- **K. KITANO.** *Biotechnology,* 1991, vol. 17, 73
- *Curr Opin Biotechnol,* 1991, vol. 2, 375
- Teratocarcinomas and embryonic stem cells: A practical approach. IRL Press Ltd, 1987
- Guide to Techniques in Mouse Development. Academic Press, 1993
- Embryonic Stem Cells: Methods and Protocols. Humana Press, 2001

- Embryonic Stem Cell Differentiation in Vitro. **M. V. WILES.** Meth. Enzymol. 1993, vol. 225, 900
- **P. D. RATHJEN et al.** Properties and uses of Embryonic Stem Cells: Prospects for Application to Human Biology and Gene Therapy. 1993
- **ROBERTSON.** *Meth Cell Biol,* 1997, vol. 75, 173
- **ROACH ; MCNEISH.** *Methods Mol Biol,* 2002, vol. 185, 1-16
- **PEDERSEN.** *Reprod Fertil Dev,* 1998, vol. 10, 31
- **R. IAN FRESHNEY ; GLYN N. STACEY ; JONATHAN M. AUERBACH.** *Culture of Human Stem Cells,* 2007
- **LAURIE C. DOERING.** *Protocols for Neural Cell Culture,* 2009
- **NAVJOT KAUR ; MOHAN C. VEMURI.** *Neural Stem Cell Assays,* 2015
- **HENNING ULRICH ; PRISCILLA DAVIDSON NEGRAES.** *Working with Stem Cells,* 2016
- **KRISHNENDU ROY.** *Biomaterials as Stem Cell Niche,* 2010
- **HEAD et al.** Library construction for next-generation sequencing: Overviews and challenges. *Biotechniques,* 2014, vol. 56 (2), 61-77
- **DEAN et al.** *Genome Res,* 2001, vol. 11, 1095-1099
- **ZHANG et al.** *Mol Diagn,* 2001, vol. 6, 141-150
- **AVIEL-RONEN et al.** *BMC Genomics,* 2006, vol. 7
- **MELLADO et al.** *Virology,* 1980, vol. 104, 84-96
- **BLANCO ; SALAS.** *Proceedings of the National Academy of Sciences,* 1984, vol. 81, 5325
- **BLANCO et al.** *Journal of Biological Chemistry,* 1989, vol. 264, 8935-8940
- **MORIN et al.** *Proceedings of the National Academy of Sciences,* 2012, vol. 109, 8115-8120
- **DEAN et al.** *Proceedings of the National Academy of Sciences,* 2002, vol. 99, 5261
- **CONG, L. ; RAN, F.A. ; COX, D. ; LIN, S. ; BARRETTO, R. ; HABIB, N. ; HSU, P.D. ; WU, X. ; JIANG, W. ; MARRAFFINI, L.A.** Multiplex genome engineering using CRISPR-Cas systems. *Science,* 15 February 2013, vol. 339 (6121), 819-23
- **JIANG W. ; BIKARD D. ; COX D. ; ZHANG F ; MARRAFFINI LA.** RNA-guided editing of bacterial genomes using CRISPR-Cas systems. *Nat Biotechnol,* March 2013, vol. 31 (3), 233-9
- **WANG H. ; YANG H. ; SHIVALILA CS. ; DAWLATY MM. ; CHENG AW. ; ZHANG F. ; JAEMSCHR.** One-Step Generation of Mice Carrying Mutations in Multiple Genes by CRISPR-Cas-Mediated Genome Engineering. *Cell,* 09 May 2013, vol. 153 (4), 910-8
- **KONERMANN S ; BRIGHAM MD ; TREVINO AE ; HSU PD ; HEIDENREICH M ; CONG L ; PLATT RJ ; SCOTT DA ; CHURCH GM ; ZHANG F.** Optical control of mammalian endogenous transcription and epigenetic states. *Nature,* 22 August 2013, vol. 500 (7463), 472-6
- **RAN, FA. ; HSU, PD. ; LIN, CY. ; GOOTENBERG, JS. ; KONERMANN, S. ; TREVINO, AE. ; SCOTT, DA. ; INOUE, A. ; MATOBA, S. ; ZHANG, Y.** Double Nicking by RNA-Guided CRISPR Cas9 for Enhanced Genome Editing Specificity. *Cell,* 28 August 2013
- **HSU, P. ; SCOTT, D. ; WEINSTEIN, J. ; RAN, FA. ; KONERMANN, S. ; AGARWALA, V. ; LI, Y. ; FINE, E. ; WU, X. ; SHALEM, O.** DNA targeting specificity of RNA-guided Cas9 nucleases. *Nat Biotechnol,* 2013
- **RAN, FA. ; HSU, PD. ; WRIGHT, J. ; AGARWALA, V. ; SCOTT, DA. ; ZHANG, F.** Genome engineering using the CRISPR-Cas9 system. *Nature Protocols,* November 2013, vol. 8 (11), 2281-308
- **SHALEM, O. ; SANJANA, NE. ; HARTENIAN, E. ; SHI, X. ; SCOTT, DA. ; MIKKELSON, T. ; HECKL, D. ; EBERT, BL. ; ROOT, DE. ; DOENCH, JG.** Genome-Scale CRISPR-Cas9 Knockout Screening in Human Cells. *Science,* 12 December 2013
- **NISHIMASU, H. ; RAN, FA. ; HSU, PD. ; KONERMANN, S. ; SHEHATA, SI. ; DOHMAE, N. ; ISHITANI, R. ; ZHANG, F. ; NUREKI, O.** Crystal structure of cas9 in complex with guide RNA and target DNA. *Cell,* 27 February 2014, vol. 156 (5), 935-49
- **WU X. ; SCOTT DA. ; KRIZ AJ. ; CHIU AC. ; HSU PD. ; DADON DB. ; CHENG AW. ; TREVINO AE. ; KONERMANN S. ; CHEN S.** Genome-wide binding of the CRISPR endonuclease Cas9 in mammalian cells. *Nat Biotechnol.,* 20 April 2014
- **PLATT RJ ; CHEN S ; ZHOU Y ; YIM MJ ; SWIECH L ; KEMPTON HR ; DAHLMAN JE ; PARNAS O ; EISENHAURE TM ; JOVANOVIC M.** CRISPR-Cas9 Knockin Mice for Genome Editing and Cancer Modeling. *Cell,* 2014, vol. 159 (2), 440-455
- **HSU PD ; LANDER ES ; ZHANG F.** Development and Applications of CRISPR-Cas9 for Genome Engineering. *Cell,* 2014, vol. 157 (6), 1262-78
- **WANG T ; WEI JJ ; SABATINI DM ; LANDER ES.** Genetic screens in human cells using the CRISPR-Cas9 system. *Science,* 03 January 2014, vol. 343 (6166), 80-84
- **DOENCH JG ; HARTENIAN E ; GRAHAM DB ; TOTHOVA Z ; HEGDE M ; SMITH I ; SULLENDER M ; EBERT BL ; XAVIER RJ ; ROOT DE.** Rational design of highly active sgRNAs for CRISPR-Cas9-mediated gene inactivation. *Nat Biotechnol.,* 03 September 2014, vol. 32 (12), 1262-7

- **SWIECH L ; HEIDENREICH M ; BANERJEE A ; HABIB N ; LI Y ; TROMBETTA J ; SUR M ; ZHANG F.** In vivo interrogation of gene function in the mammalian brain using CRISPR-Cas9. *Nat Biotechnol.,* 19 October 2014, vol. 33 (1), 102-6
- **KONERMANN S ; BRIGHAM MD ; TREVINO AE ; JOUNG J ; ABUDAYYEH OO ; BARCENA C ; HSU PD ; HABIB N ; GOOTENBERG JS ; NISHIMASU H.** Genome-scale transcriptional activation by an engineered CRISPR-Cas9 complex. *Nature,* 2015, vol. 517 (7536), 583-8
- **ZETSCHE B ; VOLZ SE ; ZHANG F.** A split-Cas9 architecture for inducible genome editing and transcription modulation. *Nat Biotechnol.,* 02 February 2015, vol. 33 (2), 139-42
- **CHEN S ; SANJANA NE ; ZHENG K ; SHALEM O ; LEE K ; SHI X ; SCOTT DA ; SONG J ; PAN JQ ; WEISSLEDER R.** Genome-wide CRISPR Screen in a Mouse Model of Tumor Growth and Metastasis. *Cell,* 12 March 2015, vol. 160, 1246-1260
- **RAN FA ; CONG L ; YAN WX ; SCOTT DA ; GOOTENBERG JS ; KRIZ AJ ; ZETSCHE B ; SHALEM O ; WU X ; MAKAROVA KS.** In vivo genome editing using Staphylococcus aureus Cas9. *Nature,* 01 April 2015, vol. 520 (7546), 186-91
- **SHALEM et al.** High-throughput functional genomics using CRISPR-Cas9. *Nature Reviews Genetics,* May 2015, vol. 16, 299-311
- **XU et al.** Sequence determinants of improved CRISPR sgRNA design. *Genome Research,* August 2015, vol. 25, 1147-1157
- **PARNAS et al.** A Genome-wide CRISPR Screen in Primary Immune Cells to Dissect Regulatory Networks. *Cell,* 30 July 2015, vol. 162, 675-686
- **RAMANAN et al.** CRISPR-Cas9 cleavage of viral DNA efficiently suppresses hepatitis B virus. *Scientific Reports,* 02 June 2015, vol. 5, 10833
- **NISHIMASU et al.** Crystal Structure of Staphylococcus aureus Cas9. *Cell,* 27 August 2015, vol. 162, 1113-1126
- **CANVER et al.** BCL11A enhancer dissection by Cas9-mediated in situ saturating mutagenesis. *Nature,* 16 September 2015, vol. 527 (7577), 192-7
- **ZETSCHE et al.** Cpf1 Is a Single RNA-Guided Endonuclease of a Class 2 CRISPR-Cas System. *Cell,* 25 September 2015, vol. 163, 759-71
- **SHMAKOV et al.** Discovery and Functional Characterization of Diverse Class 2 CRISPR-Cas Systems. *Molecular Cell,* 22 October 2015, vol. 60 (3), 385-397
- **SLAYMAKER et al.** Rationally engineered Cas9 nucleases with improved specificity. *Science,* 01 December 2015, vol. 351 (6268), 84-88
- **GAO et al.** Engineered Cpf1 Enzymes with Altered PAM Specificities. *bioRxiv 091611,* 04 December 2016
- **COX et al.** RNA editing with CRISPR-Cas13. *Science,* 25 October 2017, vol. 358 (6366), 1019-1027
- **SHENGDAR Q. TSAI ; NICOLAS WYVEKENS ; CYD KHAYTER ; JENNIFER A. FODEN ; VISHAL THAPAR ; DEEPAK REYON ; MATHEW J. GOODWIN ; MARTIN J. ARYEE ; J. KEITH JOUNG.** Dimeric CRISPR RNA-guided FokI nucleases for highly specific genome editing. *Nature Biotechnology,* 2014, vol. 32 (6), 569-77
- **KIM, Y. G. et al.** Chimeric restriction endonuclease. *Proc. Natl. Acad. Sci. U.S.A.,* 1994, vol. 91, 883-887
- **KIM, Y. G. et al.** Hybrid restriction enzymes: zinc finger fusions to Fok I cleavage domain. *Proc. Natl. Acad. Sci. U.S.A.,* 1996, vol. 93, 1156-1160
- **DOYON, Y. et al.** Enhancing zinc-finger-nuclease activity with improved obligate heterodimeric architectures. *Nat. Methods,* 2011, vol. 8, 74-79
- **MOSCOU et al.** *Science,* 2009, vol. 326, 1501
- **BOCH et al.** *Science,* 2009, vol. 326, 1509-1512
- **ZHANG et al.** *Nature Biotechnology,* 2011, vol. 29, 149-153
- **KOREN et al.** *Cell,* 20 November 2014, vol. 159 (5), 1015-26
- **ROACH et al.** *Science,* 30 April 2010, vol. 328 (5978), 636-639
- **AGGELI et al.** Diff-seq: A high throughput sequencing-based mismatch detection assay for DNA variant enrichment and discovery. *Nucleic Acids Res.,* 20 April 2018, vol. 46 (7), e42
- **TOMASETTI ; VOGELSTEIN.** Variation in cancer risk among tissues can be explained by the number of stem cell divisions. *Science,* 02 January 2015, vol. 347 (6217), 78-81
- **ALBERTSON TM ; OGAWA M ; BUGNI JM ; HAYS LE ; CHEN Y ; WANG Y ; TREUTING PM ; HEDDLE J ; GOLDSBY RE ; PRESTON BD.** DNA polymerase epsilon and delta proofreading suppress discrete mutator and cancer phenotypes in mice. *Proc Natl Acad Sci USA,* 2009, vol. 106, 171014, www.ncbi.nlm.nih.gov/pubmed/19805137%5Cnwww.pubmedcentral.nih.gov/articlerender.fcgi?artid=PMC2761330
- **ALEXANDROV LB ; NIK-ZAINAL S ; WEDGE DC ; APARICIO SAJR ; BEHJATI S ; BIANKIN A V ; BIGNELL GR ; BOLLI N ; BORG A ; BORRESEN-DALE A-L et al.** Signatures of mutational processes in human cancer. *Nature,* 2013, vol. 500, 415-21, www.ncbi.nlm.nih.gov/pubmed/23945592
- **ALEXANDROV LB ; NIK-ZAINAL S ; WEDGE DC ; CAMPBELL PJ ; STRATTON MR.** Deciphering Signatures of Mutational Processes Operative in Human Cancer. *Cell Rep,* 2013, vol. 3, 246-259

- **ARATEN DJ ; GOLDE DW ; ZHANG RH ; THALER HT ; GARGIULO L ; NOTARO R ; LUZZATTO L.** A quantitative measurement of the human somatic mutation rate. *Cancer Res,* 2005, vol. 65, 8111-7, www.ncbi.nlm.nih.gov/pubmed/16166284
- **BARRETINA J ; CAPONIGRO G ; STRANSKY N ; VENKATESAN K ; MARGOLIN AA ; KIM S ; WILSON CJ ; LEHAR J ; KRYUKOV G V ; SONKIN D et al.** The Cancer Cell Line Encyclopedia enables predictive modelling of anticancer drug sensitivity. *Nat (London, UK),* 2012, vol. 483, 603-607
- **BASLAN T ; HICKS J.** Unravelling biology and shifting paradigms in cancer with single-cell sequencing. *Nat Rev Cancer,* 2017, vol. 17, 557-569
- **BI X.** Mechanism of DNA damage tolerance. *World J Biol Chem,* 2015, vol. 6, 48, www.wjgnet.com/1949-8454/full/v6/i3/48.htm
- **BLOKZIJL F ; DE LIGT J ; JAGER M ; SASSELLI V ; ROERINK S ; SASAKI N ; HUCH M ; BOYMANS S ; KUIJK E ; PRINS P et al.** Tissue-specific mutation accumulation in human adult stem cells during life. *Nature,* 2016, vol. 538, 260-264
- **BODNAR AG.** Extension of Life-Span by Introduction of Telomerase into Normal Human Cells. *Science,* 1998, vol. 279, 349-352, www.sciencemag.org/cgi/doi/10.1126/science.279.5349.349
- **CAMPBELL PJ ; MARTINOCORENA I ; CAMPBELL PJ.** Somatic mutation in cancer and normal cells. *Science,* 2015, vol. 349, 1483-1489
- **CHEN C ; XING D ; TAN L ; LI H ; ZHOU G ; HUANG L ; XIE XS.** Single-cell whole-genome analyses by Linear Amplification via Transposon Insertion (LIAN-TI). *Science,* 2017, vol. 356, 189-194
- **CIBULSKIS K ; LAWRENCE MS ; CARTER SL ; SIVACHENKO A ; JAFFE D ; SOUGNEZ C ; GABRIEL S ; MEYERSON M ; LANDER ES ; GETZ G.** Sensitive detection of somatic point mutations in impure and heterogeneous cancer samples. *Nat Biotechnol,* 2013, vol. 31, 213-219, www.nature.com/doifinder/10.1038/nbt2514
- **DROST J ; VAN BOXTEL R ; BLOKZIJL F ; MIZUTANI T ; SASAKI N ; SASSELLI V ; DE LIGT J ; BEHJATI S ; GROLLEMAN JE ; VAN WEZEL T et al.** Use of CRISPR-modified human stem cell organoids to study the origin of mutational signatures in cancer. *Science,* 2017, vol. 3130, eaao3130, www.ncbi.nlm.nih.gov/pubmed/28912133
- **DULAK AM ; STOJANOV P ; PENG S ; LAWRENCE MS ; FOX C ; STEWART C ; BANDLA S ; IMAMURA Y ; SCHUMACHER SE ; SHEFLER E et al.** Exome and whole-genome sequencing of esophageal adenocarcinoma identifies recurrent driver events and mutational complexity. *Nat Genet,* 2013, vol. 45, 478-486
- **FERNÁNDEZ LC ; TORRES M ; REAL FX.** Somatic mosaicism: on the road to cancer. *Nat Rev Cancer,* 2016, vol. 16, 43-55, www.ncbi.nlm.nih.gov/pubmed/26678315
- **FORBES SA ; BEARE D ; GUNASEKARAN P ; LEUNG K ; BINDAL N ; BOUTSELAKIS H ; DING M ; BAMFORD S ; COLE C ; WARD S et al.** COSMIC: Exploring the world's knowledge of somatic mutations in human cancer. *Nucleic Acids Res,* 2015, vol. 43, D805-D811, www.ncbi.nlm.nih.gov/pubmed/25355519
- **FRIEDA KL ; LINTON JM ; HORMOZ S ; CHOI J ; CHOW KHK ; SINGER ZS ; BUDDE MW ; ELOWITZ MB ; CAI L.** Synthetic recording and in situ readout of lineage information in single cells. *Nature,* 2017, vol. 541, 107-111
- **FRIGOLA J ; SABARINATHAN R ; MULARONI L ; MUINÕS F ; GONZALEZ-PEREZ A ; LOPEZ-BIGAS N.** Reduced mutation rate in exons due to differential mismatch repair. *Nat Genet,* 2017, vol. 49, 1684-1692
- **GARBE JC ; VRBA L ; SPUTOVA K ; FUCHS L ; NOVAK P ; BROTHMAN AR ; JACKSON M ; CHIN K ; LA BARGE MA ; WATTS G et al.** Immortalization of normal human mammary epithelial cells in two steps by direct targeting of senescence barriers does not require gross genomic alterations. *Cell Cycle,* 2014, vol. 13, 3423-3435
- **GAWAD C ; KOH W ; QUAKE SR.** Single-cell genome sequencing: Current state of the science. *Nat Rev Genet,* 2016, vol. 17, 175-188
- **GEACINTOV NE ; BROYDE S.** Repair-Resistant DNA Lesions. *Chem Res Toxicol,* 2017, vol. 30, 1517-1548
- **HARADHVALA NJ ; POLAK P ; STOJANOV P ; COVINGTON KR ; SHINBROT E ; HESS JM ; RHEINBAY E ; KIM J ; MARUVKA YE ; BRAUNSTEIN LZ et al.** Mutational Strand Asymmetries in Cancer Genomes Reveal Mechanisms of DNA Damage and Repair. *Cell,* 2016, vol. 164, 538-549
- **HOLSTEGE H ; PFEIFFER W ; SIE D ; HULSMAN M ; NICHOLAS TJ ; LEE CC ; ROSS T ; LIN J ; MILLER M A ; YLSTRA B et al.** Somatic mutations found in the healthy blood compartment of a 115-yr-old woman demonstrate oligoclonal hematopoiesis. *Genome Res,* 2014, vol. 24, 733-42, www.pubmedcentral.nih.gov/articlerender.fcgi?artid=4009603&tool=pmcentrez&rendertype=ab stract
- **HUFF JT ; PLOCIK AM ; GUTHRIE C ; YAMAMOTO KR.** Reciprocal intronic and exonic histone modification regions in humans. *Nat Struct Mol Biol,* 2010, vol. 17, 1495-1499, www.nature.com/doifinder/10.1038/nsmb.1924

- **JU YS ; MARTINCORENA I ; GERSTUNG M ; PETLJAK M ; ALEXANDROV LB ; RAHBARI R ; WEDGE DC ; DAVIES HR ; RAMAKRISHNA M ; FULLAM A et al.** Somatic mutations reveal asymmetric cellular dynamics in the early human embryo. *Nature,* 2017, vol. 543, 714-718, www.nature.com/doifinder/10.1038/nature21703
- **KIMMERLING RJ ; LEE SZETO G ; LI JW ; GENSHAFT AS ; KAZER SW ; PAYER KR ; DE RIBA BORRAJO J ; BLAINEY PC ; IRVINE DJ ; SHALEK AK et al.** A microfluidic platform enabling single-cell RNA-seq of multigenerational lineages. *Nat Commun,* 2016, vol. 7, 10220, www.pubmedcentral.nih.gov/articlerender.fcgi?artid=4729820&tool=pmcentrez&rendertype=ab stract
- **KOREN A ; POLAK P ; NEMESH J ; MICHAELSON JJ ; SEBAT J ; SUNYAEV SR ; MCCARROLL SA.** Differential relationship of DNA replication timing to different forms of human mutation and variation. *Am J Hum Genet,* 2012, vol. 91, 1033-1040
- **KUNKEL TA ; ERIE DA.** Eukaryotic Mismatch Repair in Relation to DNA Replication. *Annu Rev Genet,* 2015, vol. 49, 291-313, www.annualreviews.org/doi/10.1146/annurev-genet-112414-054722
- **LANGE SS ; TAKATA K ; WOOD RD.** DNA polymerases and cancer. *Nat Rev Cancer,* 2011, vol. 11, 96-110
- **LI F ; MAO G ; TONG D ; HUANG J ; GU L ; YANG W ; LI GM.** The histone mark H3K36me3 regulates human DNA mismatch repair through its interaction with MutS$\alpha$. *Cell,* 2013, vol. 153, 590-600
- **LI J ; POURSAT MA ; DRUBAY D ; MOTZ A ; SACI Z ; MORILLON A ; MICHIELS S ; GAUTHERET D.** A Dual Model for Prioritizing Cancer Mutations in the Non-coding Genome Based on Germline and Somatic Events. *PLoS Comput Biol,* 2015, vol. 11, 1-17
- **LI R ; MONTPETIT A ; ROUSSEAU M ; WU SYM ; GREENWOOD CMT ; SPECTOR TD ; POLLAK M ; POLYCHRONAKOS C ; RICHARDS JB.** Somatic point mutations occurring early in development: a monozygotic twin study. *J Med Genet,* 2014, vol. 51, 28-34, www.ncbi.nlm.nih.gov/pubmed/24123875
- **LODATO MA ; RODIN RE ; BOHRSON CL ; COULTER ME ; BARTON AR ; KWON M ; SHERMAN MA ; VITZTHUM CM ; LUQUETTE LJ ; YANDAVA C et al.** Aging and neurodegeneration are associated with increased mutations in single human neurons. *Science,* 2017, www.ncbi.nlm.nih.gov/pubmed/29217584
- **LODATO MA ; WOODWORTH MB ; LEE S ; EVRONY GD ; MEHTA BK ; KARGER A ; LEE S ; CHITTENDEN TW ; D'GAMA AM ; CAI X et al.** Somatic mutation in single human neurons tracks developmental and transcriptional history. *Science,* 2015, vol. 350, 94-98, www.sciencemag.org/cgi/doi/10.1126/science.aab1785
- **LYNCH M.** Rate, molecular spectrum, and consequences of human mutation. *Proc Natl Acad Sci USA,* 2010, vol. 107, 961-8, www.pubmedcentral.nih.gov/articlerender.fcgi?artid=2824313&tool=pmcentrez&rendertype=ab stract
- **MAKRIDAKIS NM ; REICHARDT JK V.** Translesion DNA polymerases and cancer. *Front Genet,* 2012, vol. 3, 1-8
- **MARTINCORENA I ; ROSHAN A ; GERSTUNG M ; ELLIS P ; VAN LOO P ; MCLAREN S ; WEDGE DC ; FULLAM A ; ALEXANDROV LB ; TUBIO JM et al.** High burden and pervasive positive selection of somatic mutations in normal human skin. *Science,* 2015, vol. 348, 880-886, www.ncbi.nlm.nih.gov/pubmed/25999502
- **MCGRANAHAN N ; SWANTON C.** Clonal Heterogeneity and Tumor Evolution: Past, Present, and the Future. *Cell,* 2017, vol. 168, 613-628
- **MCKENNA A ; FINDLAY GM ; GAGNON JA ; HORWITZ MS ; SCHIER AF ; SHENDURE J.** Whole-organism lineage tracing by combinatorial and cumulative genome editing. *Science,* 2016, vol. 353, aaf7907, www.sciencemag.org/lookup/doi/10.1126/science.aaf7907
- **MERKLE FT ; GHOSH S ; KAMITAKI N ; MITCHELL J ; AVIOR Y ; MELLO C ; KASHIN S ; MEKHOUBAD S ; ILIC D ; CHARLTON M et al.** Human pluripotent stem cells recurrently acquire and expand dominant negative P53 mutations. *Nature,* 2017, vol. 545, 229-233, www.nature.com/doifinder/10.1038/nature22312
- **MILHOLLAND B ; DONG X ; ZHANG L ; HAO X ; SUH Y ; VIJG J.** Differences between germline and somatic mutation rates in humans and mice. *Nat Commun,* 2017, vol. 8, 1-8
- **PETLJAK M ; ALEXANDROV LB.** Understanding mutagenesis through delineation of mutational signatures in human cancer. *Carcinogenesis,* 2016, vol. 37, 531-540
- **PRUITT KD ; TATUSOVA T ; MAGLOTT DR.** NCBI reference sequences (RefSeq): A curated non-redundant sequence database of genomes, transcripts and proteins. *Nucleic Acids Res,* 2007, vol. 35, 61-65

- **ROACH JC ; GLUSMAN G ; SMIT AFA ; HUFF CD ; HUBLEY R ; SHANNON PT ; ROWEN L ; PANT KP ; GOODMAN N ; BAMSHAD M et al.** Analysis of genetic inheritance in a family quartet by whole-genome sequencing. *Science,* 2010, vol. 328, 636-9, www.ncbi.nlm.nih.gov/pubmed/20220176
- **ROSSI DJ ; JAMIESON CHM ; WEISSMAN IL.** Stems Cells and the Pathways to Aging and Cancer. *Cell,* 2008, vol. 132, 681-696
- **RUBIN AG et al.** Bayesian Data Analysis. CRC Press, 2013
- **SALE JE.** Translesion DNA synthesis and mutagenesis in eukaryotes. *Cold Spring Harb Perspect Biol,* 2013, vol. 5
- **SALE JE ; LEHMANN AR ; WOODGATE R.** Y-family DNA polymerases and their role in tolerance of cellular DNA damage. *Nat Rev Mol Cell Biol,* 2012, vol. 13, 141-152
- **SCHWARTZ S ; MESHORER E ; AST G.** Chromatin organization marks exon-intron structure. *Hum Mol Genet.,* 2009
- **SHINBROT E ; HENNINGER EE ; WEINHOLD N ; COVINGTON KR ; GÖKSENIN AY ; SCHULTZ N ; CHAO H ; DODDAPANENI H ; MUZNY DM ; GIBBS RA et al.** Exonuclease mutations in DNA polymerase epsilon reveal replication strand specific mutation patterns and human origins of replication. *Genome Res,* 2014, vol. 24, 1740-50, www.ncbi.nlm.nih.gov/pubmed/25228659
- **SMITH KS ; LIU LL ; GANESAN S ; MICHOR F ; DE S.** Nuclear topology modulates the mutational landscapes of cancer genomes. *Nat Struct Mol Biol,* 2017, vol. 24, 1000-1006, www.ncbi.nlm.nih.gov/pubmed/28967881
- **SUPEK F ; LEHNER B.** Differential DNA mismatch repair underlies mutation rate variation across the human genome. *Nature,* 2015, vol. 521, 81-84, www.nature.com/doifinder/10.1038/nature14173
- **SZIKRISZT B ; POTI Á ; PIPEK O ; KRZYSTANEK M ; KANU N ; MOLNÁR J ; RIBLI D ; SZELTNER Z ; TUSNÁDY GE ; CSABAI I et al.** A comprehensive survey of the mutagenic impact of common cancer cytotoxics. *Genome Biol,* 2016, vol. 17, 99
- **TENAILLON O ; BARRICK JE ; RIBECK N ; DEATHERAGE DE ; BLANCHARD JL ; DASGUPTA A ; WU GC ; WIELGOSS S ; CRUVEILLER S ; MÉDIGUE C et al.** Tempo and mode of genome evolution in a 50,000-generation experiment. *Nature,* 2016, vol. 536, 165-170, www.nature.com/doifinder/10.1038/nature18959
- **TOMASETTI C ; DURRETT R ; KIMMEL M ; LAMBERT A ; PARMIGIANI G ; ZAUBER A ; VOGELSTEIN B.** Role of stem-cell divisions in cancer risk. *Nature,* 2017, vol. 548, E13-E14, www.nature.com/doifinder/10.1038/nature23302
- **VIJG J ; DONG X ; MILHOLLAND B ; ZHANG L.** Genome instability: a conserved mechanism of ageing?. *Essays Biochem,* 2017, vol. 61, 305-315, www.ncbi.nlm.nih.gov/pubmed/28550046
- **WOODWORTH MB ; GIRSKIS KM ; WALSH CA.** Building a lineage from single cells: genetic techniques for cell lineage tracking. *Nat Rev Genet,* 2017, vol. 18, 230-244, www.ncbi.nlm.nih.gov/pubmed/28111472
- **WU S ; POWERS S ; ZHU W ; HANNUN YA.** Substantial contribution of extrinsic risk factors to cancer development. *Nature,* 2016, vol. 529, 43-7, www.nature.com/doifinder/10.1038/nature16166
- **ZIV O ; ZEISEL A ; MIRLAS-NEISBERG N ; SWAIN U ; NEVO R ; BEN-CHETRIT N ; MARTELLI MP ; ROSSI R ; SCHIESSER S ; CANMAN CE et al.** Identification of novel DNA-damage tolerance genes reveals regulation of translesion DNA synthesis by nucleophosmin. *Nat Commun,* 2014, vol. 5